# EUROPEAN PATENT APPLICATION

(11) **EP 0 831 149 A1**
(43) Date of publication of application: **25.03.1998**
(21) Application number: 96900433.2
(22) Date of filing: 12.01.1996
(51) Int. Cl.: C12N 15/52, C12N 1/21, C12P 7/64

(54) **GENES CODING FOR BIOSYNTHETIC ENZYME GROUP FOR ICOSAPENTAENOIC ACID AND PROCESS FOR PRODUCING ICOSAPENTAENOIC ACID**

(30) Priority: 13.01.1995 JP 4299/95
(71) Applicant: SAGAMI CHEMICAL RESEARCH CENTER, Sagamihara-shi, Kanagawa 229 (JP)
(72) Inventor: YAZAWA, Kazunaga, Sagamihara-shi, Kanagawa 228 (JP); YAMADA, Akiko, Sagamihara-shi, Kanagawa 228 (JP); KONDO, Kiyosi, Yamato-shi, Kanagawa 242 (JP); KATO, Seishi, Sagamihara-shi, Kanagawa 228 (JP)
(74) Representative: Keller, Günter, Dr.
(86) International application number: JP9600030
(87) International publication number: WO9621735

(57) **Abstract**

An advantageous process for producing eicosapentaenoic acid (EPA) useful as medicines, agricultural chemicals, food, feeds, and so forth, which comprises obtaining from microorganisms a gene coding for the biosynthetic enzyme group for EPA, connecting the gene and a vector to prepare a plasmid, transforming Escherichia coli with the plasmid, and culturing the transformant.

## Description

### FIELD OF THE ART

The present invention relates to genes coding for eicosapentaenoic acid (designated EPA hereinafter) synthesizing enzymes, plasmids containing these genes, microorganisms transformed with the plasmid, and a process for production of eicosapentaenoic acid using the microorganism. The EPA is useful as a starting material for pharmaceuticals, foods, feeds and the like.

### BACKGROUND ART

Polyunsaturated fatty acids represented by eicosapentaenoic acid (EPA) play an important roll as a component of a biomembrane. So far, the following pharmacological actions of EPA are known. (1) Platelet coagulation inhibitory action (thrombolytic action), (2) blood neutral fat-lowering action, (3) actions for lowering blood VLDL-cholesterol and LDL-cholesterol and increasing HDL-cholesterol (anti-arterial sclerosis action), (4) blood viscosity-lowering action, (5) blood pressure lowering action, (6) anti-inflammatory action, (7) anti-tumor action.

In addition, EPA is a substrate for biosynthesis of prostaglandins and exhibits an essential function in vivo in higher mammals including humans. In particular EPA is important as a substrate for production of three types of prostaglandins, has platelet coagulation inhibitory action and is studied for applications to treatment and prophylactic agents for thrombosis. In addition EPA has especially high activity for lowering plasma cholesterol level among polyunsaturated fatty acids having said action, and is highly effective in comparison with linoleic acid and the like usually contained in plant oil. Additionally, EPA is known as an essential nutrient for fish.

Thus, epidemiological research by Dyerberg, Denmark (Am. J. Clin. Nutur, 28, 959, 1975) showed a possibility for use as health foods or pharmaceuticals on the basis of thrombosis inhibitory action or lipid-lowering actions of EPA. However, as can be seen from its chemical structure, the chemical synthesis of EPA is very difficult. Accordingly, in Japan, it is recommended to eat blue back fish such as sardine, salmon, saury, and the like.

At present, most commercially available EPA products are fractionation products from fish oil obtained by a boiling process, and the EPA content thereof is about 10 to 30%. Fish oil extracted by a boiling process is a mixed glyceride containing various kind of fatty acids as component fatty acids, and not only is isolation and purification of each component difficult, but also since EPA is a polyunsaturated fatty acid having 20 carbon atoms and five double bonds all of which are cis-type, EPA is also a unstable and highly oxidation-liable fatty acid. Therefore, it is necessary for EPA to be concentrated from fish oil with prevention of oxygen, light, heat and the like. In addition, since fish oil contains various fatty acids in addition to EPA, their fractionation is difficult. Moreover, although various organic solvents used for fractionation of EPA are eliminated under a reduced pressure, complete elimination of the organic solvents is difficult from a technical and economical point of view.

Most EPA preparations used for pharmaceuticals are those having at least 90% EPA concentration, produced by extracting fish oil by various Processes, hydrolysing the fish oil enzymatically or under an alkaline condition to generate free fatty acids, optionally converting the free fatty acids to corresponding methyl or ethyl esters, and further purifying by fractional crystallization at a low temperature, applying a urea-addition method, distillation under a reduced pressure, reverse phase chromatography, or the like. However, since these processes use many organic solvents and heating to near 200°C, it is possible that an EPA concentrate obtained by using such process may be denaturated by residual organic solvent, and polymerization, isomerization or oxidation of EPA. Moreover, where a fish oil is used as a starting material for production of EPA, it is difficult to eliminate docosanoic acid or the like which is considered to be a cause of cardiodiseases, and therefore problems remain in the use for health foods, pharmaceuticals or the like.

On the other hand, recently, processes for production of EPA using microorganisms such as chlorella, unicellular algae Monodus, Eugrena or Diatomaceae have been studied in place of extraction methods from fish oil having drawbacks such as residual fish odor due to incomplete purification and concentration, and the production of EPA using microorganisms has been considered. Recently, fungi producing EPA were reported by Gellerman and Schlenk (J. L. Gellerman and H. Schlenk, BBA, 573, 23, 1979) and Yamada et al. (Meeting of The society of Fermentation Technology, Japan, 1986).

The present inventors sought marine bacteria having an ability to produce EPA to find a new fermentation process for production of EPA using bacteria from which genes can be easily obtained, and which can be cultured in a short time and be easily controlled, and as a result, the present inventors found a new bacteria belonging to the genus Pseudomonas, Alteromonas or Shewanella (K. Yazawa et al., J. Biochem., 103, 5 (1988); K. Yazawa et al., Nippon Suisan Gakkai shi, 54, 1835 (1988)).

It has been suggested that biosynthesis of polyunsaturated fatty acids including EPA works by site-specific aerobic unsaturation of corresponding saturated fatty acids (for example, R. Jeffcoat and A. T. James (1984) in: S. Numa (Ed.): Fatty acid metabolism and its regulation, Elsevier, Amsterdam, pp85 - 112). Moreover, there is a report of WO93/23545-A (EP0594868-A) relating to biosynthetic enzymes which participate in EPA synthesis, and gene coding therefor.

### DISCLOSURE OF THE INVENTION

Generally, the ability of a wild strain to produce a useful substance is low, and therefore where it is intended that the ability of the microorganism is industrially used, an improvement, i.e., an increase of productivity of the microorganism is carried out by various methods. The present inventors intended to carry out research for increasing an EPA productivity by finding genes not described in literature for EPA biosynthetic enzymes using gene recombination techniques and introducing the same into another organism, to impart an EPA biosynthesis ability to an organism not having an EPA biosynthesis ability, and eventually to establish an advantageous process for production of EPA.

Accordingly, the present invention provides genes for EPA biosynthetic enzymes, expression plasmids containing said genes, organisms transformed with said plasmid, and a process for production of EPA using said organism.

### BRIEF EXPLANATION OF THE DRAWINGS

Figure 1 represents the structure of the plasmid pEPA containing a group of the present genes.

Fig. 2 represents a restriction enzyme map of a DNA fragment containing a group of the present genes.

### Best Mode for Carrying Out the Invention

According to the present invention, an EPA producing strain is constructed by extracting DNA from a microorganism having an ability to produce EPA (microbial origin of genes), cutting this DNA with restriction enzymes to excise genes coded for a group of EPA biosynthesis enzymes, introducing the genes into an appropriate vector to construct an expression plasmid, and transforming a host organism with the plasmid to construct an EPA producing strain. EPA can be produced by this organism.

### Gene source

Although organisms which can be used as a gene source according to the present invention are not limited to specific genera, species or strains, usually, microorganisms classified to the genus Pseudomonas, Alteromonas, Shewanella or the like can be used. These microorganisms can be easily obtained from official or public depository institutes for microorganisms.

As examples of microorganisms belonging to the genus Pseudomonas, Pseudomonas putrefaciens SCRC-2181 (FERM BP-2917), SCRC-2201 (FERM BP-2916), SCRC-2271 (FERM BP-2195), SCRC-2341 (FERM BP-2918), SCRC-2451 (FERM BP-2919), SCRC-2642 (FERM BP-2920), SCRC-2792 (FERM BP-2921), SCRC-2878 (FERM BP-1623), SCRC-3011 (FERM BP-2913), and SCRC-3022 (FERM BP-2914) may be mentioned.

As example of microorganisms belonging to the genus Alteromonas, Alteromonas putrefaciens SCRC-2871 (FERM BP-1624) and Alteromonas putrefaciens subspecies sagamifaciens SCRC-1162 (FERM BP-1626) may be mentioned.

As an example of microorganism belonging to the genus Shewanella, Shewanella putrefaciens SCRC-2874 (FERM BP-1625) may be mentioned.

### Cloning of Genes Coding for a Group of EPA Biosynthesis Enzymes and Construction of Expression Plasmid

In the present invention, the case wherein Shewanella putrefaciens SCRC-2874 (FERM BP-1625) was used as a source of genes for a group of EPA biosynthesis enzymes is concretely explained. However, as described above, various EPA producing microorganisms can be similarly used as a gene source. A process for cloning genes is described in the Examples of the present invention.

According to the present invention, EPA producing strains can be artificially generated by transforming an heterogeneous host such as Escherichia coli or a homogeneous host such as Shewanella, or further yeast, fungus or the like. In addition, EPA producing plants can be generated by introducing the present genes into a higher plant such as soybean, sunflower, rape, or the like. As a region for expression of a group of EPA biosynthesis genes, although a control region natively accompanying these enzymes can be used, it is advantageous to prepare another promoter/operator system for increasing an amount of expression or allowing inducible expression. Where E. coli is used as a host, as the promoter/operator system, trp, tac, lavUV5, P_{L}, P_{R} or 1pp promoter/operator system and the like can be used, and as an SD sequence, an SD sequence of trp leader peptide, lacZ, metapyrocatechase or cII gene can be used. In addition, a transcriptional terminator, for example, rrnBT₁T₂ terminator of E. coli ribosome gene or the like can be provided downstream of a coding region. In addition, for expression of the above-mentioned gene, a host/vector system of Saccharomyces cerevisiae can be used, wherein as a promoter there can be used a promoter of alcohol dehydrogenase gene, a promoter of acid phosphatase gene, a promoter of glycelaldehyde-3-phosphate dehydrogenase gene, a promoter of enolase gene or the like can be used, wherein a plasmid preferably contains a sequence for replication in yeast, and an auxotrophic marker as a selectable maker for selection of yeast containing said plasmid, such as Leu, Trp, His or the like.

For introduction of the genes into a plant, there are a method using a vector, and a direct introduction method. As vectors, Ti plasmid; DNA viruses such as cauliflower mosaic virus (CaMV), Geminivirus, cassava roten virus, tomato golden mosaic virus, and the like; and RNA viruses such as brome mosaic virus (BMV), and tobacco mosaic virus (TMV) can be used, wherein as a promoter, 35S promoter of CaMV or the like may be mentioned. On the other hand, as direct protoplast introduction methods there can be mentioned a calcium phosphate method, polyethylene glycol method, microinjection, electroporation, liposome method and the like. Moreover, as a direct plant cell introduction method, there may be mentioned a particle gun method.

In addition, a fatty acid composition in a host plant can be changed by using a part of the group of the genes.

Note, that generally, an amount of expression of a particular protein in E. coli is affected by the number of copies of the genes, an efficiency of transcription, stability of mRNA, efficiency of translation, and stability of the protein. To modify control regions such as a promoter, SD region, terminator and the like, a smaller plasmid can be easily treated. The number of copies of the genes depends on the size of the plasmid, and there is a tendency for the plasmid to be smaller as the number of copies increases. For this purpose, a smaller plasmid can be obtained by inserting a DNA fragment containing a group of genes for EPA biosynthesis described in the Examples of the present invention into a plasmid, and repeating subcloning of the plasmid to cut off unnecessary portions of the gene DNA fragment. Smaller EPA biosynthesis enzyme genes thus obtained are included in the present invention. In addition, to enhance stability or activity of the enzyme, the nucleotide sequence of the gene (amino acid sequence) can be modified by a known technique, and the present invention includes such a modified gene.

As a host E. coli of the present invention, any strain derived from E. coli K12 may be used. For example, JM83, JM101, JM103, JM105, JM109, RR1, RB791, W3110, C600, HB101, DH1, AG1, NM554 or the like can be used. As a yeast host, AH22, DC5, D-13-1A, YNN144 or the like can be mentioned.

Note, that a group of the enzymes encoded by the present genes can convert higher fatty acids synthesized by a biosynthetic system natively possessed by the host organism to eicosapentaenoic acid.

In practicing the present invention, an organism such as a microorganism transformed with the present genes is cultured in a medium according to a conventional procedure to obtain microbial cells. In this case, for example, a medium having a composition shown in Table 1 is prepared.

**Table 1**

| | |
|---|---|
| Yeast extract | 0.5% |
| Pepton | 1.0% |
| Sea water | 1/2 concentration |
| pH 7.0 | |

From the microbial cells thus obtained, EPA can be obtained according to a conventional procedure such as extraction with an organic solvent. The details are described in the following Examples.

### EXAMPLES

Next, the present invention is explained in more detail by way of Examples.

### Example 1-1 Preparation of Genomic DNA Containing Genes Coding for a Group of EPA Biosynthesis Enzymes

Shewanella putrefaciens SCRC-2874 (FERM BP-1625) was inoculated in 125 ml of a medium (1% pepton, 0.5% yeast extract, 1/2 concentration artificial sea water), and cultured at 15°C for 18 hours with shaking (OD610 = 8.6). The resulting microbial cells were washed once with 1M NaCl, and suspended in 20 ml of 1M NaCl. The suspension was allowed to stand at 55°C for 30 minutes, 20 ml of 0.1M EDTA was added thereto, and after being allowed to stand at 55°C for 15 minutes, the suspension was centrifuged at 10,000 rpm for 10 minutes. To the precipitate, was added 10 ml of TES buffer (1 mM EDTA, 0.1 mM NaCl, 10 mM Tris-HCl, pH 8.0) containing 100 mg of lysozyme, and the cells were suspended. After being allowed to stand at 37°C for an hour, 1 ml of 10% SDS was added to the suspension, which was then allowed to stand at 60°C for an hour. 11 ml of neutralized phenol was added to the suspension, which was then gently shaken for 5 minutes and centrifuged at 6,500 rpm for 5 minutes to obtain the upper layer. 20 ml of ethanol was added to the layer and the mixture was gently shaken. Precipitated DNA was wound onto a glass bar, washed in ethanol, dissolved in 10 ml of TES buffer, and the solution was allowed to stand at 4°C overnight. 0.5 mg of RNase A was added to the solution, which was then gently shaken at 37°C for 3 hours, and after 1 mg of proteinase K was added thereto, the solution was further shaken for 4.5 hours; 5 ml each of neutralized phenol and chloroform were gradually added to the mixture, which was then gently shaken for 5 minutes and centrifuged to recover the upper layer. 10 ml of chloroform was added to the layer, which was then gently shaken and centrifuged to obtain the upper layer. 20 ml of ethanol was added to the layer, which was then gently shaken, and precipitated DNA was wound onto a glass bar. The DNA was washed in ethanol, and dissolved in 3 ml of TES buffer. An amount of DNA thus obtained was about 2.8 mg. Next, 200 µg of the DNA was partially digested with restriction enzyme Sau3A1 and subjected to electrophoresis on 0.3% agarose, and DNA fragments larger than about 20 Kb were isolated by electroelusion. The DNA fragments were extracted with phenol/chloroform, and precipitated with ethanol, and the precipitate was dissolved in 500 µl of TE buffer (1 mM EDTA, 10 mM Tris-HCl, pH 7.4).

### Example 1-2 Insertion of Chromosomal DNA Fragments into Vector

As a vector, cosmid pWE15 (STRATAGENE) was used. 10 µg of pWE15 was completely digested with restriction enzyme BamHI, treated with calf intestine alkaline phosphatase at 37°C for an hour, extracted with phenol/chloroform, and precipitated with ethanol, and the precipitate was dissolved in 10 µl of TE buffer. 1.5 µg of the vector DNA thus obtained was mixed with 1 µg of the chromosomal DNA prepared in Example 1 and partially digested with restriction enzyme Sau3A1, and these DNA were ligated using T4 DNA ligase at 26°C for 10 minutes. One fourth of the reaction mixture was packaged according to a conventional method to form phage, which was then infected to E. coli K12/AG-1.

### Example 1-3 Screening of Recombinant EPA Producing Strain

The E. coli suspension infected with the phage of Example 1-2 was plated on an LB agar medium (trypton 1%, yeast extract 0.5%, NaCl 1%, Agar 2%) containing 50 µg/ml ampicillin and cultured at 37°C overnight. The developed colony was inoculated in 1.5 ml of LB medium containing 50 µg/ml ampicillin, and cultured at 25°C for 1 to 7 days with shaking. The culture was centrifuged to collect the microbial cells, and after removing the medium, the cells were suspended in 0.5 ml of methanol saturated with hydrogen chloride. The cell suspension was sealed and incubated at 80°C for an hour to methyl-esterify fatty acids. After allowing the suspension to cool, it was extracted three times with 0.3 ml hexane, the hexane layer was dried and the residue was dissolved in 20 µl of methanol. 2 µl of the solution was spotted on a silica gel plate which was then developed three times with a developing solvent of hexane and ether 19:1, dried in air, and colored with iodine.

In this way, as a result of tests of about 390 recombinant clones, one clone showing a thin layer chromatography spot at the same position as a standard methyl ester of EPA was obtained. From the clone, cosmid was extracted using an alkali/SDS method. This cosmid was designated as pEPA. The pEPA is a cosmid wherein a San3AI fragment of about 38 Kbp was inserted into BamHI site of pWE15.

### Example 1-4 Preparation of Restriction Enzyme Map of pEPA

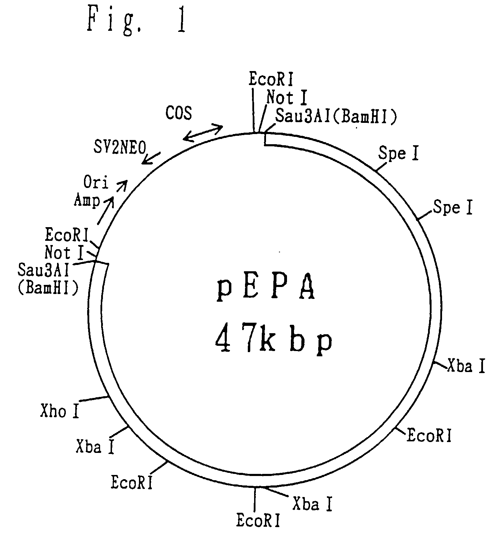

Cosmid pEPA was prepared from transformant AG-1/pEPA. The pEPA was cleaved with various restriction enzymes, and a restriction enzyme map was prepared (Fig. 1).

### Example 1-5 Analysis of Sequence

An entire nucleotide sequence of a Sau3A1-Sau3A1 fragment containing a genomic DNA insert in the cosmid pEPA is shown in SEQ ID NO: 1. In the nucleotide sequence, 9 open reading frames, ORFs 2 to 10, can be identified, and these nucleotide sequences and corresponding amino acid sequences are shown in SEQ ID NOs: 2 to 10 respectively. The relationship between the entire nucleotide sequence (SEQ ID NO: 1) and ORFs 2 to 10 (SEQ ID NOs: 2 to 10, respectively) is shown in Table 2.

**Table 2**

| SEQ ID NO | Length of sequence | Positions on SEQ ID NO: 1 |
|---|---|---|
| 2 | 1983 | 6121- 8103 |
| 4 | 831 | 9016- 8186* |
| 6 | 2910 | 9681-12590 |
| 8 | 864 | 13040-13903 |
| 10 | 8268 | 13906-22173 |
| 12 | 2340 | 22176-24515 |
| 14 | 6012 | 24518-30529 |
| 16 | 1629 | 30730-32358 |
| 18 | 1575 | 32753-34327 |

| | | |
|---|---|---|
| *Reversed sequence extending from the position No. 9016 to the position No. 8186 in the nucleotide sequence shown in SEQ ID NO: 1. | | |

By comparison of the amino acid sequences of various ORFs with known amino acid sequences, it was found that five regions in the ORF 6, and 2 regions in the ORF 8 have homology to some extent with amino acid sequences of enzymes participating in fatty acid synthesis. The results are shown in Table 3.

**Table 3**

| SEQ ID NO (ORF No.) | Position of amino acid sequence | Similar enzyme and position | References |
|---|---|---|---|
| 10 (6) | 668(Leu)-930(Leu) | MalonylCoA-ACP transferase 56(Leu)-309(Leu) | (1) |
| 10 (6) | 189(Phe)-424(His) | Fatty acid synthetase 120(Phe)-350(His) | (2) |
| 10 (6) | 200(Ser)-483(Leu) | Fatty acid synthetase (3-ketoacyl-ACP synthetase domain) 137(Ala)-406(Asp) | (3) |
| 10 (6) | 204(Ser)-488(Gln) | 3-Ketoacyl-ACP synthetase 137(Ala)-406(Asp) | (4) |
| 10 (6) | 2261(Phe)-2392(Gly) | 2-Oxoacylreductase 1470(Leu)-1604(Gly) | (5) |
| 14 (8) | 205(Ala)-442(Lys) | 3-Ketoacyl-ACP synthetase 187(Ala)-416(Asn) | (6) |
| 14 (8) | 1373(Thr)-1547(Val) | 3-Hydroxydecanoyl-ACP dehydratase 29(Leu)-163(Val) | (7) |
| References (1) Magnuson K. et al., FEBS Lett. (1992) 299:262-266 (2) Kameda K. et al., J. Biol. Chem. (1991) 266:419-426 (3) Huang W. Y. et al., Arch. Biochem. Biophys. (1989) 270:92-98 (4) Kauppinen S. et al., Carlsberg Res. Commun. (1988) 53:357-370 (5) Beck J. et al., Eur. J. Biochem. (1990) 192:487-498 (6) Siggaard-Andersen M. et al., Proc. Natl. Acad.Sci. U.S.A. (1991) 88:4114-4118 (7) Cronan Jr. J. E. et al., J. Biol. Chem. (1988) 263:4641-4646 | | | |

Accordingly, the present invention provides genes coding for amino acid sequences shown in SEQ ID Nos. 4, 8, 10, 14 and 18, as well as DNA coding for amino acid regions having a homology to amino acid sequences of said known enzymes, or coding for amino acid sequences comprising said amino acid regions.

### Example 2 Production of EPA by Transformant AG-1/pEPA

Transformant AG-1/pEPA was inoculated in 100 ml of LB medium containing 50 µl/ml ampicillin, and cultured at 25°C for 48 hours. The cells were obtained by centrifugation, washed once and suspended in 2 ml of pure water, and the suspension was extracted three times with 12 ml of a solvent composed of chloroform and methanol 2:1. The solvent layer was dried, and the residue was dissolved in 1.5 ml of methanol saturated with hydrogen chloride, and the solution was sealed and incubated at 80°C for an hour to methyl-esterify fatty acids. After allowing the solution to cool, it was extracted three times with 2 ml of hexane, and after the hexane layer was dried, the residue was dissolved in 20 µl of methanol. A part of the solution was analyzed by gas chromatography. As a result, a peak of EPA was observed, and a ratio of EPA relating to total fatty acid esters was calculated as about 1.36% from the area of peaks. The amount of EPA per culture volume was about 0.5 mg/l. The ester mixture thus obtained was spotted on an argentation silica gel plate, which was then developed by a solvent composed of hexane and ether at a ratio of 3:1. The plate was colored with fluorescein and ultraviolet light, the spot of the ester of the polyunsaturated fatty acid was scraped off, 1.8 ml of methanol and 0.2 ml of 10% NaCl were added thereto, and the mixture was shaken at room temperature for 30 minutes. The mixture was extracted three times with 2 ml of hexane, the hexane layer was dried, the residue was dissolved in 40 µl of hexane, and GC-MS analysis was carried out. As a result, molecular weight of the substance of the desired peak on the chromatography was 316, and the peak of the fragment conformed to that of an authentic sample, and the substance was identified as EPA. Note that MS fragment peaks were as follow:
- Mass:: 316(M⁺), 287, 273, 262, 247, 234, 220, 201, 180, 161, 148, 133, 119, 108, 93, 79, 67, 55, 41, 28.

### Example 3 Production of EPA by Transformant JM109/pEPA

According to a conventional procedure the cosmid pEPA was used to transform E. coli K12/JM109. JM109/pEPA (FERM BP-4257) was obtained by selection using an LB agar medium containing 50 µl/ml ampicillin. According to the same procedure as described in Example 2, extraction of lipid from the cells, methyl-esterification and analysis by gas chromatography were carried out and a peak of EPA was detected. A ratio of EPA relating to a total of esters of fatty acids was calculated as about 1.43% on the basis of the area of peaks. An amount of EPA per culture volume was about 0.6 mg/l.

### Reference to Deposited Microorganism under Rule 13-2 and Depository Authority

Depository Authority: National Institute of Bioscience and Human-Technology Agency of Industrial Science and Technology
Address: 1-3 Higashi 1-chome, Tsukuba-shi, Ibaraki-ken 305 Japan
Deposition Number and Deposition Date: FERM BP-4257 May 14, 1992

### Example 4 Construction of Partially-deleted pEPA and Production of EPA

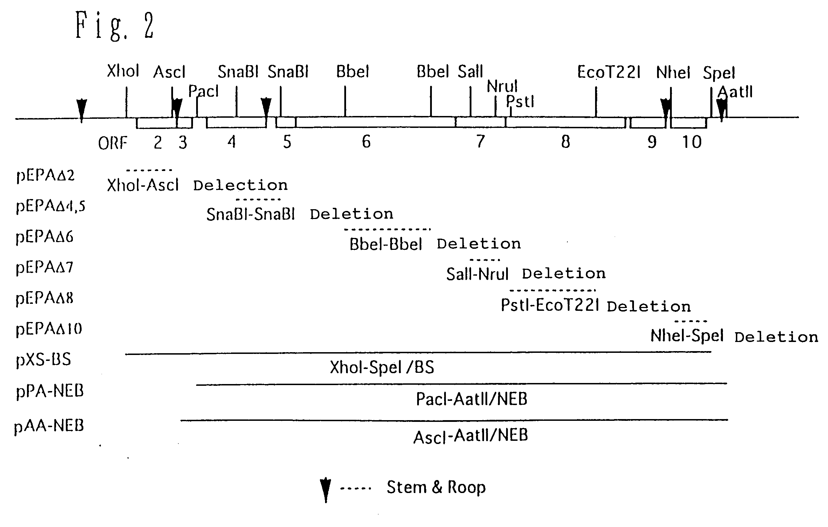

A part of SEQ ID NO: 1 was removed from pEPA by using various restriction enzymes, and the resulting pEPA was rejoined. In addition, an XhoI-SpeI fragment of pEPA was ligated to XhoI-SpeI site of plasmid pBluescript (STRATAGENE) so as to construct pXS-BS. Further, a PacI(9061)-AatII(35564) fragment of pEPA was ligated to PacI-AatII site of plasmid pNEB (New England Biolabs) to construct pPA-NEB. In Addition, an AscI(7710)-AatII(35564) fragment of pEPA was ligated to AscI-AatII site of plasmid pNEB (New England Biolabs) to construct pAA-NEB. These plasmids were used to transform E. coli, and production of eicosapentaenoic acid was tested. Result is shown in Table 4 and Fig. 2.

**Table 4**

| Name of plasmid | Position of deletion in SEQ ID NO: 1 | Deleted ORF | Productivity of EPA |
|---|---|---|---|
| pEPAΔ2 | XhoI(5666)-AscI(7709) | 2 | + |
| pEPAΔ4,5 | SnaBI(10944)-SnaBI(13226) | 4, 5 | ** |
| pEPAΔ6 | Bbel(16563)-BbeI(20702) | 6 | - |
| pEPAΔ7 | SalI(22265)-NruI(23847) | 7 | - |
| pEPAΔ8 | PstI(24814)-EcoT22I(28946) | 8 | - |
| pEPAΔ9 | SpeI(31446)-SpeI(34626)* | 9 | - |
| pEPAΔ10 | NheI(32526)-SpeI(34630) | 10 | - |
| pXS-BS | Sau3AI(1)-XhoI(5660), SpeI(34632)-Sau3AI(37895) | - | + |
| pPA-NEB | Sau3AI(1)-PacI(9060), AatII(35565)-Sau3AI(37895) | 2, 3 | - |
| pAA-NEB | Sau3AI(1)-AscI(7709) AatII(35565)-Sau3AI(37895) | 2 | + |

| | | | |
|---|---|---|---|
| ** One tenth or less of pEPA | | | |
| * The sequence was inserted in a reverse direction. | | | |

From the above-mentioned result, it is suggested that an upstream region of ORF 2, a downstream region of ORF 10, and ORF 2 itself do not participate in the synthesis of eicosapentaenoic acid. Also, it is observed that ORFs 3, 6, 7, 8, 9 and 10 are necessary for the synthesis of eicosapentaenoic acid, and at least one of ORFs 4 and 5 participates in the synthesis of eicosapentaenoic acid.

### Example 5 Subcloning of each ORF

Among ORFs in the pEPA, ORFs 4, 7, 8 and 9 were separately subcloned into pUC118. For the ORFs 4, 8 and 9, DNA sequence upstream of the translation start codon was shortened by replacing with the synthesized DNA sequence by PCR (polymerase chain reaction). The length of the inserted region and its position for each subclone are shown in Table 5, and primers used for construction of the subclones are shown in Table 6.

**Table 5**

| Plasmid | SEQ ID NO | Length of Sequence inserted | Position in SEQ ID NO: |
|---|---|---|---|
| pUCP2 | 6 | 3365 | 9573 - 12937 |
| pUCO5 | 12 | 3430 | 22119 - 25548 |
| pUCP6 | 14 | 7083 | 24364 - 31446 |
| pUCP7 | 16 | 2144 | 30629 - 32772 |
| pUCP8 | 18 | 2070 | 32557 - 34626 |

**Table 6**

| Plasmid | Primers | Sequence of primer (5' → 3') |
|---|---|---|
| pUCP2 | 1 | AGCTCAAACAACGCGCTTACA |
| | 2 | TGTTAGTCCCATCACGTTCTTG |
| pUCP6 | 1 | GCCATCATCAGGTGCCATTATCGGT |
| | 2 | GTCTGGGTAGGCGTGGAAGATT |
| pUCP7 | 1 | AGTATCTGCGTCCTAACTCGAT |
| | 2 | CCACCTGAATCGGCCTCTG |

### Example 6-1 Preparation of proteins

E. coli JM109/pXS-BS having an ability of synthesize EPA, and JM109 having a subclones constructed in Example 5 were cultured in 50 ml of LB-ampicillin medium at 25°C for 24 hours with shaking. Each culture was centrifuged at 4°C, 3,000 rpm (Hitachi RPR-20-2) for 20 minutes to collect the microbial cells. The cells were suspended in 10 ml of 10 mM PKB (10 mM potassium phosphate buffer, pH 7.0, 2 mM β-mercaptoethanol, 10 mM EDTA), and the suspension was centrifuged at 4°C, 3,000 rpm for 10 minutes to wash the cells. The cell precipitate was suspended in 2 ml of 10 mM PKB, and sonicated. The sonicated cells were centrifuged at 4°C, 33,000 rpm (Beckmann, SW55) for 80 minutes to obtain a supernatant solution, which was then used as an enzyme protein.

### Example 6-2 Detection of activity of enzyme protein to extend carbon chain

A reaction mixture for an enzyme reaction contained [1-¹⁴C]stearoyl-CoA (19 nmoles/µCi) and stearoyl-CoA (total concentration 25 µM), 25 µM malonyl-CoA, 100 µg E. coli acyl carrier-protein (ACP), 1.5 mM NADPH, 1.5 mM NADH, 10 µM cerulenin, 20 µM PMSF and 250 µg of an enzyme protein obtained in Example 6-1, in 0.5 ml of 0.1M potassium phosphate buffer (pH 7.0). A reaction was carried out at 25°C for 30 minutes with shaking.

The reaction mixture was lyophilized overnight, and to the lyophilizate was added 1 ml of 8% HCl-methanol, and the mixture was heated at 80°C for one hour so as to esterify fatty acids produced during the enzyme reaction. The reaction mixture was extracted three times with 1 ml each of n-hexane, and the hexane layer was evaporated in vacuum. The residue was extracted three times with 0.2 ml each of n-hexane. After concentration of the n-hexane extract, a part of the concentrate was co-chromatographed with methyl stearate and methyl arachidate on a reverse phase TLC plate (MERCK RP-8F₂₅₄S), and developed three times with acetonitrile:water (7:1 v/v) for 25 minutes. Distribution of radioisotope on the TLC plate was detected by an AMBIS-RI imaging system and autoradiography.

As a result, the concentrates derived from the enzyme proteins obtained from cultivation of pUCO5 and pUCP6 provided spots near to position of methyl arachidate, which is formed by two carbon-extension of stearic acid.

To identify the spots near to the position of methyl arachidate, radio gas chromatography (RGLC) was carried out using 2m of glass column of 5% synchrome E-71. The detection was carried out with FID (N₂ 60 ml/min.) and an aeration type proportional counter (3,400V) at RI side (CH₄ 250 ml/min.). An amount of about two thirds of the product obtained by at one run of the reaction was applied to the RGLC. As a result, the protein products expressed by the plasmids pUCO5 and pUCP6, which comprise ORF 7 and ORF 8 respectively, provided peak corresponding to methyl arachidate. Radioactivity of the peak is shown in Table 7. Each of these ORFs has an ability to convert stearic acid (C₁₈) to arachidic acid (C₂₀) by 2-carbon extension.

**Table 7**

| Plasmid | SEQ ID NO | CPM |
|---|---|---|
| pUCP2 | 6 | 15.8 |
| pUCO5 | 12 | 42.0 |
| pUCP6 | 14 | 29.0 |
| pUCP7 | 16 | 9.0 |
| pUCP8 | 18 | 9.2 |

SEQUENCE LISTING
SEQ ID NO: 1
Sequence Length: 37895
Sequence Type: Nucleic acid
Strandedness: Double strand
Topology: Linear
Molecular Type: Genomic DNA
Source: Shewanella putrefaciens SCRC-2874 (FERM BP-1625)
Sequence
GATCTCTTAC AAAGAAACTA TCTCAATGTG AATTTAACCT TAATTCCGTT TAATTACGGC 60
CTGATAGAGC ATCACCCAAT CAGCCATAAA ACTGTAAAGT GGGTACTCAA AGGTGGCTGG 120
GCGATTCTTC TCAAATACAA AGTGCCCAAC CCAAGCAAAT CCATATCCGA TAACAGGTAA 180
AAGTAGCAAT AAACCCCAGC GCTGAGTTAG TAATACATAA GCGAATAATA GGATCACTAA 240
ACTACTGCCG AAATAGTGTA ATATTCGACA GTTTCTATGC TGATGTTGAG ATAAATAAAA 300
AGGGTAAAAT TCAGCAAAAG AACGATAGCG CTTACTCATT ACTCACACCT CGGTAAAAAA 360
GCAACTCGCC ATTAACTTGG CCAATCGTCA GTTGTTCTAT CGTCTCAAAG TTATGCCGAC 420
TAAATAACTC TATATGTGCA TTATGATTAG CAAAAACTCC GATACCATCA AGATGAAGTT 480
GTTCATCACA CCAACTCAAA ACTGCGTCGA TAAGCTTACT GCCATAGCCC TTGCCTTGCT 540
CCACATTTGC GATAGCAATA AACTGTAAAA TGCCACATTG GCCACTTGGT AAGCTCTCTA 600
TAATCTGATT TTCTTTGTTA ATAAGTGCCT GAGTTGAATA CCAACCAGTA CTTAACAACA 660
TCTTTAAACG CCAATGCCAA AAACGCGCTT CACCTAAGGG AACCTGCTGA GTCACTATGC 720
AGGCTACGCC TATCAATCTA TCCCCAACGA ACATACCAAT AAGTGCTTGC TCCTGTTGCC 780
AGAGCTCATT GAGTTCTTCT CGAATAGCCC CGCGAAGCTT TTGCTCATAC TGCGCTTGAT 840
CACCACTAAA AAGTGTTTCG ATAAAAAAGG GATCATCATG ATAGGCGTTA TAGAGAATAG 900
AGGCTGCTAT GCGTAAATCT TCTGCCGTGA GATAAACTGC ACGACACTCT TCCATGGCTT 960
GATCTTCCAT TGTTATTGTC CTTGACCTTG ATCACACAAC ACCAATGTAA CAAGACTGTA 1020
TAGAAGTGCA ATTAATAATC AATTCGTGCA TTAAGCAGGT CAGCATTTCT TTGCTAAACA 1080
AGCTTTATTG GCTTTGACAA AACTTTGCCT AGACTTTAAC GATAGAAATC ATAATGAAAG 1140
AGAAAAGCTA CAACCTAGAG GGGAATAATC AAACAACTGC TAAGATCTAG ATAATGTAAT 1200
AAACACCGAG TTTATCGACC ATACTTAGAT AGAGTCATAG CAACGAGAAT AGTTATGGAT 1260
ACAACGCCGC AAGATCTATC ACACCTGTTT TTACAGCTAG GATTAGCAAA TGATCAACCC 1320
GCAATTGAAC AGTTTATCAA TGACCATCAA TTAGCGGACA ATATATTGCT ACATCAAGCA 1380
AGCTTTTGGA GCCCATCGCA AAAGCACTTC TTAATTGAGT CATTTAATGA AGATGCCCAG 1440
TGGACCGAAG TCATCGACCA CTTAGACACC TTATTAAGAA AAAACTAACC ATTACAACAG 1500
CAACTTTAAA TTTTGCCGTA AGCCATCTCC CCCCACCCCA CAACAGCGTT GTTGCTTATG 1560
ACCACTGGAG TACATTCGTC TTTAGTCGTT TTACCATCAC CATGGGTACG TTGAGTGCGA 1620
TAAAAAAGCA CATAAACTTC TTTATCGGCC TGAATATAGG CTTCGTTAAA ATCAGCTGTT 1680

CCCATTAAAG TAACCACTTG CTCTTTACTC ATGCCTAGAG ATATCTTTGT CAAATTGTCA 1740
CGGTTTTTAT CTTGAGTTTT CTCCCAAGCA CCGTGATTAT CCCAGTCAGA TTCCCCATCA 1800
CCAACATTGA CCACACAGCC CGTTAGCCCT AAGCTTGCAA TCCCAAAACA TGCTAAACCT 1860
AATAATTTAT TTTTCATTTT AACTTCCTGT TATGACATTA TTTTTGCTTA GAAGAAAAGC 1920
AACTTACATG CCAAAACACA AGCTGTTGTT TTAAATGACT TTATTTATTA TTAGCCTTTT 1980
AGGATATGCC TAGAGCAATA ATAATTACCA ATGTTTAAGG AATTTGACTA ACTATGAGTC 2040
CGATTGAGCA AGTGCTAACA GCTGCTAAAA AAATCAATGA ACAAGGTAGA GAACCAACAT 2100
TAGCATTGAT TAAAACCAAA CTTGGTAATA GCATCCCAAT GCGCGAGTTA ATCCAAGGTT 2160
TGCAACAGTT TAAGTCTATG AGTGCAGAAG AAAGACAAGC AATACCTAGC AGCTTAGCAA 2220
CAGCAAAAGA AACTCAATAT GGTCAATCAA GCTTATCTCA ATCTGAACAA GCTGATAGGA 2280
TCCTCCAGCT AGAAAACGCC CTCAATGAAT TAAGAAACGA ATTTAATGGG CTAAAAAGTC 2340
AATTTGATAA CTTACAACAA AACCTGATGA ATAAAGAGCC TGACACCAAA TGCATGTAAT 2400
TGAACTACGA TTTGAATGTT TTGATAACAC CACGATTACT GCAGCAGAAA AAGCCATTAA 2460
TGGTTTGCTT GAAGCTTATC GAGCCAATGG CCAGGTTCTA GGTCGTGAAT TTGCCGTTGC 2520
ATTTAACGAT GGTGAGTTTA AAGCACGCAT GTTAACCCCA GAAAAAAGCA GCTTATCTAA 2580
ACGCTTTAAT AGTCCTTGGG TAAATAGTGC ACTCGAAGAG CTAACCGAAG CCAAATTGCT 2640
TGCGCCACGT GAAAAGTATA TTGGCCAAGA TATTAATTCT GAAGCATCTA GCCAAGACAC 2700
ACCAAGTTGG CAGCTACTTT ACACAAGTTA TGTGCACATG TGCTCACCAC TAAGAAATGG 2760
CGACACCTTG CAGCCTATTC CACTGTATCA AATTCCAGCA ACTGCCAACG GCGATCATAA 2820
ACGAATGATC CGTTGGCAAA CAGAATGGCA AGCTTGTGAT GAATTGCAAA TGGCCGCAGC 2880
TACTAAAGCT GAATTTGCCG CACTTGAAGA GCTAACCAGT CATCAGAGTG ATCTATTTAG 2940
GCGTGGTTGG GACTTACGTG GCAGAGTCGA ATACTTGACG AAAATTCCGA CCTATTACTA 3000
TTTATACCGT GTTGGCGGTG AAAGCTTAGC AGTAGAAAAG CAGCGCTCTT GTCCTAAGTG 3060
TGGCAGTCAA GAATGGCTGC TCGATAAACC ATTATTGGAT ATGTTCCATT TTCGCTGTGA 3120
CACCTGCCGC ATCGTATCTA ATATCTCTTG GGACCATTTA TAACTCTTCC GAGTCTTATC 3180
ACACTAGAGT TTAGTCAGCA TAAAAATGGC GCTTATATTT CAATTAAAAG AAATATAAGC 3240
GCCATTTTCA TCGATACTAT ATATCAGCAG ACTATTTTCC GCGTAAATTA GCCCACATTA 3300
ATTTCATTCT TTGCCAGATC CCTGGATGAT CTAGTTGTGG CATCGACTCT TCAATAGGTT 3360
TAACCGCAGG TGTAACCCTT GGAGTCAATT CGTTTATAAA CTCGTTTAAA CTGTCACTTA 3420
ATTTAACGCT TTGTACTTCA CCTGGAATTT CAATCCATAC GCTGCCATCA CTATTATTAA 3480
CCGTCAACAT TTTATCTTCA TCATCAAGAA TACCAATAAA CCAAGTCGGC TCTTGCTTAA 3540
GCTTTCTCTT CATCATTAAA TGACCAATGA TGTTTTGTTG TAAGTATTCA AAATCAGTTT 3600
GATCCCACAC TTGGATTAGC TCACCTTGGC CCCATTGTGA GTCAAAAAAT AGCGGTGCAG 3660
AAAAATGACT GCCAAAAAAT GGATTAATTT CTGCAGATAA TGTCATTTCA AGTGCTGTTT 3720
CAACATTAGC AAATTCACCA GGTTGTTGAC GTACAACCGA TTGCCAAAAC ACTGCGCCAT 3780
CGGAGCCCGC TTCGGCGACA ACACACTCAG ACTTTTGTCC TTGCGCATAA TATCTTGGCT 3840
GTTCACCAAG CTTATCCATG TAGGCTTGTT GATATTTAGA TAAAAAAAGA TCTAAAGCAG 3900

GTAAAGAAGA CACTTAAGCC AGTTCCAAAA TCAGTTATAA TAGGGGTCTA TTTTGACATG 3960
GAAACCGTAT TGATGACACA ACATCATGAT CCCTACAGTA ACGCCCCCGA ACTTTCTGAA 4020
TTAACTTTAG GAAAGTCGAC CGGTTATCAA GAGCAGTATG ATGCATCTTT ACTACAAGCG 4080
TGCCGCGTAA ATTAAACCGT GATGCTATCG GTCTAACCAA TGAGCTACCT TTTCATGGCT 4140
GTGATATTTG GACTGGCTAC GAACTGTCTT GGCTAAATGC TAAAGGCAAG CCAATGATTG 4200
CTATTGCAGA CTTTAACCTA AGTTTTGATA GTAAAAATCT GATCGAGTCT AAGTCGTTTA 4260
AGCTGTATTT AAACAGCTAT AACCAAACAC GATTTGATAG CGTTCAAGCG GTTCAAGAAC 4320
GTTTAACTGA AGACTTAAGC GCCTGTGCCC AAGGCACAGT TACGGTAAAA GTGATTGAAC 4380
CTAAGCAATT TAACCACCTG AGAGTGGTTG ATATGCCAGG TACCTGCATT GACGATTTAG 4440
ATATTGAAGT TGATGACTAT AGCTTTAACT CTGACTATCT CACCGACAGT GTTGATGACA 4500
AAGTCATGGT TGCTGAAACG CTAACGTCAA ACTTATTGAA ATCAAACTGC CTAATCACTT 4560
CTCAGCCTGA CTGGGGTACA GTGATGATCC GTTATCAAGG GCCTAAGATA GACCGTGAAA 4620
AGCTACTTAG ATATCTGATT TCATTTAGAC AGCACAATGA ATTTCATGAG CAGTGTGTTG 4680
AGCGTATATT TGTTGATTTA AAGCACTATT GCCAATGTGC CAAACTTACT GTCTATGCAC 4740
GTTATACCCG CCGTGGTGGT TTAGATATCA ACCCATATCG TAGCGACTTT GAAAACCCTG 4800
CAGAAAATCA GCGCCTAGCG AGACAGTAAT TGATTGCAGT ACCTACAAAA AACAATGCCT 4860
ATAAGCCAAG CTTATGGGCA TTTTTATATT ATCAACTTGT CATCAAACCT CAGCCGCCAA 4920
GCCTTTTAGT TTTATCGCTA AATTAAGCCG CTCTCTCAGC CAAATATTTG CAGGATTTTG 4980
CTGTAATTTA TGGCTCCACA CCATGAAATA CTCTATCGGC TCTACCGCAA AAGGTAAGTC 5040
AAATACCTGT AAGCCAAACA GCTTGGCATA TTCGTCAGTG TGGGCTTTTG ACGCGATAGC 5100
TAACGCATCA CTTTTTGAGG CAACCGACAT CATACTTAAT ATTGATGATT GCTCGCTGTG 5160
CATTTGCCTT GCCGGTAACA CCTGTTTAGT CAGCAAGTCG GCAACACTTA AATTGTAGCG 5220
GCGCATCTTA AAAATAATAT GCTTTTCATT AAAGTATTGC TCTTGCGTCA ACCCACCTTG 5280
GATCCTTGGG TGAGCATTTC GTGCCACACA AACTAATTTA TCCTGCATTA CTTTTTGACT 5340
CTTAAATGCC GCAGATTCTG GCAGCCAAAT ATCTAAGGCT AAATCCACCT TTTCTAGTTG 5400
TAGGTCCATC TGCAACTCTT CTTCAATGAG CGGCGGCTCA CGAAATACAA TATTAATTGC 5460
AGTGCCCTGT AACACTTGCT CAATTTGATC TTGCAAGAGT TGTATTGCCG ACTCGCTGGC 5520
ATACACATAA AAAGTTCGCT CACTTGAAGT GGGGTCAAAT GCTTCAAAGC TAGTCGCAAC 5580
TTGCTCAATT GTTGACATAG CGCCCGCGAG CTGTTGATAA AGCGTCATCG CACTTGCGGT 5640
AGGTTTAACT CCCCTACCCA CTCGAGTAAA CAACTCTTCT CCAACAATAC TTTTTAGCCT 5700
CGAAATCGCA TTACTAACCG ACGACTGAGT CAAATCCAGC TCTTCTGCCG CCCGGCTAAA 5760
AGATGAGGTG CGATACACCG CAGTAAAAAC GCGAAATAAA TTAAGATCAA AAGCTTTTTG 5820
CTGCGACATA AATCAGCTAT CTCCTTATCC TTATCCTTAT CCTTATAAAA AGTTAGCTCC 5880
AGAGCACTCT AGCTCAAAAA CAACTCAGCG TATTAAGCCA ATATTTTGGG AACTCAATTA 5940
ATATTCATAA TAAAAGTATT CATAATATAA ATACCAAGTC ATAATTTAGC CCTAATTATT 6000
AATCAATTCA AGTTACCTAT ACTGGCCTCA ATTAAGCAAA TGTCTCATCA GTCTCCCTGC 6060
AACTAAATGC AATATTGAGA CATAAAGCTT TGAACTGATT CAATCTTACG AGGGTAACTT 6120

ATGAAACAGA CTCTAATGGC TATCTCAATC ATGTCGCTTT TTTCATTCAA TGCGCTAGCA 6180
GCGCAACATG AACATGACCA CATCACTGTT GATTACGAAG GGAAAGCCGC AACAGAACAC 6240
ACCATAGCTC ACAACCAAGC TGTAGCTAAA ACACTTAACT TTGCCGACAC GCGTGCATTT 6300
GAGCAATCGT CTAAAAATCT AGTCGCCAAG TTTGATAAAG CAACTGCCGA TATATTACGT 6360
GCCGAATTTG CTTTTATTAG CGATGAAATC CCTGACTCGG TTAACCCGTC TCTCTACCGT 6420
CAGGCTCAGC TTAATATGGT GCCTAATGGT CTGTATAAAG TGAGCGATGG CATTTACCAG 6480
GTCCGCGGTA CCGACTTATC TAACCTTACA CTTATCCGCA GTGATAACGG TTGGATAGCA 6540
TACGATGTTT TGTTAACCAA AGAAGCAGCA AAAGCCTCAC TACAATTTGC GTTAAAGAAT 6600
CTACCTAAAG ATGGCGATTT ACCCGTTGTT GCGATGATTT ACTCCCATAG CCATGCGGAC 6660
CACTTTGGCG GAGCTCGCGG TGTTCAAGAG ATGTTCCCTG ATGTCAAAGT CTACGGCTCA 6720
GATAACATCA CTAAAGAAAT TGTCGATGAG AACGTACTTG CCGGTAACGC CATGAGCCGC 6780
CGCGCAGCTT ATCAATACGG CGCAACACTG GGCAAACATG ACCACGGTAT TGTTGATGCT 6840
GCGCTAGGTA AAGGTCTATC AAAAGGTGAA ATCACTTACG TCGCCCCAGA CTACACCTTA 6900
AACAGTGAAG GCAAATGGGA AACGCTGACG ATTGATGGTC TAGAGATGGT GTTTATGGAT 6960
GCCTCGGGCA CCGAAGCTGA GTCAGAAATG ATCACTTATA TTCCCTCTAA AAAAGCGCTC 7020
TGGACGGCGG AGCTTACCTA TCAAGGTATG CACAACATTT ATACGCTGCG CGGCGCTAAA 7080
GTACGTGATG CGCTCAAGTG GTCAAAAGAT ATCAACGAAA TGATCAATGC CTTTGGTCAA 7140
GATGTCGAAG TGCTGTTTGC CTCGCACTCT GCGCCAGTGT GGGGTAACCA AGCGATCAAC 7200
GATTTCTTAC GCCTACAGCG TGATAACTAC GGCCTAGTGC ACAATCAAAC CTTGAGACTT 7260
GCCAACGATG GTGTCGGTAT ACAAGATATT GGCGATGCGA TTCAAGACAC GATTCCAGAG 7320
TCTATCTACA AGACGTGGCA TACCAATGGT TACCACGGCA CTTATAGCCA TAACGCTAAA 7380
GCGGTTTATA ACAAGTATCT AGGCTACTTC GATATGAACC CAGCCAACCT TAATCCGCTG 7440
CCAACCAAGC AAGAATCTGC CAAGTTTGTC GAATACATGG GCGGCGCAGA TGCCGCAATT 7500
AAGCGCGCTA AAGATGATTA CGCTCAAGGT GAATACCGCT TTGTTGCAAC GGCATTAAAT 7560
AAGGTGGTGA TGGCCGAGCC AGAAAATGAC TCCGCTCGTC AATTGCTAGC CGATACCTAT 7620
GAGCAACTTG GTTATCAAGC AGAAGGGGCT GGCTGGAGAA ACATTTACTT AACTGGCGCA 7680
CAAGAGCTAC GAGTAGGTAT TCAAGCTGGC GCGCCTAAAA CCGCATCGGC AGATGTCATC 7740
AGTGAAATGG ACATGCCGAC TCTATTTGAC TTCCTCGCGG TGAAGATTGA TAGTCAACAG 7800
GCGGCTAAGC ACGGCTTAGT TAAGATGAAT GTTATCACCC CTGATACTAA AGATATTCTC 7860
TATATTGAGC TAAGCAACGG TAACTTAAGC AACGCAGTGG TCGACAAAGA GCAAGCAGCT 7920
GACGCAAACC TTATGGTTAA TAAAGCTGAC GTTAACCGCA TCTTACTTGG CCAAGTAACC 7980
CTAAAAGCGT TATTAGCCAG CGGCGATGCC AAGCTCACTG GTGATAAAAC GGCATTTAGT 8040
AAAATAGCCG ATAGCATGGT CGAGTTTACA CCTGACTTCG AAATCGTACC AACGCCTGTT 8100
AAATGAGGCA TTAATCTCAA CAAGTGCAAG CTAGACATAA AAATGGGGCG ATTAGACGCC 8160
CCATTTTTTA TGCAATTTTG AACTAGCTAG TCTTAGCTGA AGCTCGAACA ACAGCTTTAA 8220
AATTCACTTC TTCTGCTGCA ATACTTATTT GCTGACACTG ACCAATACTC AGTGCAAAAC 8280
GATAACTATC ATCAAGATGG CCCAGTAAAC AATGCCAATT ATCAGCAGCG TTCATTTGCT 8340

GTTCTTTAGC CTCAATCAAA CCTAAACCAG ACTTTTGTGG CTCAGCGTTA GGCTTATTAG 8400
AACTCGACTC TAGTAAAGCA AGACCAATAT CTTGTTTTAA CAAAACCTGT CGCTGATTAA 8460
GTTGATGCTC AACCTTGTGA TCCGCAATAG CATCGGAAAT ATCAACACAA TGGCTCAAGC 8520
TTTTAGGTGC ATTAACTCCA AGAAAAGTTT CGCTCAGTGC AGAGAAGTCA AACGCAAAAG 8580
ATTTTAGCGA TAATGCCAGC CCAAGTCCTT TCGCTTTAAT GTAAGACTCC TTGAGCGCCC 8640
ACAAATCAAA AAAGCGGTCT CGCTGCAAGG CCTCTGGTAA CGCTAACAAG GCTCGCTTTT 8700
CTGATTCAGA GAAATAATGA CTAAGAATAG AGTGGATATT GGTGCTGTTA CGGCAACGCT 8760
CAATGTCGAC GCCAAACTCA ATACTAGCAG AGTCAGTTTC CTCCTTGCTT GCCTGACTGG 8820
CGCCTTTATT ATCAGCAGTG CAAATGCCTA CTAATAGCCA ATCTCCACTA TGACTCACAT 8880
TAAAGTGGAC CCCGGTTTGA GCAAATTGCG CATCACTCAA TCTAGGCTTA CCTTTGTCGC 8940
CATATTCAAA GCGCCATTCA TTGGGGCGTA TTTCACTATG TTGTGACAAT AAAGCGCGCA 9000
AATAGCCTCT TACCATTAAA CCTTGAGTTT TAGCTTCTTG TTTAATGTAG CGATTAACCT 9060
TAATTAACTC ATCTTCAGGC AGCCATGACT TAACCAACTC TGTAGTCTGG TTATCGCACT 9120
CTTGTATTGT TAACGGACAG AAGTATAAGG AAATCAATCG AGAAGTTAGC AATTTTTCAG 9180
GACACTCTTT AAAGCAACAA ACATAACCCC TATTTTTACC AATTTAAGAT CAAAACTAAA 9240
GCCAAAACTA ATTGAGAATA GTGTCAAACT AGCTTTAAAG GAAAAAAATA TAAAAAGAAC 9300
ATTATACTTG TATAAATTAT TTTACACACC AAAGCCATGA TCTTCACAAA ATTAGCTCCC 9360
TCTCCCTAAA ACAAGATTGA ATAAAAAAAT AAACCTTAAC TTTCATATAG ATAAAACAAA 9420
CCAATGGGAT AAAGTATATT GAATTCATTT TTAAGGAAAA ATTCAAATTG AATTCAAGCT 9480
CTTCAGTAAA AGCATATTTT GCCGTTAGTG TGAAAAAAAA CAAATTTAAA AACCAACATA 9540
GAACAAATAA GCAGACAATA AAACCAAGGC GCAACACAAA CAACGCGCTT ACAATTTTCA 9600
CAAAAAAGCA ACAAGAGTAA CGTTTAGTAT TTGGATATGG TTATTGTAAT TGAGAATTTT 9660
ATAACAATTA TATTAAGGGA ATGAGTATGT TTTTAAATTC AAAACTTTCG CGCTCAGTCA 9720
AACTTGCCAT ATCCGCAGGC TTAACAGCCT CGCTAGCTAT GCCTGTTTTT GCAGAAGAAA 9780
CTGCTGCTGA AGAACAAATA GAAAGAGTCG CAGTGACCGG ATCGCGAATC GCTAAAGCAG 9840
AGCTAACTCA ACCAGCTCCA GTCGTCAGCC TTTCAGCCGA AGAACTGACA AAATTTGGTA 9900
ATCAAGATTT AGGTAGCGTA CTAGCAGAAT TACCTGCTAT TGGTGCAACC AACACTATTA 9960
TTGGTAATAA CAATAGCAAC TCAAGCGCAG GTGTTAGCTC AGCAGACTTG CGTCGTCTAG 10020
GTGCTAACAG AACCTTAGTA TTAGTCAACG GTAAGCGCTA CGTTGCCGGC CAACCGGGCT 10080
CAGCTGAGGT AGATTTGTCA ACTATACCAA CTAGCATGAT CTCGCGAGTT GAGATTGTAA 10140
CCGGCGGTGC TTCAGCAATT TATGGTTCGG ACGCTGTATC AGGTGTTATC AACGTTATCC 10200
TTAAAGAAGA CTTTGAAGGC TTTGAGTTTA ACGCACGTAC TAGCGGTTCT ACTGAAAGTG 10260
TAGGCACTCA AGAGCACTCT TTTGACATTT TGGGTGGTGC AAACGTTGCA GATGGACGTG 10320
GTAATGTAAC CTTCTACGCA GGTTATGAAC GTACAAAAGA AGTCATGGCT ACCGACATTC 10380
GCCAATTCGA TGCTTGGGGA ACAATTAAAA ACGAAGCCGA TGGTGGTGAA GATGATGGTA 10440
TTCCAGACAG ACTACGTGTA CCACGAGTTT ATTCTGAAAT GATTAATGCT ACCGGTGTTA 10500
TCAATGCATT TGGTGGTGGA ATTGGTCGCT CAACCTTTGA CAGTAACGGC AATCCTATTG 10560

CACAACAAGA ACGTGATGGG ACTAACAGCT TTGCATTTGG TTCATTCCCT AATGGCTGTG 10620
ACACATGTTT CAACACTGAA GCATACGAAA ACTATATTCC AGGGGTAGAA AGAATAAACG 10680
TTGGCTCATC ATTCAACTTT GATTTTACCG ATAACATTCA ATTTTACACT GACTTCAGAT 10740
ATGTAAAGTC AGATATTCAG CAACAATTTC AGCCTTCATT CCGTTTTGGT AACATTAATA 10800
TCAATGTTGA AGATAACGCC TTTTTGAATG ACGACTTGCG TCAGCAAATG CTCGATGCGG 10860
GTCAAACCAA TGCTAGTTTT GCCAAGTTTT TTGATGAATT AGGAAATCGC TCAGCAGAAA 10920
ATAAACGCGA ACTTTTCCGT TACGTAGGTG GCTTTAAAGG TGGCTTTGAT ATTAGCGAAA 10980
CCATATTTGA TTACGACCTT TACTATGTTT ATGGCGAGAC TAATAACCGT CGTAAAACCC 11040
TTAATGACCT AATTCCTGAT AACTTTGTCG CAGCTGTCGA CTCTGTTATT GATCCTGATA 11100
CTGGCTTAGC AGCGTGTCGC TCACAAGTAG CAAGCGCTCA AGGCGATGAC TATACAGATC 11160
CCGCGTCTGT AAATGGTAGC GACTGTGTTG CTTATAACCC ATTTGGCATG GGTCAAGCTT 11220
CAGCAGAAGC CCGCGACTGG GTTTCTGCTG ATGTGACTCG TGAAGACAAA ATAACTCAAC 11280
AAGTGATTGG TGGTACTCTC GGTACCGATT CTGAAGAACT ATTTGAGCTT CAAGGTGGTG 11340
CAATCGCTAT GGTTGTTGGT TTTGAATACC GTGAAGAAAC GTCTGGTTCA ACAACCGATG 11400
AATTTACTAA AGCAGGTTTC TTGACAAGCG CTGCAACGCC AGATTCTTAT GGCGAATACG 11460
ACGTGACTGA GTATTTTGTT GAGGTGAACA TCCCAGTACT AAAAGAATTA CCTTTTGCAC 11520
ATGAGTTGAG CTTTGACGGT GCATACCGTA ATGCTGATTA CTCACATGCC GGTAAGACTG 11580
AAGCATGGAA AGCTGGTATG TTCTACTCAC CATTAGAGCA ACTTGCATTA CGTGGTACGG 11640
TAGGTGAAGC AGTACGAGCA CCAAACATTG CAGAAGCCTT TAGTCCACGC TCTCCTGGTT 11700
TTGGCCGCGT TTCAGATCCA TGTGATGCAG ATAACATTAA TGACGATCCG GATCGCGTGT 11760
CAAACTGTGC AGCATTGGGG ATCCCTCCAG GATTCCAAGC TAATGATAAC GTCAGTGTAG 11820
ATACCTTATC TGGTGGTAAC CCAGATCTAA AACCTGAAAC ATCAACATCC TTTACAGGTG 11880
GTCTTGTTTG GACACCAACG TTTGCTGACA ATCTATCATT CACTGTCGAT TATTATGATA 11940
TTCAAATTGA GGATGCTATT TTGTCAGTAG CCACCCAGAC TGTGGCTGAT AACTGTGTTG 12000
ACTCAACTGG CGGACCTGAC ACCGACTTCT GTAGTCAAGT TGATCGTAAT CCAACGACCT 12060
ATGATATTGA ACTTGTTCGC TCTGGTTATC TAAATGCCGC GGCATTGAAT ACCAAAGGTA 12120
TTGAATTTCA AGCTGCATAC TCATTAGATC TAGAGTCTTT CAACGCGCCT GGTGAACTAC 12180
GCTTCAACCT ATTGGGGAAC CAATTACTTG AACTAGAACG TCTTGAATTC CAAAATCGTC 12240
CTGATGAGAT TAATGATGAA AAAGGCGAAG TAGGTGATCC AGAGCTGCAG TTCCGCCTAG 12300
GCATCGATTA CCGTCTAGAT GATCTAAGTG TTAGCTGGAA CACGCGTTAT ATTGATAGCG 12360
TAGTAACTTA TGATGTCTCT GAAAATGGTG GCTCTCCTGA AGATTTATAT CCAGGCCACA 12420
TAGGCTCAAT GACAACTCAT GACTTGAGCG CTACATACTA CATCAATGAG AACTTCATGA 12480
TTAACGGTGG TGTACGTAAC CTATTTGACG CACTTCCACC TGGATACACT AACGATGCGC 12540
TATATGATCT AGTTGGTCGC CGTGCATTCC TAGGTATTAA GGTAATGATG TAATTAATTA 12600
TTACGCCTCT AACTAATAAA AATGCAATCT CTTCGTAGAG ATTGCATTTT TTTATGAAAT 12660
CCAATCTTAA ACTGGTTCTC CGAGCATCTT ACGCCTTAAA AACCCCGCCC CTCAATGTAA 12720
CGCCAAAGTT AATTGCTTAC ACGCACTTAC ACAAACGAAC AATTTCATTA ACACGAGACA 12780

CAGCTCACGC TTTTTATTTT ACCCTTGATT TTACTACATA AAATTGCGTT TTAGCGCACA 12840
AGTGTTCTCC CAAGCTGGTC GTATCTGTAA TTATTCAGTC CCAGGTGATT GTATTGACCC 12900
ATAAGCTCAG GTAGTCTGCT CTGCCATTAG CTAAACAATA TTGACAAAAT GGCGATAAAA 12960
TGTGGCTTAG CGCTAAGTTC ACCGTAAGTT TTATCGGCAT TAAGTCCCAA CAGATTATTA 13020
ACGGAAACCC GCTAAACTGA TGGCAAAAAT AAATAGTGAA CACTTGGATG AAGCTACTAT 13080
TACTTCGAAT AAGTGTACGC AAACAGAGAC TGAGGCTCGG CATAGAAATG CCACTACAAC 13140
ACCTGAGATG CGCCGATTCA TACAAGAGTC GGATCTCAGT GTTAGCCAAC TGTCTAAAAT 13200
ATTAAATATC AGTGAAGCTA CCGTACGTAA GTGGCGCAAG CGTGACTCTG TCGAAAACTG 13260
TCCTAATACC CCGCACCATC TCAATACCAC GCTAACCCCT TTGCAAGAAT ATGTGGTTGT 13320
GGGCCTGCGT TATCAATTGA AAATGCCATT AGACAGATTG CTCAAAGCAA CCCAAGAGTT 13380
TATCAATCCA AACGTGTCGC GCTCAGGTTT AGCAAGATGT TTGAAGCGTT ATGGCGTTTC 13440
ACGGGTGAGT GATATCCAAA GCCCACACGT ACCAATGCGC TACTTTAATC AAATTCCAGT 13500
CACTCAAGGC AGCGATGTGC AAACCTACAC CCTGCACTAT GAAACGCTGG CAAAAACCTT 13560
AGCCTTACCT AGTACCGATG GTGACAATGT GGTGCAAGTG GTGTCTCTCA CCATTCCACC 13620
AAAGTTAACC GAAGAAGCAC CCAGTTCAAT TTTGCTCGGC ATTGATCCTC ATAGCGACTG 13680
GATCTATCTC GACATATACC AAGATGGCAA TACACAAGCC ACGAATAGAT ATATGGCTTA 13740
TGTGCTAAAA CACGGGCCAT TCCATTTACG AAAGTTACTC GTGCGTAACT ATCACACCTT 13800
TTTACAGCGC TTTCCTGGAG CGACGCAAAA TCGCCGCCCC TCTAAAGATA TGCCTGAAAC 13860
AATCAACAAG ACGCCTGAAA CACAGGCACC CAGTGGAGAC TCATAATGAG CCAGACCTCT 13920
AAACCTACAA ACTCAGCAAC TGAGCAAGCA CAAGACTCAC AAGCTGACTC TCGTTTAAAT 13980
AAACGACTAA AAGATATGCC AATTGCTATT GTTGGCATGG CGAGTATTTT TGCAAACTCT 14040
CGCTATTTGA ATAAGTTTTG GGACTTAATC AGCGAAAAAA TTGATGCGAT TACTGAATTA 14100
CCATCAACTC ACTGGCAGCC TGAAGAATAT TACGACGCAG ATAAAACCGC AGCAGACAAA 14160
AGCTACTGTA AACGTGGTGG CTTTTTGCCA GATGTAGACT TCAACCCAAT GGAGTTTGGC 14220
CTGCCGCCAA ACATTTTGGA ACTGACCGAT TCATCGCAAC TATTATCACT CATCGTTGCT 14280
AAAGAAGTGT TGGCTGATGC TAACTTACCT GAGAATTACG ACCGCGATAA AATTGGTATC 14340
ACCTTAGGTG TCGGCGGTGG TCAAAAAATT AGCCACAGCC TAACAGCGCG TCTGCAATAC 14400
CCAGTATTGA AGAAAGTATT CGCCAATAGC GGCATTAGTG ACACCGACAG CGAAATGCTT 14460
ATCAAGAAAT TCCAAGACCA ATATGTACAC TGGGAAGAAA ACTCGTTCCC AGGTTCACTT 14520
GGTAACGTTA TTGCGGGCCG TATCGCCAAC CGCTTCGATT TTGGCGGCAT GAACTGTGTG 14580
GTTGATGCTG CCTGTGCTGG ATCACTTGCT GCTATGCGTA TGGCGCTAAC AGAGCTAACT 14640
GAAGGTCGCT CTGAAATGAT GATCACCGGT GGTGTGTGTA CTGATAACTC ACCCTCTATG 14700
TATATGAGCT TTTCAAAAAC GCCCGCCTTT ACCACTAACG AAACCATTCA GCCATTTGAT 14760
ATCGACTCAA AAGGCATGAT GATTGGTGAA GGTATTGGCA TGGTGGCGCT AAAGCGTCTT 14820
GAAGATGCAG AGCGCGATGG CGACCGCATT TACTCTGTAA TTAAAGGTGT GGGTGCATCA 14880
TCTGACGGTA AGTTTAAATC AATCTATGCC CCTCGCCCAT CAGGCCAAGC TAAAGCACTT 14940
AACCGTGCCT ATGATGACGC AGGTTTTGCG CCGCATACCT TAGGTCTAAT TGAAGCTCAC 15000

GGAACAGGTA CTGCAGCAGG TGACGCGGCA GAGTTTGCCG GCCTTTGCTC AGTATTTGCT 15060
GAAGGCAACG ATACCAAGCA ACACATTGCG CTAGGTTCAG TTAAATCACA AATTGGTCAT 15120
ACTAAATCAA CTGCAGGTAC AGCAGGTTTA ATTAAAGCTG CTCTTGCTTT GCATCACAAG 15180
GTACTGCCGC CGACCATTAA CGTTAGTCAG CCAAGCCCTA AACTTGATAT CGAAAACTCA 15240
CCGTTTTATC TAAACACTGA GACTCGTCCA TGGTTACCAC GTGTTGATGG TACGCCGCGC 15300
CGCGCGGGTA TTAGCTCATT TGGTTTTGGT GGCACTAACT TCCATTTTGT ACTAGAAGAG 15360
TACAACCAAG AACACAGCCG TACTGATAGC GAAAAAGCTA AGTATCGTCA ACGCCAAGTG 15420
GCGCAAAGCT TCCTTGTTAG CGCAAGCGAT AAAGCATCGC TAATTAACGA GTTAAACGTA 15480
CTAGCAGCAT CTGCAAGCCA AGCTGAGTTT ATCCTCAAAG ATGCAGCAGC AAACTATGGC 15540
GTACGTGAGC TTGATAAAAA TGCACCACGG ATCGGTTTAG TTGCAAACAC AGCTGAAGAG 15600
TTAGCAGGCC TAATTAAGCA AGCACTTGCC AAACTAGCAG CTAGCGATGA TAACGCATGG 15660
CAGCTACCTG GTGGCACTAG CTACCGCGCC GCTGCAGTAG AAGGTAAAGT TGCCGCACTG 15720
TTTGCTGGCC AAGGTTCACA ATATCTCAAT ATGGGCCGTG ACCTTACTTG TTATTACCCA 15780
GAGATGCGTC AGCAATTTGT AACTGCAGAT AAAGTATTTG CCGCAAATGA TAAAACGCCG 15840
TTATCGCAAA CTCTGTATCC AAAGCCTGTA TTTAATAAAG ATGAATTAAA GGCTCAAGAA 15900
GCCATTTTGA CCAATACCGC CAATGCCCAA AGCGCAATTG GTGCGATTTC AATGGGTCAA 15960
TACGATTTGT TTACTGCGGC TGGCTTTAAT GCCGACATGG TTGCAGGCCA TAGCTTTGGT 16020
GAGCTAAGTG CACTGTGTGC TGCAGGTGTT ATTTCAGCTG ATGACTACTA CAAGCTGGCT 16080
TTTGCTCGTG GTGAGGCTAT GGCAACAAAA GCACCGGCTA AAGACGGCGT TGAAGCAGAT 16140
GCAGGAGCAA TGTTTGCAAT CATAACCAAG AGTGCTGCAG ACCTTGAAAC CGTTGAAGCC 16200
ACCATCGCTA AATTTGATGG GGTGAAAGTC GCTAACTATA ACGCGCCAAC GCAATCAGTA 16260
ATTGCAGGCC CAACAGCAAC TACCGCTGAT GCGGCTAAAG CGCTAACTGA GCTTGGTTAC 16320
AAAGCGATTA ACCTGCCAGT ATCAGGTGCA TTCCACACTG AACTTGTTGG TCACGCTCAA 16380
GCGCCATTTG CTAAAGCGAT TGACGCAGCC AAATTTACTA AAACAAGCCG AGCACTTTAC 16440
TCAAATGCAA CTGGCGGACT TTATGAAAGC ACTGCTGCAA AGATTAAAGC CTCGTTTAAG 16500
AAACATATGC TTCAATCAGT GCGCTTTACT AGCCAGCTAG AAGCCATGTA CAACGACGGC 16560
GCCCGTGTAT TTGTTGAATT TGGTCCAAAG AACATCTTAC AAAAATTAGT TCAAGGCACG 16620
CTTGTCAACA CTGAAAATGA AGTTTGCACT ATCTCTATCA ACCCTAATCC TAAAGTTGAT 16680
AGTGATCTGC AGCTTAAGCA AGCAGCAATG CAGCTAGCGG TTACTGGTGT GGTACTCAGT 16740
GAAATTGACC CATACCAAGC CGATATTGCC GCACCAGCGA AAAAGTCGCC AATGAGCATT 16800
TCGCTTAATG CTGCTAACCA TATCAGCAAA GCAACTCGCG CTAAGATGGC CAAGTCTTTA 16860
GAGACAGGTA TCGTCACCTC GCAAATAGAA CATGTTATTG AAGAAAAAAT CGTTGAAGTT 16920
GAGAAACTGG TTGAAGTCGA AAAGATCGTC GAAAAAGTGG TTGAAGTAGA GAAAGTTGTT 16980
GAGGTTGAAG CTCCTGTTAA TTCAGTGCAA GCCAATGCAA TTCAAACCCG TTCAGTTGTC 17040
GCTCCAGTAA TAGAGAACCA AGTCGTGTCT AAAAACAGTA AGCCAGCAGT CCAGAGCATT 17100
AGTGGTGATG CACTCAGCAA CTTTTTTGCT GCACAGCAGC AAACCGCACA GTTGCATCAG 17160
CAGTTCTTAG CTATTCCGCA GCAATATGGT GAGACGTTCA CTACGCTGAT GACCGAGCAA 17220

GCTAAACTGG CAAGTTCTGG TGTTGCAATT CCAGAGAGTC TGCAACGCTC AATGGAGCAA 17280
TTCCACCAAC TACAAGCGCA AACACTACAA AGCCACACCC AGTTCCTTGA GATGCAAGCG 17340
GGTAGCAACA TTGCAGCGTT AAACCTACTC AATAGCAGCC AAGCAACTTA CGCTCCAGCC 17400
ATTCACAATG AAGCGATTCA AAGCCAAGTG GTTCAAAGCC AAACTGCAGT CCAGCCAGTA 17460
ATTTCAACAC AAGTTAACCA TGTGTCAGAG CAGCCAACTC AAGCTCCAGC TCCAAAAGCG 17520
CAGCCAGCAC CTGTGACAAC TGCAGTTCAA ACTGCTCCGG CACAAGTTGT TCGTCAAGCC 17580
GCACCAGTTC AAGCCGCTAT TGAACCGATT AATACAAGTG TTGCGACTAC AACGCCTTCA 17640
GCCTTCAGCG CCGAAACAGC CCTGAGCGCA ACAAAAGTCC AAGCCACTAT GCTTGAAGTG 17700
GTTGCTGAGA AAACCGGTTA CCCAACTGAA ATGCTAGAGC TTGAAATGGA TATGGAAGCC 17760
GATTTAGGCA TCGATTCTAT CAAGCGTGTA GAAATTCTTG GCACAGTACA AGATGAGCTA 17820
CCGGGTCTAC CTGAGCTTAG CCCTGAAGAT CTAGCTGAGT GTCGAACGCT AGGCGAAATC 17880
GTTGACTATA TGGGCAGTAA ACTGCCGGCT GAAGGCTCTA TGAATTCTCA GCTGTCTACA 17940
GGTTCCGCAG CTGCGACTCC TGCAGCGAAT GGTCTTTCTG CGGAGAAAGT TCAAGCGACT 18000
ATGATGTCTG TGGTTGCCGA AAAGACTGGC TACCCAACTG AAATGCTAGA GCTTGAAATG 18060
GATATGGAAG CCGATTTAGG CATAGATTCT ATCAAGCGCG TTGAAATTCT TGGCACAGTA 18120
CAAGATGAGC TACCGGGTCT ACCTGAGCTT AGCCCTGAAG ATCTAGCTGA GTGTCGTACT 18180
CTAGGCGAAA TCGTTGACTA TATGAACTCT AAACTCGCTG ACGGCTCTAA GCTGCCGGCT 18240
GAAGGCTCTA TGAATTCTCA GCTGTCTACA AGTGCCGCAG CTGCGACTCC TGCAGCGAAT 18300
GGTCTCTCTG CGGAGAAAGT TCAAGCGACT ATGATGTCTG TGGTTGCCGA AAAGACTGGC 18360
TACCCAACTG AAATGCTAGA ACTTGAAATG GATATGGAAG CTGACCTTGG CATCGATTCA 18420
ATCAAGCGCG TTGAAATTCT TGGCACAGTA CAAGATGAGC TACCGGGTTT ACCTGAGCTA 18480
AATCCAGAAG ATTTGGCAGA GTGTCGTACT CTTGGCGAAA TCGTGACTTA TATGAACTCT 18540
AAACTCGCTG ACGGCTCTAA GCTGCCAGCT GAAGGCTCTA TGCACTATCA GCTGTCTACA 18600
AGTACCGCTG CTGCGACTCC TGTAGCGAAT GGTCTCTCTG CAGAAAAAGT TCAAGCGACC 18660
ATGATGTCTG TAGTTGCAGA TAAAACTGGC TACCCAACTG AAATGCTTGA ACTTGAAATG 18720
GATATGGAAG CCGATTTAGG TATCGATTCT ATCAAGCGCG TTGAAATTCT TGGCACAGTA 18780
CAAGATGAGC TACCGGGTTT ACCTGAGCTA AATCCAGAAG ATCTAGCAGA GTGTCGCACC 18840
CTAGGCGAAA TCGTTGACTA TATGGGCAGT AAACTGCCGG CTGAAGGCTC TGCTAATACA 18900
AGTGCCGCTG CGTCTCTTAA TGTTAGTGCC GTTGCGGCGC CTCAAGCTGC TGCGACTCCT 18960
GTATCGAACG GTCTCTCTGC AGAGAAAGTG CAAAGCACTA TGATGTCAGT AGTTGCAGAA 19020
AAGACCGGCT ACCCAACTGA AATGCTAGAA CTTGGCATGG ATATGGAAGC CGATTTAGGT 19080
ATCGACTCAA TTAAACGCGT TGAGATTCTT GGCACAGTAC AAGATGAGCT ACCGGGTCTA 19140
CCAGAGCTTA ATCCTGAAGA TTTAGCTGAG TGCCGTACGC TGGGCGAAAT CGTTGACTAT 19200
ATGAACTCTA AGCTGGCTGA CGGCTCTAAG CTTCCAGCTG AAGGCTCTGC TAATACAAGT 19260
GCCACTGCTG CGACTCCTGC AGTGAATGGT CTTTCTGCTG ACAAGGTACA GGCGACTATG 19320
ATGTCTGTAG TTGCTGAAAA GACCGGCTAC CCAACTGAAA TGCTAGAACT TGGCATGGAT 19380
ATGGAAGCAG ACCTTGGTAT TGATTCTATT AAGCGCGTTG AAATTCTTGG CACAGTACAA 19440

GATGAGCTCC CAGGTTTACC TGAGCTTAAT CCTGAAGATC TCGCTGAGTG CCGCACGCTT 19500
GGCGAAATCG TTAGCTATAT GAACTCTCAA CTGGCTGATG GCTCTAAACT TTCTACAAGT 19560
GCGGCTGAAG GCTCTGCTGA TACAAGTGCT GCAAATGCTG CAAAGCCGGC AGCAATTTCG 19620
GCAGAACCAA GTGTTGAGCT TCCTCCTCAT AGCGAGGTAG CGCTAAAAAA GCTTAATGCG 19680
GCGAACAAGC TAGAAAATTG TTTCGCCGCA GACGCAAGTG TTGTGATTAA CGATGATGGT 19740
CACAACGCAG GCGTTTTAGC TGAGAAACTT ATTAAACAAG GCCTAAAAGT AGCCGTTGTG 19800
CGTTTACCGA AAGGTCAGCC TCAATCGCCA CTTTCAAGCG ATGTTGCTAG CTTTGAGCTT 19860
GCCTCAAGCC AAGAATCTGA GCTTGAAGCC AGTATCACTG CAGTTATCGC GCAGATTGAA 19920
ACTCAGGTTG GCGCTATTGG TGGCTTTATT CACTTGCAAC CAGAAGCGAA TACAGAAGAG 19980
CAAACGGCAG TAAACCTAGA TGCGCAAAGT TTTACTCACG TTAGCAATGC GTTCTTGTGG 20040
GCCAAATTAT TGCAACCAAA GCTCGTTGCT GGAGCAGATG CGCGTCGCTG TTTTGTAACA 20100
GTAAGCCGTA TCGACGGTGG CTTTGGTTAC CTAAATACTG ACGCCCTAAA AGATGCTGAG 20160
CTAAACCAAG CAGCATTAGC TGGTTTAACT AAAACCTTAA GCCATGAATG GCCACAAGTG 20220
TTCTGTCGCG CGCTAGATAT TGCAACAGAT GTTGATGCAA CCCATCTTGC TGATGCAATC 20280
ACCAGTGAAC TATTTGATAG CCAAGCTCAG CTACCTGAAG TGGGCTTAAG CTTAATTGAT 20340
GGCAAAGTTA ACCGCGTAAC TCTAGTTGCT GCTGAAGCTG CAGATAAAAC AGCAAAAGCA 20400
GAGCTTAACA GCACAGATAA AATCTTAGTG ACTGGTGGGG CAAAAGGGGT GACATTTGAA 20460
TGTGCACTGG CATTAGCATC TCGCAGCCAG TCTCACTTTA TCTTAGCTGG GCGCAGTGAA 20520
TTACAAGCTT TACCAAGCTG GGCTGAGGGT AAGCAAACTA GCGAGCTAAA ATCAGCTGCA 20580
ATCGCACATA TTATTTCTAC TGGTCAAAAG CCAACGCCTA AGCAAGTTGA AGCCGCTGTG 20640
TGGCCAGTGC AAAGCAGCAT TGAAATTAAT GCCGCCCTAG CCGCCTTTAA CAAAGTTGGC 20700
GCCTCAGCTG AATACGTCAG CATGGATGTT ACCGATAGCG CCGCAATCAC AGCAGCACTT 20760
AATGGTCGCT CAAATGAGAT CACCGGTCTT ATTCATGGCG CAGGTGTACT AGCCGACAAG 20820
CATATTCAAG ACAAGACTCT TGCTGAACTT GCTAAAGTTT ATGGCACTAA AGTCAACGGC 20880
CTAAAAGCGC TGCTCGCGGC ACTTGAGCCA AGCAAAATTA AATTACTTGC TATGTTCTCA 20940
TCTGCAGCAG GTTTTTACGG TAATATCGGC CAAAGCGATT ACGCGATGTC GAACGATATT 21000
CTTAACAAGG CAGCGCTGCA GTTCACCGCT CGCAACCCAC AAGCTAAAGT CATGAGCTTT 21060
AACTGGGGTC CTTGGGATGG CGGCATGGTT AACCCAGCGC TTAAAAAGAT GTTTACCGAG 21120
CGTGGTGTGT ACGTTATTCC ACTAAAAGCA GGTGCAGAGC TATTTGCCAC TCAGCTATTG 21180
GCTGAAACTG GCGTGCAGTT GCTCATTGGT ACGTCAATGC AAGGTGGCAG CGACACTAAA 21240
GCAACTGAGA CTGCTTCTGT AAAAAAGCTT AATGCGGGTG AGGTGCTAAG TGCATCGCAT 21300
CCGCGTGCTG GTGCACAAAA AACACCACTA CAAGCTGTCA CTGCAACGCG TCTGTTAACC 21360
CCAAGTGCCA TGGTCTTCAT TGAAGATCAC CGCATTGGCG GTAACAGTGT GTTGCCAACG 21420
GTATGCGCCA TCGACTGGAT GCGTGAAGCG GCAAGCGACA TGCTTGGCGC TCAAGTTAAG 21480
GTACTTGATT ACAAGCTATT AAAAGGCATT GTATTTGAGA CTGATGAGCC GCAAGAGTTA 21540
ACACTTGAGC TAACGCCAGA CGATTCAGAC GAAGCTACGC TACAAGCATT AATCAGCTGT 21600
AATGGGCGTC CGCAATACAA GGCGACGCTT ATCAGTGATA ATGCCGATAT TAAGCAACTT 21660

AACAAGCAGT TTGATTTAAG CGCTAAGGCG ATTACCACAG CAAAAGAGCT TTATAGCAAC 21720
GGCACCTTGT TCCACGGTCC GCGTCTACAA GGGATCCAAT CTGTAGTGCA GTTCGATGAT 21780
CAAGGCTTAA TTGCTAAAGT CGCTCTGCCT AAGGTTGAAC TTAGCGATTG TGGTGAGTTC 21840
TTGCCGCAAA CCCACATGGG TGGCAGTCAA CCTTTTGCTG AGGACTTGCT ATTACAAGCT 21900
ATGCTGGTTT GGGCTCGCCT TAAAACTGGC TCGGCAAGTT TGCCATCAAG CATTGGTGAG 21960
TTTACCTCAT ACCAACCAAT GGCCTTTGGT GAAACTGGTA CCATAGAGCT TGAAGTGATT 22020
AAGCACAACA AACGCTCACT TGAAGCGAAT GTTGCGCTAT ATCGTGACAA CGGCGAGTTA 22080
AGTGCCATGT TTAAGTCAGC TAAAATCACC ATTAGCAAAA GCTTAAATTC AGCATTTTTA 22140
CCTGCTGTCT TAGCAAACGA CAGTGAGGCG AATTAGTGGA ACAAACGCCT AAAGCTAGTG 22200
CGATGCCGCT GCGCATCGCA CTTATCTTAC TGCCAACACC GCAGTTTGAA GTTAACTCTG 22260
TCGACCAGTC AGTATTAGCC AGCTATCAAA CACTGCAGCC TGAGCTAAAT GCCCTGCTTA 22320
ATAGTGCGCC GACACCTGAA ATGCTCAGCA TCACTATCTC AGATGATAGC GATGCAAACA 22380
GCTTTGAGTC GCAGCTAAAT GCTGCGACCA ACGCAATTAA CAATGGCTAT ATCGTCAAGC 22440
TTGCTACGGC AACTCACGCT TTGTTAATGC TGCCTGCATT AAAAGCGGCG CAAATGCGGA 22500
TCCATCCTCA TGCGCAGCTT GCCGCTATGC AGCAAGCTAA ATCGACGCCA ATGAGTCAAG 22560
TATCTGGTGA GCTAAAGCTT GGCGCTAATG CGCTAAGCCT AGCTCAGACT AATGCGCTGT 22620
CTCATGCTTT AAGCCAAGCC AAGCGTAACT TAACTGATGT CAGCGTGAAT GAGTGTTTTG 22680
AGAACCTCAA AAGTGAACAG CAGTTCACAG AGGTTTATTC GCTTATTCAG CAACTTGCTA 22740
GCCGCACCCA TGTGAGAAAA GAGGTTAATC AAGGTGTGGA ACTTGGCCCT AAACAAGCCA 22800
AAAGCCACTA TTGGTTTAGC GAATTTCACC AAAACCGTGT TGCTGCCATC AACTTTATTA 22860
ATGGCCAACA AGCAACCAGC TATGTGCTTA CTCAAGGTTC AGGATTGTTA GCTGCGAAAT 22920
CAATGCTAAA CCAGCAAAGA TTAATGTTTA TCTTGCCGGG TAACAGTCAG CAACAAATAA 22980
CCGCATCAAT AACTCAGTTA ATGCAGCAAT TAGAGCGTTT GCAGGTAACT GAGGTTAATG 23040
AGCTTTCTCT AGAATGCCAA CTAGAGCTGC TCAGCATAAT GTATGACAAC TTAGTCAACG 23100
CAGACAAACT CACTACTCGC GATAGTAAGC CCGCTTATCA GGCTGTGATT CAAGCAAGCT 23160
CTGTTAGCGC TGCAAAGCAA GAGTTAAGCG CGCTTAACGA TGCACTCACA GCGCTGTTTG 23220
CTGAGCAAAC AAACGCCACA TCAACGAATA AAGGCTTAAT CCAATACAAA ACACCGGCGG 23280
GCAGTTACTT AACCCTAACA CCGCTTGGCA GCAACAATGA CAACGCCCAA GCGGGTCTTG 23340
CTTTTGTCTA TCCGGGTGTG GGAACGGTTT ACGCCGATAT GCTTAATGAG CTGCATCAGT 23400
ACTTCCCTGC GCTTTACGCC AAACTTGAGC GTGAAGGCGA TTTAAAGGCG ATGCTACAAG 23460
CAGAAGATAT CTATCATCTT GACCCTAAAC ATGCTGCCCA AATGAGCTTA GGTGACTTAG 23520
CCATTGCTGG CGTGGGGAGC AGCTACCTGT TAACTCAGCT GCTCACCGAT GAGTTTAATA 23580
TTAAGCCTAA TTTTGCATTA GGTTACTCAA TGGGTGAAGC ATCAATGTGG GCAAGCTTAG 23640
GCGTATGGCA AAACCCGCAT GCGCTGATCA GCAAAACCCA AACCGACCCG CTATTTACTT 23700
CTGCTATTTC CGGCAAATTG ACCGCGGTTA GACAAGCTTG GCAGCTTGAT GATACCGCAG 23760
CGGAAATCCA GTGGAATAGC TTTGTGGTTA GAAGTGAAGC AGCGCCGATT GAAGCCTTGC 23820
TAAAAGATTA CCCACACGCT TACCTCGCGA TTATTCAAGG GGATACCTGC GTAATCGCTG 23880

GCTGTGAAAT CCAATGTAAA GCGCTACTTG CAGCACTGGG TAAACGCGGT ATTGCAGCTA 23940
ATCGTGTAAC GGCGATGCAT ACGCAGCCTG CGATGCAAGA GCATCAAAAT GTGATGGATT 24000
TTTATCTGCA ACCGTTAAAA GCAGAGCTTC CTAGTGAAAT AAGCTTTATC AGCGCCGCTG 24060
ATTTAACTGC CAAGCAAACG GTGAGTGAGC AAGCACTTAG CAGCCAAGTC GTTGCTCAGT 24120
CTATTGCCGA CACCTTCTGC CAAACCTTGG ACTTTACCGC GCTAGTACAT CACGCCCAAC 24180
ATCAAGGCGC TAAGCTGTTT GTTGAAATTG GCGCGGATAG ACAAAACTGC ACCTTGATAG 24240
ACAAGATTGT TAAACAAGAT GGTGCCAGCA GTGTACAACA TCAACCTTGT TGCACAGTGC 24300
CTATGAACGC AAAAGGTAGC CAAGATATTA CCAGCGTGAT TAAAGCGCTT GGCCAATTAA 24360
TTAGCCATCA GGTGCCATTA TCGGTGCAAC CATTTATTGA TGGACTCAAG CGCGAGCTAA 24420
CACTTTGCCA ATTGACCAGC CAACAGCTGG CAGCACATGC AAATGTTGAC AGCAAGTTTG 24480
AGTCTAACCA AGACCATTTA CTTCAAGGGG AAGTCTAATG TCATTACCAG ACAATGCTTC 24540
TAACCACCTT TCTGCCAACC AGAAAGGCGC ATCTCAGGCA AGTAAAACCA GTAAGCAAAG 24600
CAAAATCGCC ATTGTCGGTT TAGCCACTCT GTATCCAGAC GCTAAAACCC CGCAAGAATT 24660
TTGGCAGAAT TTGCTGGATA AACGCGACTC TCGCAGCACC TTAACTAACG AAAAACTCGG 24720
CGCTAACAGC CAAGATTATC AAGGTGTGCA AGGCCAATCT GACCGTTTTT ATTGTAATAA 24780
AGGCGGCTAC ATTGAGAACT TCAGCTTTAA TGCTGCAGGC TACAAATTGC CGGAGCAAAG 24840
CTTAAATGGC TTGGACGACA GCTTCCTTTG GGCGCTCGAT ACTAGCCGTA ACGCACTAAT 24900
TGATGCTGGT ATTGATATCA ACGGCGCTGA TTTAAGCCGC GCAGGTGTAG TCATGGGCGC 24960
GCTGTCGTTC CCAACTACCC GCTCAAACGA TCTGTTTTTG CCAATTTATC ACAGCGCCGT 25020
TGAAAAAGCC CTGCAAGATA AACTAGGCGT AAAGGCATTT AAGCTAAGCC CAACTAATGC 25080
TCATACCGCT CGCGCGGCAA ATGAGAGCAG CCTAAATGCA GCCAATGGTG CCATTGCCCA 25140
TAACAGCTCA AAAGTGGTGG CCGATGCACT TGGCCTTGGC GGCGCACAAC TAAGCCTAGA 25200
TGCTGCCTGT GCTAGTTCGG TTTACTCATT AAAGCTTGCC TGCGATTACC TAAGCACTGG 25260
CAAAGCCGAT ATCATGCTAG CAGGCGCAGT ATCTGGCGCG GATCCTTTCT TTATTAATAT 25320
GGGATTCTCA ATCTTCCACG CCTACCCAGA CCATGGTATC TCAGTACCGT TTGATGCCAG 25380
CAGTAAAGGT TTGTTTGCTG GCGAAGGCGC TGGCGTATTA GTGCTTAAAC GTCTTGAAGA 25440
TGCCGAGCGC GACAATGACA AAATCTATGC GGTTGTTAGC GGCGTAGGTC TATCAAACGA 25500
CGGTAAAGGC CAGTTTGTAT TAAGCCCTAA TCCAAAAGGT CAGGTGAAGG CCTTTGAACG 25560
TGCTTATGCT GCCAGTGACA TTGAGCCAAA AGACATTGAA GTGATTGAGT GCCACGCAAC 25620
AGGCACACCG CTTGGCGATA AAATTGAGCT CACTTCAATG GAAACCTTCT TTGAAGACAA 25680
GCTGCAAGGC ACCGATGCAC CGTTAATTGG CTCAGCTAAG TCTAACTTAG GCCACCTATT 25740
AACTGCAGCG CATGCGGGGA TCATGAAGAT GATCTTCGCC ATGAAAGAAG GTTACCTGCC 25800
GCCAAGTATC AATATTAGTG ATGCTATCGC TTCGCCGAAA AAACTCTTCG GTAAACCAAC 25860
CCTGCCTAGC ATGGTTCAAG GCTGGCCAGA TAAGCCATCG AATAATCATT TTGGTGTAAG 25920
AACCCGTCAC GCAGGCGTAT CGGTATTTGG CTTTGGTGGC TGTAACGCCC ATCTGTTGCT 25980
TGAGTCATAC AACGGCAAAG GAACAGTAAA GGCAGAAGCC ACTCAAGTAC CGCGTCAAGC 26040
TGAGCCGCTA AAAGTGGTTG GCCTTGCCTC GCACTTTGGG CCTCTTAGCA GCATTAATGC 26100

ACTCAACAAT GCTGTGACCC AAGATGGGAA TGGCTTTATC GAACTGCCGA AAAAGCGCTG 26160
GAAAGGCCTT GAAAAGCACA GTGAACTGTT AGCTGAATTT GGCTTAGCAT CTGCGCCAAA 26220
AGGTGCTTAT GTTGATAACT TCGAGCTGGA CTTTTTACGC TTTAAACTGC CGCCAAACGA 26280
AGATGACCGT TTGATCTCAC AGCAGCTAAT GCTAATGCGA GTAACAGACG AAGCCATTCG 26340
TGATGCCAAG CTTGAGCCGG GGCAAAAAGT AGCTGTATTA GTGGCAATGG AAACTGAGCT 26400
TGAACTGCAT CAGTTCCGCG GCCGGGTTAA CTTGCATACT CAATTAGCGC AAAGTCTTGC 26460
CGCCATGGGC GTGAGTTTAT CAACGGATGA ATACCAAGCG CTTGAAGCCA TCGCCATGGA 26520
CAGCGTGCTT GATGCTGCCA AGCTCAATCA GTACACCAGC TTTATTGGTA ATATTATGGC 26580
GTCACGCGTG GCGTCACTAT GGGACTTTAA TGGCCCAGCC TTCACTATTT CAGCAGCAGA 26640
GCAATCTGTG AGCCGCTGTA TCGATGTGGC GCAAAACCTC ATCATGGAGG ATAACCTAGA 26700
TGCGGTGGTG ATTGCAGCGG TCGATCTCTC TGGTAGCTTT GAGCAAGTCA TTCTTAAAAA 26760
TGCCATTGCA CCTGTAGCCA TTGAGCCAAA CCTCGAAGCA AGCCTTAATC CAACATCAGC 26820
AAGCTGGAAT GTCGGTGAAG GTGCTGGCGC GGTCGTGCTT GTTAAAAATG AAGCTACATC 26880
GGGCTGCTCA TACGGCCAAA TTGATGCACT TGGCTTTGCT AAAACTGCCG AAACAGCGTT 26940
GGCTACCGAC AAGCTACTGA GCCAAACTGC CACAGACTTT AATAAGGTTA AAGTGATTGA 27000
AACTATGGCA GCGCCTGCTA GCCAAATTCA ATTAGCGCCA ATAGTTAGCT CTCAAGTGAC 27060
TCACACTGCT GCAGAGCAGC GTGTTGGTCA CTGCTTTGCT GCAGCGGGTA TGGCAAGCCT 27120
ATTACACGGC TTACTTAACT TAAATACTGT AGCCCAAACC AATAAAGCCA ATTGCGCGCT 27180
TATCAACAAT ATCAGTGAAA ACCAATTATC ACAGCTGTTG ATTAGCCAAA CAGCGAGCGA 27240
ACAACAAGCA TTAACCGCGC GTTTAAGCAA TGAGCTTAAA TCCGATGCTA AACACCAACT 27300
GGTTAAGCAA GTCACCTTAG GTGGCCGTGA TATCTACCAG CATATTGTTG ATACACCGCT 27360
TGCAAGCCTT GAAAGCATTA CTCAGAAATT GGCGCAAGCG ACAGCATCGA CAGTGGTCAA 27420
CCAAGTTAAA CCTATTAAGG CCGCTGGCTC AGTCGAAATG GCTAACTCAT TCGAAACGGA 27480
AAGCTCAGCA GAGCCACAAA TAACAATTGC AGCACAACAG ACTGCAAACA TTGGCGTCAC 27540
CGCTCAGGCA ACCAAACGTG AATTAGGTAC CCCACCAATG ACAACAAATA CCATTGCTAA 27600
TACAGCAAAT AATTTAGACA AGACTCTTGA GACTGTTGCT GGCAATACTG TTGCTAGCAA 27660
GGTTGGCTCT GGCGACATAG TCAATTTTCA ACAGAACCAA CAATTGGCTC AACAAGCTCA 27720
CCTCGCCTTT CTTGAAAGCC GCAGTGCGGG TATGAAGGTG GCTGATGCTT TATTGAAGCA 27780
ACAGCTAGCT CAAGTAACAG GCCAAACTAT CGATAATCAG GCCCTCGATA CTCAAGCCGT 27840
CGATACTCAA ACAAGCGAGA ATGTAGCGAT TGCCGCAGAA TCACCAGTTC AAGTTACAAC 27900
ACCTGTTCAA GTTACAACAC CTGTTCAAAT CAGTGTTGTG GAGTTAAAAC CAGATCACGC 27960
TAATGTGCCA CCATACACGC CGCCAGTGCC TGCATTAAAG CCGTGTATCT GGAACTATGC 28020
CGATTTAGTT GAGTACGCAG AAGGCGATAT CGCCAAGGTA TTTGGCAGTG ATTATGCCAT 28080
TATCGACAGC TACTCGCGCC GCGTACGTCT ACCGACCACT GACTACCTGT TGGTATCGCG 28140
CGTGACCAAA CTTGATGCGA CCATCAATCA ATTTAAGCCA TGCTCAATGA CCACTGAGTA 28200
CGACATCCCT GTTGATGCGC CGTACTTAGT AGACGGACAA ATCCCTTGGG CGGTAGCAGT 28260
AGAATCAGGC CAATGTGACT TGATGCTTAT TAGCTATCTC GGTATCGACT TTGAGAACAA 28320

AGGCGAGCGG GTTTATCGAC TACTCGATTG TACCCTCACC TTCCTAGGCG ACTTGCCACG 28380
TGGCGGAGAT ACCCTACGTT ACGACATTAA GATCAATAAC TATGCTCGCA ACGGCGACAC 28440
CCTGCTGTTC TTCTTCTCGT ATGAGTGTTT TGTTGGCGAC AAGATGATCC TCAAGATGGA 28500
TGGCGGCTGC GCTGGCTTCT TCACTGATGA AGAGCTTGCC GACGGTAAAG GCGTGATTCG 28560
CACAGAAGAA GAGATTAAAG CTCGCAGCCT AGTGCAAAAG CAACGCTTTA ATCCGTTACT 28620
AGATTGTCCT AAAACCCAAT TTAGTTATGG TGATATTCAT AAGCTATTAA CTGCTGATAT 28680
TGAGGGTTGT TTTGGCCCAA GCCACAGTGG CGTCCACCAG CCGTCACTTT GTTTCGCATC 28740
TGAAAAATTC TTGATGATTG AACAAGTCAG CAAGGTTGAT CGCACTGGCG GTACTTGGGG 28800
ACTTGGCTTA ATTGAGGGTC ATAAGCAGCT TGAAGCAGAC CACTGGTACT TCCCATGTCA 28860
TTTCAAGGGC GACCAAGTGA TGGCTGGCTC GCTAATGGCT GAAGGTTGTG GCCAGTTATT 28920
GCAGTTCTAT ATGCTGCACC TTGGTATGCA TACCCAAACT AAAAATGGTC GTTTCCAACC 28980
TCTTGAAAAC GCCTCACAGC AAGTACGCTG TCGCGGTCAA GTGCTGCCAC AATCAGGCGT 29040
GCTAACTTAC CGTATGGAAG TGACTGAAAT CGGTTTCAGT CCACGCCCAT ATGCTAAAGC 29100
TAACATCGAT ATCTTGCTTA ATGGCAAAGC GGTAGTGGAT TTCCAAAACC TAGGGGTGAT 29160
GATAAAAGAG GAAGATGAGT GTACTCGTTA TCCACTTTTG ACTGAATCAA CAACGGCTAG 29220
CACTGCACAA GTAAACGCTC AAACAAGTGC GAAAAAGGTA TACAAGCCAG CATCAGTCAA 29280
TGCGCCATTA ATGGCACAAA TTCCTGATCT GACTAAAGAG CCAAACAAGG GCGTTATTCC 29340
GATTTCCCAT GTTGAAGCAC CAATTACGCC AGACTACCCG AACCGTGTAC CTGATACAGT 29400
GCCATTCACG CCGTATCACA TGTTTGAGTT TGCTACAGGC AATATCGAAA ACTGTTTCGG 29460
GCCAGAGTTC TCAATCTATC GCGGCATGAT CCCACCACGT ACACCATGCG GTGACTTACA 29520
AGTGACCACA CGTGTGATTG AAGTTAACGG TAAGCGTGGC GACTTTAAAA AGCCATCATC 29580
GTGTATCGCT GAATATGAAG TGCCTGCAGA TGCGTGGTAT TTCGATAAAA ACAGCCACGG 29640
CGCAGTGATG CCATATTCAA TTTTAATGGA GATCTCACTG CAACCTAACG GCTTTATCTC 29700
AGGTTACATG GGCACAACCC TAGGCTTCCC TGGCCTTGAG CTGTTCTTCC GTAACTTAGA 29760
CGGTAGCGGT GAGTTACTAC GTGAAGTAGA TTTACGTGGT AAAACCATCC GTAACGACTC 29820
ACGTTTATTA TCAACAGTGA TGGCCGGCAC TAACATCATC CAAAGCTTTA GCTTCGAGCT 29880
AAGCACTGAC GGTGAGCCTT TCTATCGCGG CACTGCGGTA TTTGGCTATT TTAAAGGTGA 29940
CGCACTTAAA GATCAGCTAG GCCTAGATAA CGGTAAAGTC ACTCAGCCAT GGCATGTAGC 30000
TAACGGCGTT GCTGCAAGCA CTAAGGTGAA CCTGCTTGAT AAGAGCTGCC GTCACTTTAA 30060
TGCGCCAGCT AACCAGCCAC ACTATCGTCT AGCCGGTGGT CAGCTGAACT TTATCGACAG 30120
TGTTGAAATT GTTGATAATG GCGGCACCGA AGGTTTAGGT TACTTGTATG CCGAGCGCAC 30180
CATTGACCCA AGTGATTGGT TCTTCCAGTT CCACTTCCAC CAAGATCCGG TTATGCCAGG 30240
CTCCTTAGGT GTTGAAGCAA TTATTGAAAC CATGCAAGCT TACGCTATTA GTAAAGACTT 30300
GGGCGCAGAT TTCAAAAATC CTAAGTTTGG TCAGATTTTA TCGAACATCA AGTGGAAGTA 30360
TCGCGGTCAA ATCAATCCGC TGAACAAGCA GATGTCTATG GATGTCAGCA TTACTTCAAT 30420
CAAAGATGAA GACGGTAAGA AAGTCATCAC AGGTAATGCC AGCTTGAGTA AAGATGGTCT 30480
GCGCATATAC GAGGTCTTCG ATATAGCTAT CAGCATCGAA GAATCTGTAT AAATCGGAGT 30540

GACTGTCTGG CTATTTTACT CAATTTCTGT GTCAAAAGTG CTCACCTATA TTCATAGGCT 30600
GCGCGCTTTT TTCTGGAAAT TGAGCAAAAG TATCTGCGTC CTAACTCGAT TTATAAGAAT 30660
GGTTTAATTG AAAAGAACAA CAGCTAAGAG CCGCAAGCTC AATATAAATA ATTAAGGGTC 30720
TTACAAATAA TGAATCCTAC AGCAACTAAC GAAATGCTTT CTCCGTGGCC ATGGGCTGTG 30780
ACAGAGTCAA ATATCAGTTT TGACGTGCAA GTGATGGAAC AACAACTTAA AGATTTTAGC 30840
CGGGCATGTT ACGTGGTCAA TCATGCCGAC CACGGCTTTG GTATTGCGCA AACTGCCGAT 30900
ATCGTGACTG AACAAGCGGC AAACAGCACA GATTTACCTG TTAGTGCTTT TACTCCTGCA 30960
TTAGGTACCG AAAGCCTAGG CGACAATAAT TTCCGCCGCG TTCACGGCGT TAAATACGCT 31020
TATTACGCAG GCGCTATGGC AAACGGTATT TCATCTGAAG AGCTAGTGAT TGCCCTAGGT 31080
CAAGCTGGCA TTTTGTGTGG TTCGTTTGGA GCAGCCGGTC TTATTCCAAG TCGCGTTGAA 31140
GCGGCAATTA ACCGTATTCA AGCAGCGCTG CCAAATGGCC CTTATATGTT TAACCTTATC 31200
CATAGTCCTA GCGAGCCAGC ATTAGAGCGT GGCAGCGTAG AGCTATTTTT AAAGCATAAG 31260
GTACGCACCG TTGAAGCATC AGCTTTCTTA GGTCTAACAC CACAAATCGT CTATTACCGT 31320
GCAGCAGGAT TGAGCCGAGA CGCACAAGGT AAAGTTGTGG TTGGTAACAA GGTTATCGCT 31380
AAAGTAAGTC GCACCGAAGT GGCTGAAAAG TTTATGATGC CAGCGCCCGC AAAAATGCTA 31440
CAAAAACTAG TTGATGACGG TTCAATTACC GCTGAGCAAA TGGAGCTGGC GCAACTTGTA 31500
CCTATGGCTG ACGACATCAC TGCAGAGGCC GATTCAGGTG GCCATACTGA TAACCGTCCA 31560
TTAGTAACAT TGCTGCCAAC CATTTTAGCG CTGAAAGAAG AAATTCAAGC TAAATACCAA 31620
TACGACACTC CTATTCGTGT CGGTTGTGGT GGCGGTGTGG GTACGCCTGA TGCAGCGCTG 31680
GCAACGTTTA ACATGGGCGC GGCGTATATT GTTACCGGCT CTATCAACCA AGCTTGTGTT 31740
GAAGCGGGCG CAAGTGATCA CACTCGTAAA TTACTTGCCA CCACTGAAAT GGCCGATGTG 31800
ACTATGGCAC CAGCTGCAGA TATGTTCGAG ATGGGCGTAA AACTGCAGGT GGTTAAGCGC 31860
GGCACGCTAT TCCCAATGCG CGCTAACAAG CTATATGAGA TCTACACCCG TTACGATTCA 31920
ATCGAAGCGA TCCCATTAGA CGAGCGTGAA AAGCTTGAGA AACAAGTATT CCGCTCAAGC 31980
CTAGATGAAA TATGGGCAGG TACAGTGGCG CACTTTAACG AGCGCGACCC TAAGCAAATC 32040
GAACGCGCAG AGGGTAACCC TAAGCGTAAA ATGGCATTGA TTTTCCGTTG GTACTTAGGT 32100
CTTTCTAGTC GCTGGTCAAA CTCAGGCGAA GTGGGTCGTG AAATGGATTA TCAAATTTGG 32160
GCTGGCCCTG CTCTCGGTGC ATTTAACCAA TGGGCAAAAG GCAGTTACTT AGATAACTAT 32220
CAAGACCGAA ATGCCGTCGA TTTGGCAAAG CACTTAATGT ACGGCGCGGC TTACTTAAAT 32280
CGTATTAACT CGCTAACGGC TCAAGGCGTT AAAGTGCCAG CACAGTTACT TCGCTGGAAG 32340
CCAAACCAAA GAATGGCCTA ATACACTTAC AAAGCACCAG TCTAAAAAGC CACTAATCTT 32400
GATTAGTGGC TTTTTTTATT GTGGTCAATA TGAGGCTATT TAGCCTGTAA GCCTGAAAAT 32460
ATCAGCACTC TGACTTTACA AGCAAATTAT AATTAAGGCA GGGCTCTACT CATTTATACT 32520
GCTAGCAAAC AAGCAAGTTG CCCAGTAAAA CAACAAGGTA CCTGATTTAT ATCGTCATAA 32580
AAGTTGGCTA GAGATTCGTT ATTGATCTTT ACTGATTAGA GTCGCTCTGT TTGGAAAAAG 32640
GTTTCTCGTT ATCATCAAAA TACACTCTCA AACCTTTAAT CAATTACAAC TTAGGCTTTC 32700
TGCGGGCATT TTTATCTTAT TTGCCACAGC TGTATTTGCC TTTAGGTTTT GGGTGCAACT 32760

ACCATTAATT GAGGCCTCAT TAGTTAAATT ATCTGAGCAA GAGCTCACCT CTTTAAATTA 32820
CGCTTTTCAG CAAATGAGAA AGCCACTACA AACCATTAAT TACGACTATG CGGTGTGGGA 32880
CAGAACCTAC AGCTATATGA AATCAAACTC AGCGAGCGCT AAAAGGTACT ATGAAAAACA 32940
TGAGTACCCA GATGATACGT TCAAGAGTTT AAAAGTCGAC GGAGTATTTA TATTCAACCG 33000
TACAAATCAG CCAGTTTTTA GTAAAGGTTT TAATCATAGA AATGATATAC CGCTGGTCTT 33060
TGAATTAACT GACTTTAAAC AACATCCACA AAACATCGCA TTATCTCCAC AAACCAAACA 33120
GGCACACCCA CCGGCAAGTA AGCCGTTAGA CTCCCCTGAT GATGTGCCTT CTACCCATGG 33180
GGTTATCGCC ACACGATACG GTCCAGCAAT TTATAGCTCT ACCAGCATTT TAAAATCTGA 33240
TCGTAGCGGC TCCCAACTTG GTTATTTAGT CTTCATTAGG TTAATTGATG AATGGTTCAT 33300
CGCTGAGCTA TCGCAATACA CTGCCGCAGG TGTTGAAATC GCTATGGCTG ATGCCGCAGA 33360
CGCACAATTA GCGAGATTAG GCGCAAACAC TAAGCTTAAT AAAGTAACCG CTACATCCGA 33420
ACGGTTAATA ACTAATGTCG ATGGTAAGCC TCTGTTGAAG TTAGTGCTTT ACCATACCAA 33480
TAACCAACCG CCGCCGATGC TAGATTACAG TATAATAATT CTATTAGTTG AGATGTCATT 33540
TTTACTGATC CTCGCTTATT TCCTTTACTC CTACTTCTTA GTCAGGCCAG TTAGAAAGCT 33600
GGCTTCAGAT ATTAAAAAAA TGGATAAAAG TCGTGAAATT AAAAAGCTAA GGTATCACTA 33660
CCCTATTACT GAGCTAGTCA AAGTTGCGAC TCACTTCAAC GCCCTAATGG GGACGATTCA 33720
GGAACAAACT AAACAGCTTA ATGAACAAGT TTTTATTGAT AAATTAACCA ATATTCCCAA 33780
TCGTCGCGCT TTTGAGCAGC GACTTGAAAC CTATTGCCAA CTGCTAGCCC GGCAACAAAT 33840
TGGCTTTACT CTCATCATTG CCGATGTGGA TCATTTTAAA GAGTACAACG ATACTCTTGG 33900
GCACCTTGCT GGGGATGAAG CATTAATAAA AGTGGCACAA ACACTATCGC AACAGTTTTA 33960
CCGTGCAGAA GATATTTGTG CCCGTTTTGG TGGTGAAGAA TTTATTATGT TATTTCGAGA 34020
CATACCTGAT GAGCCCTTGC AGAGAAAGCT CGATGCGATG CTGCACTCTT TTGCAGAGCT 34080
CAACCTACCT CATCCAAACT CATCAACCGC TAATTACGTT ACTGTGAGCC TTGGGGTTTG 34140
CACAGTTGTT GCTGTTGATG ATTTTGAATT TAAAAGTGAG TCGCATATTA TTGGCAGTCA 34200
GGCTGCATTA ATCGCAGATA AGGCGCTTTA TCATGCTAAA GCCTGTGGTC GTAACCAGTT 34260
GTCAAAAACT ACTATTACTG TTGATGAGAT TGAGCAATTA GAAGCAAATA AAATCGGTCA 34320
TCAAGCCTAA ACTCGTTCGA GTACTTTCCC CTAAGTCAGA GCTATTTGCC ACTTCAAGAT 34380
GTGGCTACAA GGCTTACTCT TTCAAAACCT GCATCAATAG AACACAGCAA AATACAATAA 34440
TTTAAGTCAA TTTAGCCTAT TAAACAGAGT TAATGACAGC TCATGGTCGC AACTTATTAG 34500
CTATTTCTAG CAATATAAAA ACTTATCCAT TAGTAGTAAC CAATAAAAAA ACTAATATAT 34560
AAAACTATTT AATCATTATT TTACAGATGA TTAGCTACCA CCCACCTTAA GCTGGCTATA 34620
TTCGCACTAG TAAAAATAAA CATTAGATCG GGTTCAGATC AATTTACGAG TCTCGTATAA 34680
AATGTACAAT AATTCACTTA ATTTAATACT GCATATTTTT ACAAGTAGAG AGCGGTGATG 34740
AAACAAAATA CGAAAGGCTT TACATTAATT GAATTAGTCA TCGTGATTAT TATTCTCGGT 34800
ATACTTGCTG CTGTGGCACT GCCGAAATTC ATCAATGTTC AAGATGACGC TAGGATCTCT 34860
GCGATGAGCG GTCAGTTTTC ATCATTTGAA AGTGCCGTAA AACTATACCA TAGCGGTTGG 34920
TTAGCCAAAG GCTACAACAC TGCGGTTGAA AAGCTCTCAG GCTTTGGCCA AGGTAATGTT 34980

GCATCAAGTG ACACAGGTTT TCCGTACTCA ACATCAGGCA CGAGTACTGA TGTGCATAAA 35040
GCTTGTGGTG AACTATGGCA TGGCATTACC GATACAGACT TCACAATTGG TGCGGTTAGT 35100
GATGGCGATC TAATGACTGC AGATGTCGAT ATTGCTTACA CCTATCGTGG TGATATGTGT 35160
ATCTATCGCG ATCTGTATTT TATTCAGCGC TCATTACCTA CTAAGGTGAT GAACTACAAA 35220
TTTAAAACTG GTGAAATAGA AATTATTGAT GCTTTCTACA ACCCTGACGG CTCAACTGGT 35280
CAATTACCAT AAATTTGGCG CTTATCTAAG TTGTACTTGC TCTGACCGAC ACAAATAATG 35340
TCGTTTCTCA GCATATATCA AAATACACAG CAAAAATTTG GGGTTAGCTA TATAGCTAAC 35400
CCCAAATCAT ATCTAACTTT ACACTGCATC TAATTCCAAA CAGTATCCAG CCAAAAGCCT 35460
AAACTATTGT TGACTCAGCG CTAAAATATG CGATGCAACA AACAAGTCTT GGATCGCAAT 35520
ACCTGAGCTA TCAAAAATGG TCACCTCATC AGCACTTTGA CGTCCTGTTG CGGACTCGTT 35580
TATCACCTGA CCAATCTCAA TTATCGGCGT ATTTCTGCTA TGTTGAAACT CACCAATAAC 35640
AATAGATTGA GAAGCAAAGT CGCAAAACAA GCGAGCATGA CTATATAGGT CAGTTGGCAA 35700
CTCTTGCTTA CCCACTTTAT CAGCGCCCAT TGCAGAAATA TGCGTTCCTG CTTGTACCCA 35760
CTGCGCTTCA AATAAAGGCG CTTGAGCTGT GGTTGCTGTG ATAATAATAT CTGCTTGTTC 35820
ACAAGCAGCT TGTGCATCAC AAGCTTCGGC ATTAATGCCT TTTTCTAATA AACGCTTAAC 35880
CAAGTTTTCA GTTTTGCTAG CACTACGGCC AACTACCAAT ACCTTAGTTA ATGAACGAAC 35940
CTTGCTCACT GCTAGCACTT CATATTCAGC CTGATGACCG GTACCAAAAA CAGTTAATAC 36000
CGTAGCATCT TCTCTCGCGA GGTAACTCAC TGCTACTGCA TCGGCAGCAC CAGTGCGGTA 36060
AGCATTAACG GTAGTGGCAG CAATCACCGN CTGCAACATA CCGGTTAATG GATCGAGTAA 36120
AAATACGTTA GTGCCGTGGC ATGGTAAACC ATGTTTATGG TTATCAGGCC AATAGCTGCC 36180
TGTTTTCCAG CCGACAAGGT TTGGCGTTGA AGCCGACTTT AATGAGAACA TTTCATTAAG 36240
GTTCGCGCCC TGTGCATTAA CTACCGGGAA CAAGGTTGCT TTATCATCTA CGGCAGCGAC 36300
AAACGCTTCT TTAACAGCGA TATAAGCCAG CTCATGGGAG ATGAGCTTTG ATGTTTGCGC 36360
TTCAGTTAAA TAGATCATAT TACCACCCCT GCACTCGATT CCAGATCTCA TAGCCACCAT 36420
TATCACCATC AGTATCAAAT ACATGGTACT GAGCGTGCAT TGAAGCTGTT GCACAGGCGT 36480
GGTTCGGCAA AATATGTAGA CGACTACCTA CCGGGAACTG CGCTAAATCA ATAACGCCGC 36540
CATCAACTGC TTCAATAATG CCGTGCTCTT GATTAACAGT TATAACCTGT AGACCTGATA 36600
ACACGTGACC GCTGTCGTCA CACACTAAAC CATAACCACA ATCTTTTGGC TGCTCTGCAG 36660
TACCTCTATC ACCCGAAAGA GCCATCCAAC CCGCATCAAT GAAAATCCAG TTTTTATCAG 36720
GATTATGACC AATAACACTG GTCACTACCG TTGCGGCAAT ATCAGTTAAC TGACACACGT 36780
TTAGCCCTGC CATGACTAAA TCGAAGAAGG TGTACACACC CGCTCTAAÇC TCGGTGATCC 36840
CATCAAGGTT TTGATAGCTT TGCGCTGTTG GTGTTGAACC AATACTAACG ATGTCACATT 36900
GCATACCCGC TGCGCGAATG CGTCAGCAGC TTGTACAGCC GCTGCAACTT CATTTTGCGC 36960
CGCATCAATT AATTGCTGTT TTTCAAAACA TTGATATGAC TCACCAGCGT GAGTGAGTAC 37020
GCCGTGAAAA CTCGCTGCGC CAGACGTTAG TATCTGAGCA ATTTCAATCA ACTTATCGGC 37080
TTCCGGTGGA ATACCACCAC GATGGCCATC ACAATCAATT TCAATTAATG CTGGTATTTG 37140
GCAGTCATAA GAACCACAGA AATGATTTAG CTGATGCGCT TGCTCAACAC TATCAAGTAA 37200

AACTCTTGCA TTAATACCTT GGTCCAACAT TTTAGCAATA CGCGGCAACT TACCATCGGC 37260
AATACCTACT GCATAAATAA TGTCTGTGTA ACCTTTAGAT GCTAAGGCCT CGGCCTCTTT 37320
TACCGTTGAT ACAGTGACTG GTGAGTTTTT AGTGGGTAAT AAAAACTCGG CTGCTTCAAG 37380
TGATCTTAAC GTTTTAAAAT GCGGTCTTAG GTTTGCACCT AATCCTTCAA TTTTTTGGCG 37440
TAGTTGACTG AGGTTATTAA TAAATACTGG CTTATTTACA TATAAAAACG GTGTATCAAT 37500
TGCTTGATAC TGACTTTGCT GAGTCGTGGA AAGTATTTGA GTAGATGGCA TCTTTAATAT 37560
CCTAGTTCAT CAATCAATCT AACAAGTTTG ATGCCTAGCC ACAGTGGCTT GTATTCATGA 37620
TGCTTTGGAA AATGCTTATA TTCAAAGTAT TTGAAAGACA TCAAACTTCT TGTTTAATGC 37680
TCAGTATCCA CCAGCACGCA TTTATTTTAT ATTAACTATT ATCAAGATAT AGATTAGGTT 37740
CAAACCAAAT GATTAGTACT GAAGATCTAC GTTTTATCAG CGTAATCGCC AGTCATCGCA 37800
CCTTAGCTGA TGCCGCTAGA ACACTAAATA TCACGCCACC ATCAGTGACA TTAAGGTTGC 37860
AGCATATTGA AAAGAAACTA TCGATTAGCC TGATC 37895

SEQ ID NO: 2
Sequence Length: 1983
Sequence Type: Nucleic acid
Strandedness: Double strand
Topology: Linear
Molecular Type: Genomic DNA
Source: Shewanella putrefaciens SCRC-2874 (FERM BP-1625)
Sequence
ATG AAA CAG ACT CTA ATG GCT ATC TCA ATC ATG TCG CTT TTT TCA TTC 48
Met Lys Gln Thr Leu Met Ala Ile Ser Ile Met Ser Leu Phe Ser Phe
1               5                   10                  15
AAT GCG CTA GCA GCG CAA CAT GAA CAT GAC CAC ATC ACT GTT GAT TAC 96
Asn Ala Leu Ala Ala Gln His Glu His Asp His Ile Thr Val Asp Tyr
            20                  25                  30
GAA GGG AAA GCC GCA ACA GAA CAC ACC ATA GCT CAC AAC CAA GCT GTA 144
Glu Gly Lys Ala Ala Thr Glu His Thr Ile Ala His Asn Gln Ala Val
        35                  40                  45
GCT AAA ACA CTT AAC TTT GCC GAC ACG CGT GCA TTT GAG CAA TCG TCT 192
Ala Lys Thr Leu Asn Phe Ala Asp Thr Arg Ala Phe Glu Gln Ser Ser
    50                  55                  60
AAA AAT CTA GTC GCC AAG TTT GAT AAA GCA ACT GCC GAT ATA TTA CGT 240
Lys Asn Leu Val Ala Lys Phe Asp Lys Ala Thr Ala Asp Ile Leu Arg
65                  70                  75                  80

GCC GAA TTT GCT TTT ATT AGC GAT GAA ATC CCT GAC TCG GTT AAC CCG 288
Ala Glu Phe Ala Phe Ile Ser Asp Glu Ile Pro Asp Ser Val Asn Pro
                85                  90                  95
TCT CTC TAC CGT CAG GCT CAG CTT AAT ATG GTG CCT AAT GGT CTG TAT 336
Ser Leu Tyr Arg Gln Ala Gln Leu Asn Met Val Pro Asn Gly Leu Tyr
            100                 105                 110
AAA GTG AGC GAT GGC ATT TAC CAG GTC CGC GGT ACC GAC TTA TCT AAC 384
Lys Val Ser Asp Gly Ile Tyr Gln Val Arg Gly Thr Asp Leu Ser Asn
        115                 120                 125
CTT ACA CTT ATC CGC AGT GAT AAC GGT TGG ATA GCA TAC GAT GTT TTG 432
Leu Thr Leu Ile Arg Ser Asp Asn Gly Trp Ile Ala Tyr Asp Val Leu
    130                 135                 140
TTA ACC AAA GAA GCA GCA AAA GCC TCA CTA CAA TTT GCG TTA AAG AAT 480
Leu Thr Lys Glu Ala Ala Lys Ala Ser Leu Gln Phe Ala Leu Lys Asn
145                 150                 155                 160
CTA CCT AAA GAT GGC GAT TTA CCC GTT GTT GCG ATG ATT TAC TCC CAT 528
Leu Pro Lys Asp Gly Asp Leu Pro Val Val Ala Met Ile Tyr Ser His
                165                 170                 175
AGC CAT GCG GAC CAC TTT GGC GGA GCT CGC GGT GTT CAA GAG ATG TTC 576
Ser His Ala Asp His Phe Gly Gly Ala Arg Gly Val Gln Glu Met Phe
            180                 185                 190
CCT GAT GTC AAA GTC TAC GGC TCA GAT AAC ATC ACT AAA GAA ATT GTC 624
Pro Asp Val Lys Val Tyr Gly Ser Asp Asn Ile Thr Lys Glu Ile Val
        195                 200                 205
GAT GAG AAC GTA CTT GCC GGT AAC GCC ATG AGC CGC CGC GCA GCT TAT 672
Asp Glu Asn Val Leu Ala Gly Asn Ala Met Ser Arg Arg Ala Ala Tyr
    210                 215                 220
CAA TAC GGC GCA ACA CTG GGC AAA CAT GAC CAC GGT ATT GTT GAT GCT 720
Gln Tyr Gly Ala Thr Leu Gly Lys His Asp His Gly Ile Val Asp Ala
225                 230                 235                 240
GCG CTA GGT AAA GGT CTA TCA AAA GGT GAA ATC ACT TAC GTC GCC CCA 768
Ala Leu Gly Lys Gly Leu Ser Lys Gly Glu Ile Thr Tyr Val Ala Pro
                245                 250                 255
GAC TAC ACC TTA AAC AGT GAA GGC AAA TGG GAA ACG CTG ACG ATT GAT 816
Asp Tyr Thr Leu Asn Ser Glu Gly Lys Trp Glu Thr Leu Thr Ile Asp
            260                 265                 270

GGT CTA GAG ATG GTG TTT ATG GAT GCC TCG GGC ACC GAA GCT GAG TCA 864
Gly Leu Glu Met Val Phe Met Asp Ala Ser Gly Thr Glu Ala Glu Ser
        275                 280                 285
GAA ATG ATC ACT TAT ATT CCC TCT AAA AAA GCG CTC TGG ACG GCG GAG 912
Glu Met Ile Thr Tyr Ile Pro Ser Lys Lys Ala Leu Trp Thr Ala Glu
    290                 295                 300
CTT ACC TAT CAA GGT ATG CAC AAC ATT TAT ACG CTG CGC GGC GCT AAA 960
Leu Thr Tyr Gln Gly Met His Asn Ile Tyr Thr Leu Arg Gly Ala Lys
305                 310                 315                 320
GTA CGT GAT GCG CTC AAG TGG TCA AAA GAT ATC AAC GAA ATG ATC AAT 1008
Val Arg Asp Ala Leu Lys Trp Ser Lys Asp Ile Asn Glu Met Ile Asn
                325                 330                 335
GCC TTT GGT CAA GAT GTC GAA GTG CTG TTT GCC TCG CAC TCT GCG CCA 1056
Ala Phe Gly Gln Asp Val Glu Val Leu Phe Ala Ser His Ser Ala Pro
            340                 345                 350
GTG TGG GGT AAC CAA GCG ATC AAC GAT TTC TTA CGC CTA CAG CGT GAT 1104
Val Trp Gly Asn Gln Ala Ile Asn Asp Phe Leu Arg Leu Gln Arg Asp
        355                 360                 365
AAC TAC GGC CTA GTG CAC AAT CAA ACC TTG AGA CTT GCC AAC GAT GGT 1152
Asn Tyr Gly Leu Val His Asn Gln Thr Leu Arg Leu Ala Asn Asp Gly
    370                 375                 380
GTC GGT ATA CAA GAT ATT GGC GAT GCG ATT CAA GAC ACG ATT CCA GAG 1200
Val Gly Ile Gln Asp Ile Gly Asp Ala Ile Gln Asp Thr Ile Pro Glu
385                 390                 395                 400
TCT ATC TAC AAG ACG TGG CAT ACC AAT GGT TAC CAC GGC ACT TAT AGC 1248
Ser Ile Tyr Lys Thr Trp His Thr Asn Gly Tyr His Gly Thr Tyr Ser
                405                 410                 415
CAT AAC GCT AAA GCG GTT TAT AAC AAG TAT CTA GGC TAC TTC GAT ATG 1296
His Asn Ala Lys Ala Val Tyr Asn Lys Tyr Leu Gly Tyr Phe Asp Met
            420                 425                 430
AAC CCA GCC AAC CTT AAT CCG CTG CCA ACC AAG CAA GAA TCT GCC AAG 1344
Asn Pro Ala Asn Leu Asn Pro Leu Pro Thr Lys Gln Glu Ser Ala Lys
        435                 440                 445
TTT GTC GAA TAC ATG GGC GGC GCA GAT GCC GCA ATT AAG CGC GCT AAA 1392
Phe Val Glu Tyr Met Gly Gly Ala Asp Ala Ala Ile Lys Arg Ala Lys
    450                 455                 460

GAT GAT TAC GCT CAA GGT GAA TAC CGC TTT GTT GCA ACG GCA TTA AAT 1440
Asp Asp Tyr Ala Gln Gly Glu Tyr Arg Phe Val Ala Thr Ala Leu Asn
465                 470                 475                 480
AAG GTG GTG ATG GCC GAG CCA GAA AAT GAC TCC GCT CGT CAA TTG CTA 1488
Lys Val Val Met Ala Glu Pro Glu Asn Asp Ser Ala Arg Gln Leu Leu
                485                 490                 495
GCC GAT ACC TAT GAG CAA CTT GGT TAT CAA GCA GAA GGG GCT GGC TGG 1536
Ala Asp Thr Tyr Glu Gln Leu Gly Tyr Gln Ala Glu Gly Ala Gly Trp
            500                 505                 510
AGA AAC ATT TAC TTA ACT GGC GCA CAA GAG CTA CGA GTA GGT ATT CAA 1584
Arg Asn Ile Tyr Leu Thr Gly Ala Gln Glu Leu Arg Val Gly Ile Gln
        515                 520                 525
GCT GGC GCG CCT AAA ACC GCA TCG GCA GAT GTC ATC AGT GAA ATG GAC 1632
Ala Gly Ala Pro Lys Thr Ala Ser Ala Asp Val Ile Ser Glu Met Asp
    530                 535                 540
ATG CCG ACT CTA TTT GAC TTC CTC GCG GTG AAG ATT GAT AGT CAA CAG 1680
Met Pro Thr Leu Phe Asp Phe Leu Ala Val Lys Ile Asp Ser Gln Gln
545                 550                 555                 560
GCG GCT AAG CAC GGC TTA GTT AAG ATG AAT GTT ATC ACC CCT GAT ACT 1728
Ala Ala Lys His Gly Leu Val Lys Met Asn Val Ile Thr Pro Asp Thr
                565                 570                 575
AAA GAT ATT CTC TAT ATT GAG CTA AGC AAC GGT AAC TTA AGC AAC GCA 1776
Lys Asp Ile Leu Tyr Ile Glu Leu Ser Asn Gly Asn Leu Ser Asn Ala
            580                 585                 590
GTG GTC GAC AAA GAG CAA GCA GCT GAC GCA AAC CTT ATG GTT AAT AAA 1824
Val Val Asp Lys Glu Gln Ala Ala Asp Ala Asn Leu Met Val Asn Lys
        595                 600                 605
GCT GAC GTT AAC CGC ATC TTA CTT GGC CAA GTA ACC CTA AAA GCG TTA 1872
Ala Asp Val Asn Arg Ile Leu Leu Gly Gln Val Thr Leu Lys Ala Leu
    610                 615                 620
TTA GCC AGC GGC GAT GCC AAG CTC ACT GGT GAT AAA ACG GCA TTT AGT 1920
Leu Ala Ser Gly Asp Ala Lys Leu Thr Gly Asp Lys Thr Ala Phe Ser
625                 630                 635                 640
AAA ATA GCC GAT AGC ATG GTC GAG TTT ACA CCT GAC TTC GAA ATC GTA 1968
Lys Ile Ala Asp Ser Met Val Glu Phe Thr Pro Asp Phe Glu Ile Val
                645                 650                 655

CCA ACG CCT GTT AAA                                                    1983
Pro Thr Pro Val Lys
            660

SEQ ID NO: 3
Sequence Length: 661
Sequence Type: Amino acid
Molecular Type: Peptide
Sequence
Met Lys Gln Thr Leu Met Ala Ile Ser Ile Met Ser Leu Phe Ser Phe
 1               5                  10                  15
Asn Ala Leu Ala Ala Gln His Glu His Asp His Ile Thr Val Asp Tyr
            20                  25                  30
Glu Gly Lys Ala Ala Thr Glu His Thr Ile Ala His Asn Gln Ala Val
        35                  40                  45
Ala Lys Thr Leu Asn Phe Ala Asp Thr Arg Ala Phe Glu Gln Ser Ser
    50                  55                  60
Lys Asn Leu Val Ala Lys Phe Asp Lys Ala Thr Ala Asp Ile Leu Arg
65                  70                  75                  80
Ala Glu Phe Ala Phe Ile Ser Asp Glu Ile Pro Asp Ser Val Asn Pro
                85                  90                  95
Ser Leu Tyr Arg Gln Ala Gln Leu Asn Met Val Pro Asn Gly Leu Tyr
            100                 105                 110
Lys Val Ser Asp Gly Ile Tyr Gln Val Arg Gly Thr Asp Leu Ser Asn
        115                 120                 125
Leu Thr Leu Ile Arg Ser Asp Asn Gly Trp Ile Ala Tyr Asp Val Leu
    130                 135                 140
Leu Thr Lys Glu Ala Ala Lys Ala Ser Leu Gln Phe Ala Leu Lys Asn
145                 150                 155                 160
Leu Pro Lys Asp Gly Asp Leu Pro Val Val Ala Met Ile Tyr Ser His
                165                 170                 175
Ser His Ala Asp His Phe Gly Gly Ala Arg Gly Val Gln Glu Met Phe
            180                 185                 190
Pro Asp Val Lys Val Tyr Gly Ser Asp Asn Ile Thr Lys Glu Ile Val
        195                 200                 205

Asp Glu Asn Val Leu Ala Gly Asn Ala Met Ser Arg Arg Ala Ala Tyr
    210                 215                 220
Gln Tyr Gly Ala Thr Leu Gly Lys His Asp His Gly Ile Val Asp Ala
225                 230                 235                 240
Ala Leu Gly Lys Gly Leu Ser Lys Gly Glu Ile Thr Tyr Val Ala Pro
                245                 250                 255
Asp Tyr Thr Leu Asn Ser Glu Gly Lys Trp Glu Thr Leu Thr Ile Asp
            260                 265                 270
Gly Leu Glu Met Val Phe Met Asp Ala Ser Gly Thr Glu Ala Glu Ser
        275                 280                 285
Glu Met Ile Thr Tyr Ile Pro Ser Lys Lys Ala Leu Trp Thr Ala Glu
    290                 295                 300
Leu Thr Tyr Gln Gly Met His Asn Ile Tyr Thr Leu Arg Gly Ala Lys
305                 310                 315                 320
Val Arg Asp Ala Leu Lys Trp Ser Lys Asp Ile Asn Glu Met Ile Asn
                325                 330                 335
Ala Phe Gly Gln Asp Val Glu Val Leu Phe Ala Ser His Ser Ala Pro
            340                 345                 350
Val Trp Gly Asn Gln Ala Ile Asn Asp Phe Leu Arg Leu Gln Arg Asp
        355                 360                 365
Asn Tyr Gly Leu Val His Asn Gln Thr Leu Arg Leu Ala Asn Asp Gly
    370                 375                 380
Val Gly Ile Gln Asp Ile Gly Asp Ala Ile Gln Asp Thr Ile Pro Glu
385                 390                 395                 400
Ser Ile Tyr Lys Thr Trp His Thr Asn Gly Tyr His Gly Thr Tyr Ser
                405                 410                 415
His Asn Ala Lys Ala Val Tyr Asn Lys Tyr Leu Gly Tyr Phe Asp Met
            420                 425                 430
Asn Pro Ala Asn Leu Asn Pro Leu Pro Thr Lys Gln Glu Ser Ala Lys
        435                 440                 445
Phe Val Glu Tyr Met Gly Gly Ala Asp Ala Ala Ile Lys Arg Ala Lys
    450                 455                 460
Asp Asp Tyr Ala Gln Gly Glu Tyr Arg Phe Val Ala Thr Ala Leu Asn
465                 470                 475                 480
Lys Val Val Met Ala Glu Pro Glu Asn Asp Ser Ala Arg Gln Leu Leu
                485                 490                 495

Ala Asp Thr Tyr Glu Gln Leu Gly Tyr Gln Ala Glu Gly Ala Gly Trp
            500                 505                 510
Arg Asn Ile Tyr Leu Thr Gly Ala Gln Glu Leu Arg Val Gly Ile Gln
        515                 520                 525
Ala Gly Ala Pro Lys Thr Ala Ser Ala Asp Val Ile Ser Glu Met Asp
    530                 535                 540
Met Pro Thr Leu Phe Asp Phe Leu Ala Val Lys Ile Asp Ser Gln Gln
545                 550                 555                 560
Ala Ala Lys His Gly Leu Val Lys Met Asn Val Ile Thr Pro Asp Thr
                565                 570                 575
Lys Asp Ile Leu Tyr Ile Glu Leu Ser Asn Gly Asn Leu Ser Asn Ala
            580                 585                 590
Val Val Asp Lys Glu Gln Ala Ala Asp Ala Asn Leu Met Val Asn Lys
        595                 600                 605
Ala Asp Val Asn Arg Ile Leu Leu Gly Gln Val Thr Leu Lys Ala Leu
    610                 615                 620
Leu Ala Ser Gly Asp Ala Lys Leu Thr Gly Asp Lys Thr Ala Phe Ser
625                 630                 635                 640
Lys Ile Ala Asp Ser Met Val Glu Phe Thr Pro Asp Phe Glu Ile Val
                645                 650                 655
Pro Thr Pro Val Lys
            660

SEQ ID NO: 4
Sequence Length: 831
Sequence Type: Nucleic acid
Strandedness: Double strand
Topology: Linear
Molecular Type: Genomic DNA
Source: Shewanella putrefaciens SCRC-2874 (FERM BP-1625)
Sequence
ATG GTA AGA GGC TAT TTG CGC GCT TTA TTG TCA CAA CAT AGT GAA ATA 48
Met Val Arg Gly Tyr Leu Arg Ala Leu Leu Ser Gln His Ser Glu Ile
                5                   10                  15

CGC CCC AAT GAA TGG CGC TTT GAA TAT GGC GAC AAA GGT AAG CCT AGA 96
Arg Pro Asn Glu Trp Arg Phe Glu Tyr Gly Asp Lys Gly Lys Pro Arg
            20                  25                  30
TTG AGT GAT GCG CAA TTT GCT CAA ACC GGG GTC CAC TTT AAT GTG AGT 144
Leu Ser Asp Ala Gln Phe Ala Gln Thr Gly Val His Phe Asn Val Ser
        35                  40                  45
CAT AGT GGA GAT TGG CTA TTA GTA GGC ATT TGC ACT GCT GAT AAT AAA 192
His Ser Gly Asp Trp Leu Leu Val Gly Ile Cys Thr Ala Asp Asn Lys
    50                  55                  60
GGC GCC AGT CAG GCA AGC AAG GAG GAA ACT GAC TCT GCT AGT ATT GAG 240
Gly Ala Ser Gln Ala Ser Lys Glu Glu Thr Asp Ser Ala Ser Ile Glu
65                  70                  75                  80
TTT GGC GTC GAC ATT GAG CGT TGC CGT AAC AGC ACC AAT ATC CAC TCT 288
Phe Gly Val Asp Ile Glu Arg Cys Arg Asn Ser Thr Asn Ile His Ser
                85                  90                  95
ATT CTT AGT CAT TAT TTC TCT GAA TCA GAA AAG CGA GCC TTG TTA GCG 336
Ile Leu Ser His Tyr Phe Ser Glu Ser Glu Lys Arg Ala Leu Leu Ala
            100                 105                 110
TTA CCA GAG GCC TTG CAG CGA GAC CGC TTT TTT GAT TTG TGG GCG CTC 384
Leu Pro Glu Ala Leu Gln Arg Asp Arg Phe Phe Asp Leu Trp Ala Leu
        115                 120                 125
AAG GAG TCT TAC ATT AAA GCG AAA GGA CTT GGG CTG GCA TTA TCG CTA 432
Lys Glu Ser Tyr Ile Lys Ala Lys Gly Leu Gly Leu Ala Leu Ser Leu
    130                 135                 140
AAA TCT TTT GCG TTT GAC TTC TCT GCA CTG AGC GAA ACT TTT CTT GGA 480
Lys Ser Phe Ala Phe Asp Phe Ser Ala Leu Ser Glu Thr Phe Leu Gly
145                 150                 155                 160
GTT AAT GCA CCT AAA AGC TTG AGC CAT TGT GTT GAT ATT TCC GAT GCT 528
Val Asn Ala Pro Lys Ser Leu Ser His Cys Val Asp Ile Ser Asp Ala
                165                 170                 175
ATT GCG GAT CAC AAG GTT GAG CAT CAA CTT AAT CAG CGA CAG GTT TTG 576
Ile Ala Asp His Lys Val Glu His Gln Leu Asn Gln Arg Gln Val Leu
            180                 185                 190
TTA AAA CAA GAT ATT GGT CTT GCT TTA CTA GAG TCG AGT TCT AAT AAG 624
Leu Lys Gln Asp Ile Gly Leu Ala Leu Leu Glu Ser Ser Ser Asn Lys
        195                 200                 205

CCT AAC GCT GAG CCA CAA AAG TCT GGT TTA GGT TTG ATT GAG GCT AAA 672
Pro Asn Ala Glu Pro Gln Lys Ser Gly Leu Gly Leu Ile Glu Ala Lys
    210                 215                 220
GAA CAG CAA ATG AAC GCT GCT GAT AAT TGG CAT TGT TTA CTG GGC CAT 720
Glu Gln Gln Met Asn Ala Ala Asp Asn Trp His Cys Leu Leu Gly His
225                 230                 235                 240
CTT GAT GAT AGT TAT CGT TTT GCA CTG AGT ATT GGT CAG TGT CAG CAA 768
Leu Asp Asp Ser Tyr Arg Phe Ala Leu Ser Ile Gly Gln Cys Gln Gln
                245                 250                 255
ATA AGT ATT GCA GCA GAA GAA GTG AAT TTT AAA GCT GTT GTT CGA GCT 816
Ile Ser Ile Ala Ala Glu Glu Val Asn Phe Lys Ala Val Val Arg Ala
            260                 265                 270
TCA GCT AAG ACT AGC 831
Ser Ala Lys Thr Ser
        275

SEQ ID NO: 5
Sequence Length: 277
Sequence Type: Amino acid
Molecular Type: Peptide
Sequence
Met Val Arg Gly Tyr Leu Arg Ala Leu Leu Ser Gln His Ser Glu Ile
                5                   10                  15
Arg Pro Asn Glu Trp Arg Phe Glu Tyr Gly Asp Lys Gly Lys Pro Arg
            20                  25                  30
Leu Ser Asp Ala Gln Phe Ala Gln Thr Gly Val His Phe Asn Val Ser
        35                  40                  45
His Ser Gly Asp Trp Leu Leu Val Gly Ile Cys Thr Ala Asp Asn Lys
    50                  55                  60
Gly Ala Ser Gln Ala Ser Lys Glu Glu Thr Asp Ser Ala Ser Ile Glu
65                  70                  75                  80
Phe Gly Val Asp Ile Glu Arg Cys Arg Asn Ser Thr Asn Ile His Ser
                85                  90                  95
Ile Leu Ser His Tyr Phe Ser Glu Ser Glu Lys Arg Ala Leu Leu Ala
            100                 105                 110

Leu Pro Glu Ala Leu Gln Arg Asp Arg Phe Phe Asp Leu Trp Ala Leu
        115                 120                 125
Lys Glu Ser Tyr Ile Lys Ala Lys Gly Leu Gly Leu Ala Leu Ser Leu
    130                 135                 140
Lys Ser Phe Ala Phe Asp Phe Ser Ala Leu Ser Glu Thr Phe Leu Gly
145                 150                 155                 160
Val Asn Ala Pro Lys Ser Leu Ser His Cys Val Asp Ile Ser Asp Ala
                165                 170                 175
Ile Ala Asp His Lys Val Glu His Gln Leu Asn Gln Arg Gln Val Leu
            180                 185                 190
Leu Lys Gln Asp Ile Gly Leu Ala Leu Leu Glu Ser Ser Ser Asn Lys
        195                 200                 205
Pro Asn Ala Glu Pro Gln Lys Ser Gly Leu Gly Leu Ile Glu Ala Lys
    210                 215                 220
Glu Gln Gln Met Asn Ala Ala Asp Asn Trp His Cys Leu Leu Gly His
225                 230                 235                 240
Leu Asp Asp Ser Tyr Arg Phe Ala Leu Ser Ile Gly Gln Cys Gln Gln
                245                 250                 255
Ile Ser Ile Ala Ala Glu Glu Val Asn Phe Lys Ala Val Val Arg Ala
            260                 265                 270
Ser Ala Lys Thr Ser
        275

SEQ ID NO: 6
Sequence Length: 2910
Sequence Type: Nucleic acid
Strandedness: Double strand
Topology: Linear
Molecular Type: Genomic DNA
Source: Shewanella putrefaciens SCRC-2874 (FERM BP-1625)
Sequence
ATG AGT ATG TTT TTA AAT TCA AAA CTT TCG CGC TCA GTC AAA CTT GCC 48
Met Ser Met Phe Leu Asn Ser Lys Leu Ser Arg Ser Val Lys Leu Ala
1               5                   10                  15

ATA TCC GCA GGC TTA ACA GCC TCG CTA GCT ATG CCT GTT TTT GCA GAA 96
Ile Ser Ala Gly Leu Thr Ala Ser Leu Ala Met Pro Val Phe Ala Glu
            20                  25                  30
GAA ACT GCT GCT GAA GAA CAA ATA GAA AGA GTC GCA GTG ACC GGA TCG 144
Glu Thr Ala Ala Glu Glu Gln Ile Glu Arg Val Ala Val Thr Gly Ser
        35                  40                  45
CGA ATC GCT AAA GCA GAG CTA ACT CAA CCA GCT CCA GTC GTC AGC CTT 192
Arg Ile Ala Lys Ala Glu Leu Thr Gln Pro Ala Pro Val Val Ser Leu
    50                  55                  60
TCA GCC GAA GAA CTG ACA AAA TTT GGT AAT CAA GAT TTA GGT AGC GTA 240
Ser Ala Glu Glu Leu Thr Lys Phe Gly Asn Gln Asp Leu Gly Ser Val
65                  70                  75                  80
CTA GCA GAA TTA CCT GCT ATT GGT GCA ACC AAC ACT ATT ATT GGT AAT 288
Leu Ala Glu Leu Pro Ala Ile Gly Ala Thr Asn Thr Ile Ile Gly Asn
                85                  90                  95
AAC AAT AGC AAC TCA AGC GCA GGT GTT AGC TCA GCA GAC TTG CGT CGT 336
Asn Asn Ser Asn Ser Ser Ala Gly Val Ser Ser Ala Asp Leu Arg Arg
            100                 105                 110
CTA GGT GCT AAC AGA ACC TTA GTA TTA GTC AAC GGT AAG CGC TAC GTT 384
Leu Gly Ala Asn Arg Thr Leu Val Leu Val Asn Gly Lys Arg Tyr Val
        115                 120                 125
GCC GGC CAA CCG GGC TCA GCT GAG GTA GAT TTG TCA ACT ATA CCA ACT 432
Ala Gly Gln Pro Gly Ser Ala Glu Val Asp Leu Ser Thr Ile Pro Thr
    130                 135                 140
AGC ATG ATC TCG CGA GTT GAG ATT GTA ACC GGC GGT GCT TCA GCA ATT 480
Ser Met Ile Ser Arg Val Glu Ile Val Thr Gly Gly Ala Ser Ala Ile
145                 150                 155                 160
TAT GGT TCG GAC GCT GTA TCA GGT GTT ATC AAC GTT ATC CTT AAA GAA 528
Tyr Gly Ser Asp Ala Val Ser Gly Val Ile Asn Val Ile Leu Lys Glu
                165                 170                 175
GAC TTT GAA GGC TTT GAG TTT AAC GCA CGT ACT AGC GGT TCT ACT GAA 576
Asp Phe Glu Gly Phe Glu Phe Asn Ala Arg Thr Ser Gly Ser Thr Glu
            180                 185                 190
AGT GTA GGC ACT CAA GAG CAC TCT TTT GAC ATT TTG GGT GGT GCA AAC 624
Ser Val Gly Thr Gln Glu His Ser Phe Asp Ile Leu Gly Gly Ala Asn
        195                 200                 205

GTT GCA GAT GGA CGT GGT AAT GTA ACC TTC TAC GCA GGT TAT GAA CGT 672
Val Ala Asp Gly Arg Gly Asn Val Thr Phe Tyr Ala Gly Tyr Glu Arg
    210                 215                 220
ACA AAA GAA GTC ATG GCT ACC GAC ATT CGC CAA TTC GAT GCT TGG GGA 720
Thr Lys Glu Val Met Ala Thr Asp Ile Arg Gln Phe Asp Ala Trp Gly
225                 230                 235                 240
ACA ATT AAA AAC GAA GCC GAT GGT GGT GAA GAT GAT GGT ATT CCA GAC 768
Thr Ile Lys Asn Glu Ala Asp Gly Gly Glu Asp Asp Gly Ile Pro Asp
                245                 250                 255
AGA CTA CGT GTA CCA CGA GTT TAT TCT GAA ATG ATT AAT GCT ACC GGT 816
Arg Leu Arg Val Pro Arg Val Tyr Ser Glu Met Ile Asn Ala Thr Gly
            260                 265                 270
GTT ATC AAT GCA TTT GGT GGT GGA ATT GGT CGC TCA ACC TTT GAC AGT 864
Val Ile Asn Ala Phe Gly Gly Gly Ile Gly Arg Ser Thr Phe Asp Ser
        275                 280                 285
AAC GGC AAT CCT ATT GCA CAA CAA GAA CGT GAT GGG ACT AAC AGC TTT 912
Asn Gly Asn Pro Ile Ala Gln Gln Glu Arg Asp Gly Thr Asn Ser Phe
    290                 295                 300
GCA TTT GGT TCA TTC CCT AAT GGC TGT GAC ACA TGT TTC AAC ACT GAA 960
Ala Phe Gly Ser Phe Pro Asn Gly Cys Asp Thr Cys Phe Asn Thr Glu
305                 310                 315                 320
GCA TAC GAA AAC TAT ATT CCA GGG GTA GAA AGA ATA AAC GTT GGC TCA 1008
Ala Tyr Glu Asn Tyr Ile Pro Gly Val Glu Arg Ile Asn Val Gly Ser
                325                 330                 335
TCA TTC AAC TTT GAT TTT ACC GAT AAC ATT CAA TTT TAC ACT GAC TTC 1056
Ser Phe Asn Phe Asp Phe Thr Asp Asn Ile Gln Phe Tyr Thr Asp Phe
            340                 345                 350
AGA TAT GTA AAG TCA GAT ATT CAG CAA CAA TTT CAG CCT TCA TTC CGT 1104
Arg Tyr Val Lys Ser Asp Ile Gln Gln Gln Phe Gln Pro Ser Phe Arg
        355                 360                 365
TTT GGT AAC ATT AAT ATC AAT GTT GAA GAT AAC GCC TTT TTG AAT GAC 1152
Phe Gly Asn Ile Asn Ile Asn Val Glu Asp Asn Ala Phe Leu Asn Asp
    370                 375                 380
GAC TTG CGT CAG CAA ATG CTC GAT GCG GGT CAA ACC AAT GCT AGT TTT 1200
Asp Leu Arg Gln Gln Met Leu Asp Ala Gly Gln Thr Asn Ala Ser Phe
385                 390                 395                 400

GCC AAG TTT TTT GAT GAA TTA GGA AAT CGC TCA GCA GAA AAT AAA CGC 1248
Ala Lys Phe Phe Asp Glu Leu Gly Asn Arg Ser Ala Glu Asn Lys Arg
                405                 410                 415
GAA CTT TTC CGT TAC GTA GGT GGC TTT AAA GGT GGC TTT GAT ATT AGC 1296
Glu Leu Phe Arg Tyr Val Gly Gly Phe Lys Gly Gly Phe Asp Ile Ser
            420                 425                 430
GAA ACC ATA TTT GAT TAC GAC CTT TAC TAT GTT TAT GGC GAG ACT AAT 1344
Glu Thr Ile Phe Asp Tyr Asp Leu Tyr Tyr Val Tyr Gly Glu Thr Asn
        435                 440                 445
AAC CGT CGT AAA ACC CTT AAT GAC CTA ATT CCT GAT AAC TTT GTC GCA 1392
Asn Arg Arg Lys Thr Leu Asn Asp Leu Ile Pro Asp Asn Phe Val Ala
    450                 455                 460
GCT GTC GAC TCT GTT ATT GAT CCT GAT ACT GGC TTA GCA GCG TGT CGC 1440
Ala Val Asp Ser Val Ile Asp Pro Asp Thr Gly Leu Ala Ala Cys Arg
465                 470                 475                 480
TCA CAA GTA GCA AGC GCT CAA GGC GAT GAC TAT ACA GAT CCC GCG TCT 1488
Ser Gln Val Ala Ser Ala Gln Gly Asp Asp Tyr Thr Asp Pro Ala Ser
                485                 490                 495
GTA AAT GGT AGC GAC TGT GTT GCT TAT AAC CCA TTT GGC ATG GGT CAA 1536
Val Asn Gly Ser Asp Cys Val Ala Tyr Asn Pro Phe Gly Met Gly Gln
            500                 505                 510
GCT TCA GCA GAA GCC CGC GAC TGG GTT TCT GCT GAT GTG ACT CGT GAA 1584
Ala Ser Ala Glu Ala Arg Asp Trp Val Ser Ala Asp Val Thr Arg Glu
        515                 520                 525
GAC AAA ATA ACT CAA CAA GTG ATT GGT GGT ACT CTC GGT ACC GAT TCT 1632
Asp Lys Ile Thr Gln Gln Val Ile Gly Gly Thr Leu Gly Thr Asp Ser
    530                 535                 540
GAA GAA CTA TTT GAG CTT CAA GGT GGT GCA ATC GCT ATG GTT GTT GGT 1680
Glu Glu Leu Phe Glu Leu Gln Gly Gly Ala Ile Ala Met Val Val Gly
545                 550                 555                 560
TTT GAA TAC CGT GAA GAA ACG TCT GGT TCA ACA ACC GAT GAA TTT ACT 1728
Phe Glu Tyr Arg Glu Glu Thr Ser Gly Ser Thr Thr Asp Glu Phe Thr
                565                 570                 575
AAA GCA GGT TTC TTG ACA AGC GCT GCA ACG CCA GAT TCT TAT GGC GAA 1776
Lys Ala Gly Phe Leu Thr Ser Ala Ala Thr Pro Asp Ser Tyr Gly Glu
            580                 585                 590

TAC GAC GTG ACT GAG TAT TTT GTT GAG GTG AAC ATC CCA GTA CTA AAA 1824
Tyr Asp Val Thr Glu Tyr Phe Val Glu Val Asn Ile Pro Val Leu Lys
        595                 600                 605
GAA TTA CCT TTT GCA CAT GAG TTG AGC TTT GAC GGT GCA TAC CGT AAT 1872
Glu Leu Pro Phe Ala His Glu Leu Ser Phe Asp Gly Ala Tyr Arg Asn
    610                 615                 620
GCT GAT TAC TCA CAT GCC GGT AAG ACT GAA GCA TGG AAA GCT GGT ATG 1920
Ala Asp Tyr Ser His Ala Gly Lys Thr Glu Ala Trp Lys Ala Gly Met
625                 630                 635                 640
TTC TAC TCA CCA TTA GAG CAA CTT GCA TTA CGT GGT ACG GTA GGT GAA 1968
Phe Tyr Ser Pro Leu Glu Gln Leu Ala Leu Arg Gly Thr Val Gly Glu
                645                 650                 655
GCA GTA CGA GCA CCA AAC ATT GCA GAA GCC TTT AGT CCA CGC TCT CCT 2016
Ala Val Arg Ala Pro Asn Ile Ala Glu Ala Phe Ser Pro Arg Ser Pro
            660                 665                 670
GGT TTT GGC CGC GTT TCA GAT CCA TGT GAT GCA GAT AAC ATT AAT GAC 2064
Gly Phe Gly Arg Val Ser Asp Pro Cys Asp Ala Asp Asn Ile Asn Asp
        675                 680                 685
GAT CCG GAT CGC GTG TCA AAC TGT GCA GCA TTG GGG ATC CCT CCA GGA 2112
Asp Pro Asp Arg Val Ser Asn Cys Ala Ala Leu Gly Ile Pro Pro Gly
    690                 695                 700
TTC CAA GCT AAT GAT AAC GTC AGT GTA GAT ACC TTA TCT GGT GGT AAC 2160
Phe Gln Ala Asn Asp Asn Val Ser Val Asp Thr Leu Ser Gly Gly Asn
705                 710                 715                 720
CCA GAT CTA AAA CCT GAA ACA TCA ACA TCC TTT ACA GGT GGT CTT GTT 2208
Pro Asp Leu Lys Pro Glu Thr Ser Thr Ser Phe Thr Gly Gly Leu Val
                725                 730                 735
TGG ACA CCA ACG TTT GCT GAC AAT CTA TCA TTC ACT GTC GAT TAT TAT 2256
Trp Thr Pro Thr Phe Ala Asp Asn Leu Ser Phe Thr Val Asp Tyr Tyr
            740                 745                 750
GAT ATT CAA ATT GAG GAT GCT ATT TTG TCA GTA GCC ACC CAG ACT GTG 2304
Asp Ile Gln Ile Glu Asp Ala Ile Leu Ser Val Ala Thr Gln Thr Val
        755                 760                 765
GCT GAT AAC TGT GTT GAC TCA ACT GGC GGA CCT GAC ACC GAC TTC TGT 2352
Ala Asp Asn Cys Val Asp Ser Thr Gly Gly Pro Asp Thr Asp Phe Cys
    770                 775                 780

AGT CAA GTT GAT CGT AAT CCA ACG ACC TAT GAT ATT GAA CTT GTT CGC 2400
Ser Gln Val Asp Arg Asn Pro Thr Thr Tyr Asp Ile Glu Leu Val Arg
785                 790                 795                 800
TCT GGT TAT CTA AAT GCC GCG GCA TTG AAT ACC AAA GGT ATT GAA TTT 2448
Ser Gly Tyr Leu Asn Ala Ala Ala Leu Asn Thr Lys Gly Ile Glu Phe
                805                 810                 815
CAA GCT GCA TAC TCA TTA GAT CTA GAG TCT TTC AAC GCG CCT GGT GAA 2496
Gln Ala Ala Tyr Ser Leu Asp Leu Glu Ser Phe Asn Ala Pro Gly Glu
            820                 825                 830
CTA CGC TTC AAC CTA TTG GGG AAC CAA TTA CTT GAA CTA GAA CGT CTT 2544
Leu Arg Phe Asn Leu Leu Gly Asn Gln Leu Leu Glu Leu Glu Arg Leu
        835                 840                 845
GAA TTC CAA AAT CGT CCT GAT GAG ATT AAT GAT GAA AAA GGC GAA GTA 2592
Glu Phe Gln Asn Arg Pro Asp Glu Ile Asn Asp Glu Lys Gly Glu Val
    850                 855                 860
GGT GAT CCA GAG CTG CAG TTC CGC CTA GGC ATC GAT TAC CGT CTA GAT 2640
Gly Asp Pro Glu Leu Gln Phe Arg Leu Gly Ile Asp Tyr Arg Leu Asp
865                 870                 875                 880
GAT CTA AGT GTT AGC TGG AAC ACG CGT TAT ATT GAT AGC GTA GTA ACT 2688
Asp Leu Ser Val Ser Trp Asn Thr Arg Tyr Ile Asp Ser Val Val Thr
                885                 890                 895
TAT GAT GTC TCT GAA AAT GGT GGC TCT CCT GAA GAT TTA TAT CCA GGC 2736
Tyr Asp Val Ser Glu Asn Gly Gly Ser Pro Glu Asp Leu Tyr Pro Gly
            900                 905                 910
CAC ATA GGC TCA ATG ACA ACT CAT GAC TTG AGC GCT ACA TAC TAC ATC 2784
His Ile Gly Ser Met Thr Thr His Asp Leu Ser Ala Thr Tyr Tyr Ile
        915                 920                 925
AAT GAG AAC TTC ATG ATT AAC GGT GGT GTA CGT AAC CTA TTT GAC GCA 2832
Asn Glu Asn Phe Met Ile Asn Gly Gly Val Arg Asn Leu Phe Asp Ala
    930                 935                 940
CTT CCA CCT GGA TAC ACT AAC GAT GCG CTA TAT GAT CTA GTT GGT CGC 2880
Leu Pro Pro Gly Tyr Thr Asn Asp Ala Leu Tyr Asp Leu Val Gly Arg
945                 950                 955                 960
CGT GCA TTC CTA GGT ATT AAG GTA ATG ATG 2910
Arg Ala Phe Leu Gly Ile Lys Val Met Met
                965                 970

SEQ ID NO: 7
Sequence Length: 970
Sequence Type: Amino acid
Molecular Type: Peptide
Sequence
Met Ser Met Phe Leu Asn Ser Lys Leu Ser Arg Ser Val Lys Leu Ala
1               5                   10                  15
Ile Ser Ala Gly Leu Thr Ala Ser Leu Ala Met Pro Val Phe Ala Glu
            20                  25                  30
Glu Thr Ala Ala Glu Glu Gln Ile Glu Arg Val Ala Val Thr Gly Ser
        35                  40                  45
Arg Ile Ala Lys Ala Glu Leu Thr Gln Pro Ala Pro Val Val Ser Leu
    50                  55                  60
Ser Ala Glu Glu Leu Thr Lys Phe Gly Asn Gln Asp Leu Gly Ser Val
65                  70                  75                  80
Leu Ala Glu Leu Pro Ala Ile Gly Ala Thr Asn Thr Ile Ile Gly Asn
                85                  90                  95
Asn Asn Ser Asn Ser Ser Ala Gly Val Ser Ser Ala Asp Leu Arg Arg
            100                 105                 110
Leu Gly Ala Asn Arg Thr Leu Val Leu Val Asn Gly Lys Arg Tyr Val
        115                 120                 125
Ala Gly Gln Pro Gly Ser Ala Glu Val Asp Leu Ser Thr Ile Pro Thr
    130                 135                 140
Ser Met Ile Ser Arg Val Glu Ile Val Thr Gly Gly Ala Ser Ala Ile
145                 150                 155                 160
Tyr Gly Ser Asp Ala Val Ser Gly Val Ile Asn Val Ile Leu Lys Glu
                165                 170                 175
Asp Phe Glu Gly Phe Glu Phe Asn Ala Arg Thr Ser Gly Ser Thr Glu
            180                 185                 190
Ser Val Gly Thr Gln Glu His Ser Phe Asp Ile Leu Gly Gly Ala Asn
        195                 200                 205
Val Ala Asp Gly Arg Gly Asn Val Thr Phe Tyr Ala Gly Tyr Glu Arg
    210                 215                 220
Thr Lys Glu Val Met Ala Thr Asp Ile Arg Gln Phe Asp Ala Trp Gly
225                 230                 235                 240

Thr Ile Lys Asn Glu Ala Asp Gly Gly Glu Asp Asp Gly Ile Pro Asp
                245                 250                 255
Arg Leu Arg Val Pro Arg Val Tyr Ser Glu Met Ile Asn Ala Thr Gly
            260                 265                 270
Val Ile Asn Ala Phe Gly Gly Gly Ile Gly Arg Ser Thr Phe Asp Ser
        275                 280                 285
Asn Gly Asn Pro Ile Ala Gln Gln Glu Arg Asp Gly Thr Asn Ser Phe
    290                 295                 300
Ala Phe Gly Ser Phe Pro Asn Gly Cys Asp Thr Cys Phe Asn Thr Glu
305                 310                 315                 320
Ala Tyr Glu Asn Tyr Ile Pro Gly Val Glu Arg Ile Asn Val Gly Ser
                325                 330                 335
Ser Phe Asn Phe Asp Phe Thr Asp Asn Ile Gln Phe Tyr Thr Asp Phe
            340                 345                 350
Arg Tyr Val Lys Ser Asp Ile Gln Gln Gln Phe Gln Pro Ser Phe Arg
        355                 360                 365
Phe Gly Asn Ile Asn Ile Asn Val Glu Asp Asn Ala Phe Leu Asn Asp
    370                 375                 380
Asp Leu Arg Gln Gln Met Leu Asp Ala Gly Gln Thr Asn Ala Ser Phe
385                 390                 395                 400
Ala Lys Phe Phe Asp Glu Leu Gly Asn Arg Ser Ala Glu Asn Lys Arg
                405                 410                 415
Glu Leu Phe Arg Tyr Val Gly Gly Phe Lys Gly Gly Phe Asp Ile Ser
            420                 425                 430
Glu Thr Ile Phe Asp Tyr Asp Leu Tyr Tyr Val Tyr Gly Glu Thr Asn
        435                 440                 445
Asn Arg Arg Lys Thr Leu Asn Asp Leu Ile Pro Asp Asn Phe Val Ala
    450                 455                 460
Ala Val Asp Ser Val Ile Asp Pro Asp Thr Gly Leu Ala Ala Cys Arg
465                 470                 475                 480
Ser Gln Val Ala Ser Ala Gln Gly Asp Asp Tyr Thr Asp Pro Ala Ser
                485                 490                 495
Val Asn Gly Ser Asp Cys Val Ala Tyr Asn Pro Phe Gly Met Gly Gln
            500                 505                 510
Ala Ser Ala Glu Ala Arg Asp Trp Val Ser Ala Asp Val Thr Arg Glu
        515                 520                 525

Asp Lys Ile Thr Gln Gln Val Ile Gly Gly Thr Leu Gly Thr Asp Ser
    530                 535                 540
Glu Glu Leu Phe Glu Leu Gln Gly Gly Ala Ile Ala Met Val Val Gly
545                 550                 555                 560
Phe Glu Tyr Arg Glu Glu Thr Ser Gly Ser Thr Thr Asp Glu Phe Thr
                565                 570                 575
Lys Ala Gly Phe Leu Thr Ser Ala Ala Thr Pro Asp Ser Tyr Gly Glu
            580                 585                 590
Tyr Asp Val Thr Glu Tyr Phe Val Glu Val Asn Ile Pro Val Leu Lys
        595                 600                 605
Glu Leu Pro Phe Ala His Glu Leu Ser Phe Asp Gly Ala Tyr Arg Asn
    610                 615                 620
Ala Asp Tyr Ser His Ala Gly Lys Thr Glu Ala Trp Lys Ala Gly Met
625                 630                 635                 640
Phe Tyr Ser Pro Leu Glu Gln Leu Ala Leu Arg Gly Thr Val Gly Glu
                645                 650                 655
Ala Val Arg Ala Pro Asn Ile Ala Glu Ala Phe Ser Pro Arg Ser Pro
            660                 665                 670
Gly Phe Gly Arg Val Ser Asp Pro Cys Asp Ala Asp Asn Ile Asn Asp
        675                 680                 685
Asp Pro Asp Arg Val Ser Asn Cys Ala Ala Leu Gly Ile Pro Pro Gly
    690                 695                 700
Phe Gln Ala Asn Asp Asn Val Ser Val Asp Thr Leu Ser Gly Gly Asn
705                 710                 715                 720
Pro Asp Leu Lys Pro Glu Thr Ser Thr Ser Phe Thr Gly Gly Leu Val
                725                 730                 735
Trp Thr Pro Thr Phe Ala Asp Asn Leu Ser Phe Thr Val Asp Tyr Tyr
            740                 745                 750
Asp Ile Gln Ile Glu Asp Ala Ile Leu Ser Val Ala Thr Gln Thr Val
        755                 760                 765
Ala Asp Asn Cys Val Asp Ser Thr Gly Gly Pro Asp Thr Asp Phe Cys
    770                 775                 780
Ser Gln Val Asp Arg Asn Pro Thr Thr Tyr Asp Ile Glu Leu Val Arg
785                 790                 795                 800
Ser Gly Tyr Leu Asn Ala Ala Ala Leu Asn Thr Lys Gly Ile Glu Phe
                805                 810                 815

Gln Ala Ala Tyr Ser Leu Asp Leu Glu Ser Phe Asn Ala Pro Gly Glu
            820                 825                 830
Leu Arg Phe Asn Leu Leu Gly Asn Gln Leu Leu Glu Leu Glu Arg Leu
        835                 840                 845
Glu Phe Gln Asn Arg Pro Asp Glu Ile Asn Asp Glu Lys Gly Glu Val
    850                 855                 860
Gly Asp Pro Glu Leu Gln Phe Arg Leu Gly Ile Asp Tyr Arg Leu Asp
865                 870                 875                 880
Asp Leu Ser Val Ser Trp Asn Thr Arg Tyr Ile Asp Ser Val Val Thr
                885                 890                 895
Tyr Asp Val Ser Glu Asn Gly Gly Ser Pro Glu Asp Leu Tyr Pro Gly
            900                 905                 910
His Ile Gly Ser Met Thr Thr His Asp Leu Ser Ala Thr Tyr Tyr Ile
        915                 920                 925
Asn Glu Asn Phe Met Ile Asn Gly Gly Val Arg Asn Leu Phe Asp Ala
    930                 935                 940
Leu Pro Pro Gly Tyr Thr Asn Asp Ala Leu Tyr Asp Leu Val Gly Arg
945                 950                 955                 960
Arg Ala Phe Leu Gly Ile Lys Val Met Met
                965                 970

SEQ ID NO: 8
Sequence Length: 864
Sequence Type: Nucleic acid
Strandedness: Double strand
Topology: Linear
Molecular Type: Genomic DNA
Source: Shewanella putrefaciens SCRC-2874 (FERM BP-1625)
Sequence
ATG GCA AAA ATA AAT AGT GAA CAC TTG GAT GAA GCT ACT ATT ACT TCG 48
Met Ala Lys Ile Asn Ser Glu His Leu Asp Glu Ala Thr Ile Thr Ser
1               5                   10                  15
AAT AAG TGT ACG CAA ACA GAG ACT GAG GCT CGG CAT AGA AAT GCC ACT 96
Asn Lys Cys Thr Gln Thr Glu Thr Glu Ala Arg His Arg Asn Ala Thr
            20                  25                  30

ACA ACA CCT GAG ATG CGC CGA TTC ATA CAA GAG TCG GAT CTC AGT GTT 144
Thr Thr Pro Glu Met Arg Arg Phe Ile Gln Glu Ser Asp Leu Ser Val
        35                  40                  45
AGC CAA CTG TCT AAA ATA TTA AAT ATC AGT GAA GCT ACC GTA CGT AAG 192
Ser Gln Leu Ser Lys Ile Leu Asn Ile Ser Glu Ala Thr Val Arg Lys
    50                  55                  60
TGG CGC AAG CGT GAC TCT GTC GAA AAC TGT CCT AAT ACC CCG CAC CAT 240
Trp Arg Lys Arg Asp Ser Val Glu Asn Cys Pro Asn Thr Pro His His
65                  70                  75                  80
CTC AAT ACC ACG CTA ACC CCT TTG CAA GAA TAT GTG GTT GTG GGC CTG 288
Leu Asn Thr Thr Leu Thr Pro Leu Gln Glu Tyr Val Val Val Gly Leu
                85                  90                  95
CGT TAT CAA TTG AAA ATG CCA TTA GAC AGA TTG CTC AAA GCA ACC CAA 336
Arg Tyr Gln Leu Lys Met Pro Leu Asp Arg Leu Leu Lys Ala Thr Gln
            100                 105                 110
GAG TTT ATC AAT CCA AAC GTG TCG CGC TCA GGT TTA GCA AGA TGT TTG 384
Glu Phe Ile Asn Pro Asn Val Ser Arg Ser Gly Leu Ala Arg Cys Leu
        115                 120                 125
AAG CGT TAT GGC GTT TCA CGG GTG AGT GAT ATC CAA AGC CCA CAC GTA 432
Lys Arg Tyr Gly Val Ser Arg Val Ser Asp Ile Gln Ser Pro His Val
    130                 135                 140
CCA ATG CGC TAC TTT AAT CAA ATT CCA GTC ACT CAA GGC AGC GAT GTG 480
Pro Met Arg Tyr Phe Asn Gln Ile Pro Val Thr Gln Gly Ser Asp Val
145                 150                 155                 160
CAA ACC TAC ACC CTG CAC TAT GAA ACG CTG GCA AAA ACC TTA GCC TTA 528
Gln Thr Tyr Thr Leu His Tyr Glu Thr Leu Ala Lys Thr Leu Ala Leu
                165                 170                 175
CCT AGT ACC GAT GGT GAC AAT GTG GTG CAA GTG GTG TCT CTC ACC ATT 576
Pro Ser Thr Asp Gly Asp Asn Val Val Gln Val Val Ser Leu Thr Ile
            180                 185                 190
CCA CCA AAG TTA ACC GAA GAA GCA CCC AGT TCA ATT TTG CTC GGC ATT 624
Pro Pro Lys Leu Thr Glu Glu Ala Pro Ser Ser Ile Leu Leu Gly Ile
        195                 200                 205
GAT CCT CAT AGC GAC TGG ATC TAT CTC GAC ATA TAC CAA GAT GGC AAT 672
Asp Pro His Ser Asp Trp Ile Tyr Leu Asp Ile Tyr Gln Asp Gly Asn
    210                 215                 220

ACA CAA GCC ACG AAT AGA TAT ATG GCT TAT GTG CTA AAA CAC GGG CCA 720
Thr Gln Ala Thr Asn Arg Tyr Met Ala Tyr Val Leu Lys His Gly Pro
225                 230                 235                 240
TTC CAT TTA CGA AAG TTA CTC GTG CGT AAC TAT CAC ACC TTT TTA CAG 768
Phe His Leu Arg Lys Leu Leu Val Arg Asn Tyr His Thr Phe Leu Gln
                245                 250                 255
CGC TTT CCT GGA GCG ACG CAA AAT CGC CGC CCC TCT AAA GAT ATG CCT 816
Arg Phe Pro Gly Ala Thr Gln Asn Arg Arg Pro Ser Lys Asp Met Pro
            260                 265                 270
GAA ACA ATC AAC AAG ACG CCT GAA ACA CAG GCA CCC AGT GGA GAC TCA 864
Glu Thr Ile Asn Lys Thr Pro Glu Thr Gln Ala Pro Ser Gly Asp Ser
        275                 280                 285

SEQ ID NO: 9
Sequence Length: 288
Sequence Type: Amino acid
Molecular Type: Peptide
Sequence
Met Ala Lys Ile Asn Ser Glu His Leu Asp Glu Ala Thr Ile Thr Ser
1               5                   10                  15
Asn Lys Cys Thr Gln Thr Glu Thr Glu Ala Arg His Arg Asn Ala Thr
            20                  25                  30
Thr Thr Pro Glu Met Arg Arg Phe Ile Gln Glu Ser Asp Leu Ser Val
        35                  40                  45
Ser Gln Leu Ser Lys Ile Leu Asn Ile Ser Glu Ala Thr Val Arg Lys
    50                  55                  60
Trp Arg Lys Arg Asp Ser Val Glu Asn Cys Pro Asn Thr Pro His His
65                  70                  75                  80
Leu Asn Thr Thr Leu Thr Pro Leu Gln Glu Tyr Val Val Val Gly Leu
                85                  90                  95
Arg Tyr Gln Leu Lys Met Pro Leu Asp Arg Leu Leu Lys Ala Thr Gln
            100                 105                 110
Glu Phe Ile Asn Pro Asn Val Ser Arg Ser Gly Leu Ala Arg Cys Leu
        115                 120                 125
Lys Arg Tyr Gly Val Ser Arg Val Ser Asp Ile Gln Ser Pro His Val
    130                 135                 140

Pro Met Arg Tyr Phe Asn Gln Ile Pro Val Thr Gln Gly Ser Asp Val
145                 150                 155                 160
Gln Thr Tyr Thr Leu His Tyr Glu Thr Leu Ala Lys Thr Leu Ala Leu
                165                 170                 175
Pro Ser Thr Asp Gly Asp Asn Val Val Gln Val Val Ser Leu Thr Ile
            180                 185                 190
Pro Pro Lys Leu Thr Glu Glu Ala Pro Ser Ser Ile Leu Leu Gly Ile
        195                 200                 205
Asp Pro His Ser Asp Trp Ile Tyr Leu Asp Ile Tyr Gln Asp Gly Asn
    210                 215                 220
Thr Gln Ala Thr Asn Arg Tyr Met Ala Tyr Val Leu Lys His Gly Pro
225                 230                 235                 240
Phe His Leu Arg Lys Leu Leu Val Arg Asn Tyr His Thr Phe Leu Gln
                245                 250                 255
Arg Phe Pro Gly Ala Thr Gln Asn Arg Arg Pro Ser Lys Asp Met Pro
            260                 265                 270
Glu Thr Ile Asn Lys Thr Pro Glu Thr Gln Ala Pro Ser Gly Asp Ser
        275                 280                 285

SEQ ID NO: 10
Sequence Length: 8268
Sequence Type: Nucleic acid
Strandedness: Double strand
Topology: Linear
Molecular Type: Genomic DNA
Source: Shewanella putrefaciens SCRC-2874 (FERM BP-1625)
Sequence
ATG AGC CAG ACC TCT AAA CCT ACA AAC TCA GCA ACT GAG CAA GCA CAA      48
Met Ser Gln Thr Ser Lys Pro Thr Asn Ser Ala Thr Glu Gln Ala Gln
  1               5                  10                  15
GAC TCA CAA GCT GAC TCT CGT TTA AAT AAA CGA CTA AAA GAT ATG CCA      96
Asp Ser Gln Ala Asp Ser Arg Leu Asn Lys Arg Leu Lys Asp Met Pro
             20                  25                  30
ATT GCT ATT GTT GGC ATG GCG AGT ATT TTT GCA AAC TCT CGC TAT TTG     144
Ile Ala Ile Val Gly Met Ala Ser Ile Phe Ala Asn Ser Arg Tyr Leu
         35                  40                  45

AAT AAG TTT TGG GAC TTA ATC AGC GAA AAA ATT GAT GCG ATT ACT GAA 192
Asn Lys Phe Trp Asp Leu Ile Ser Glu Lys Ile Asp Ala Ile Thr Glu
    50                  55                  60
TTA CCA TCA ACT CAC TGG CAG CCT GAA GAA TAT TAC GAC GCA GAT AAA 240
Leu Pro Ser Thr His Trp Gln Pro Glu Glu Tyr Tyr Asp Ala Asp Lys
65                  70                  75                  80
ACC GCA GCA GAC AAA AGC TAC TGT AAA CGT GGT GGC TTT TTG CCA GAT 288
Thr Ala Ala Asp Lys Ser Tyr Cys Lys Arg Gly Gly Phe Leu Pro Asp
                85                  90                  95
GTA GAC TTC AAC CCA ATG GAG TTT GGC CTG CCG CCA AAC ATT TTG GAA 336
Val Asp Phe Asn Pro Met Glu Phe Gly Leu Pro Pro Asn Ile Leu Glu
            100                 105                 110
CTG ACC GAT TCA TCG CAA CTA TTA TCA CTC ATC GTT GCT AAA GAA GTG 384
Leu Thr Asp Ser Ser Gln Leu Leu Ser Leu Ile Val Ala Lys Glu Val
        115                 120                 125
TTG GCT GAT GCT AAC TTA CCT GAG AAT TAC GAC CGC GAT AAA ATT GGT 432
Leu Ala Asp Ala Asn Leu Pro Glu Asn Tyr Asp Arg Asp Lys Ile Gly
    130                 135                 140
ATC ACC TTA GGT GTC GGC GGT GGT CAA AAA ATT AGC CAC AGC CTA ACA 480
Ile Thr Leu Gly Val Gly Gly Gly Gln Lys Ile Ser His Ser Leu Thr
145                 150                 155                 160
GCG CGT CTG CAA TAC CCA GTA TTG AAG AAA GTA TTC GCC AAT AGC GGC 528
Ala Arg Leu Gln Tyr Pro Val Leu Lys Lys Val Phe Ala Asn Ser Gly
                165                 170                 175
ATT AGT GAC ACC GAC AGC GAA ATG CTT ATC AAG AAA TTC CAA GAC CAA 576
Ile Ser Asp Thr Asp Ser Glu Met Leu Ile Lys Lys Phe Gln Asp Gln
            180                 185                 190
TAT GTA CAC TGG GAA GAA AAC TCG TTC CCA GGT TCA CTT GGT AAC GTT 624
Tyr Val His Trp Glu Glu Asn Ser Phe Pro Gly Ser Leu Gly Asn Val
        195                 200                 205
ATT GCG GGC CGT ATC GCC AAC CGC TTC GAT TTT GGC GGC ATG AAC TGT 672
Ile Ala Gly Arg Ile Ala Asn Arg Phe Asp Phe Gly Gly Met Asn Cys
    210                 215                 220
GTG GTT GAT GCT GCC TGT GCT GGA TCA CTT GCT GCT ATG CGT ATG GCG 720
Val Val Asp Ala Ala Cys Ala Gly Ser Leu Ala Ala Met Arg Met Ala
225                 230                 235                 240

CTA ACA GAG CTA ACT GAA GGT CGC TCT GAA ATG ATG ATC ACC GGT GGT 768
Leu Thr Glu Leu Thr Glu Gly Arg Ser Glu Met Met Ile Thr Gly Gly
                245                 250                 255
GTG TGT ACT GAT AAC TCA CCC TCT ATG TAT ATG AGC TTT TCA AAA ACG 816
Val Cys Thr Asp Asn Ser Pro Ser Met Tyr Met Ser Phe Ser Lys Thr
            260                 265                 270
CCC GCC TTT ACC ACT AAC GAA ACC ATT CAG CCA TTT GAT ATC GAC TCA 864
Pro Ala Phe Thr Thr Asn Glu Thr Ile Gln Pro Phe Asp Ile Asp Ser
        275                 280                 285
AAA GGC ATG ATG ATT GGT GAA GGT ATT GGC ATG GTG GCG CTA AAG CGT 912
Lys Gly Met Met Ile Gly Glu Gly Ile Gly Met Val Ala Leu Lys Arg
    290                 295                 300
CTT GAA GAT GCA GAG CGC GAT GGC GAC CGC ATT TAC TCT GTA ATT AAA 960
Leu Glu Asp Ala Glu Arg Asp Gly Asp Arg Ile Tyr Ser Val Ile Lys
305                 310                 315                 320
GGT GTG GGT GCA TCA TCT GAC GGT AAG TTT AAA TCA ATC TAT GCC CCT 1008
Gly Val Gly Ala Ser Ser Asp Gly Lys Phe Lys Ser Ile Tyr Ala Pro
                325                 330                 335
CGC CCA TCA GGC CAA GCT AAA GCA CTT AAC CGT GCC TAT GAT GAC GCA 1056
Arg Pro Ser Gly Gln Ala Lys Ala Leu Asn Arg Ala Tyr Asp Asp Ala
            340                 345                 350
GGT TTT GCG CCG CAT ACC TTA GGT CTA ATT GAA GCT CAC GGA ACA GGT 1104
Gly Phe Ala Pro His Thr Leu Gly Leu Ile Glu Ala His Gly Thr Gly
        355                 360                 365
ACT GCA GCA GGT GAC GCG GCA GAG TTT GCC GGC CTT TGC TCA GTA TTT 1152
Thr Ala Ala Gly Asp Ala Ala Glu Phe Ala Gly Leu Cys Ser Val Phe
    370                 375                 380
GCT GAA GGC AAC GAT ACC AAG CAA CAC ATT GCG CTA GGT TCA GTT AAA 1200
Ala Glu Gly Asn Asp Thr Lys Gln His Ile Ala Leu Gly Ser Val Lys
385                 390                 395                 400
TCA CAA ATT GGT CAT ACT AAA TCA ACT GCA GGT ACA GCA GGT TTA ATT 1248
Ser Gln Ile Gly His Thr Lys Ser Thr Ala Gly Thr Ala Gly Leu Ile
                405                 410                 415
AAA GCT GCT CTT GCT TTG CAT CAC AAG GTA CTG CCG CCG ACC ATT AAC 1296
Lys Ala Ala Leu Ala Leu His His Lys Val Leu Pro Pro Thr Ile Asn
            420                 425                 430

GTT AGT CAG CCA AGC CCT AAA CTT GAT ATC GAA AAC TCA CCG TTT TAT 1344
Val Ser Gln Pro Ser Pro Lys Leu Asp Ile Glu Asn Ser Pro Phe Tyr
        435                 440                 445
CTA AAC ACT GAG ACT CGT CCA TGG TTA CCA CGT GTT GAT GGT ACG CCG 1392
Leu Asn Thr Glu Thr Arg Pro Trp Leu Pro Arg Val Asp Gly Thr Pro
    450                 455                 460
CGC CGC GCG GGT ATT AGC TCA TTT GGT TTT GGT GGC ACT AAC TTC CAT 1440
Arg Arg Ala Gly Ile Ser Ser Phe Gly Phe Gly Gly Thr Asn Phe His
465                 470                 475                 480
TTT GTA CTA GAA GAG TAC AAC CAA GAA CAC AGC CGT ACT GAT AGC GAA 1488
Phe Val Leu Glu Glu Tyr Asn Gln Glu His Ser Arg Thr Asp Ser Glu
                485                 490                 495
AAA GCT AAG TAT CGT CAA CGC CAA GTG GCG CAA AGC TTC CTT GTT AGC 1536
Lys Ala Lys Tyr Arg Gln Arg Gln Val Ala Gln Ser Phe Leu Val Ser
            500                 505                 510
GCA AGC GAT AAA GCA TCG CTA ATT AAC GAG TTA AAC GTA CTA GCA GCA 1584
Ala Ser Asp Lys Ala Ser Leu Ile Asn Glu Leu Asn Val Leu Ala Ala
        515                 520                 525
TCT GCA AGC CAA GCT GAG TTT ATC CTC AAA GAT GCA GCA GCA AAC TAT 1632
Ser Ala Ser Gln Ala Glu Phe Ile Leu Lys Asp Ala Ala Ala Asn Tyr
    530                 535                 540
GGC GTA CGT GAG CTT GAT AAA AAT GCA CCA CGG ATC GGT TTA GTT GCA 1680
Gly Val Arg Glu Leu Asp Lys Asn Ala Pro Arg Ile Gly Leu Val Ala
545                 550                 555                 560
AAC ACA GCT GAA GAG TTA GCA GGC CTA ATT AAG CAA GCA CTT GCC AAA 1728
Asn Thr Ala Glu Glu Leu Ala Gly Leu Ile Lys Gln Ala Leu Ala Lys
                565                 570                 575
CTA GCA GCT AGC GAT GAT AAC GCA TGG CAG CTA CCT GGT GGC ACT AGC 1776
Leu Ala Ala Ser Asp Asp Asn Ala Trp Gln Leu Pro Gly Gly Thr Ser
            580                 585                 590
TAC CGC GCC GCT GCA GTA GAA GGT AAA GTT GCC GCA CTG TTT GCT GGC 1824
Tyr Arg Ala Ala Ala Val Glu Gly Lys Val Ala Ala Leu Phe Ala Gly
        595                 600                 605
CAA GGT TCA CAA TAT CTC AAT ATG GGC CGT GAC CTT ACT TGT TAT TAC 1872
Gln Gly Ser Gln Tyr Leu Asn Met Gly Arg Asp Leu Thr Cys Tyr Tyr
    610                 615                 620

CCA GAG ATG CGT CAG CAA TTT GTA ACT GCA GAT AAA GTA TTT GCC GCA 1920
Pro Glu Met Arg Gln Gln Phe Val Thr Ala Asp Lys Val Phe Ala Ala
625                 630                 635                 640
AAT GAT AAA ACG CCG TTA TCG CAA ACT CTG TAT CCA AAG CCT GTA TTT 1968
Asn Asp Lys Thr Pro Leu Ser Gln Thr Leu Tyr Pro Lys Pro Val Phe
                645                 650                 655
AAT AAA GAT GAA TTA AAG GCT CAA GAA GCC ATT TTG ACC AAT ACC GCC 2016
Asn Lys Asp Glu Leu Lys Ala Gln Glu Ala Ile Leu Thr Asn Thr Ala
            660                 665                 670
AAT GCC CAA AGC GCA ATT GGT GCG ATT TCA ATG GGT CAA TAC GAT TTG 2064
Asn Ala Gln Ser Ala Ile Gly Ala Ile Ser Met Gly Gln Tyr Asp Leu
        675                 680                 685
TTT ACT GCG GCT GGC TTT AAT GCC GAC ATG GTT GCA GGC CAT AGC TTT 2112
Phe Thr Ala Ala Gly Phe Asn Ala Asp Met Val Ala Gly His Ser Phe
    690                 695                 700
GGT GAG CTA AGT GCA CTG TGT GCT GCA GGT GTT ATT TCA GCT GAT GAC 2160
Gly Glu Leu Ser Ala Leu Cys Ala Ala Gly Val Ile Ser Ala Asp Asp
705                 710                 715                 720
TAC TAC AAG CTG GCT TTT GCT CGT GGT GAG GCT ATG GCA ACA AAA GCA 2208
Tyr Tyr Lys Leu Ala Phe Ala Arg Gly Glu Ala Met Ala Thr Lys Ala
                725                 730                 735
CCG GCT AAA GAC GGC GTT GAA GCA GAT GCA GGA GCA ATG TTT GCA ATC 2256
Pro Ala Lys Asp Gly Val Glu Ala Asp Ala Gly Ala Met Phe Ala Ile
            740                 745                 750
ATA ACC AAG AGT GCT GCA GAC CTT GAA ACC GTT GAA GCC ACC ATC GCT 2304
Ile Thr Lys Ser Ala Ala Asp Leu Glu Thr Val Glu Ala Thr Ile Ala
        755                 760                 765
AAA TTT GAT GGG GTG AAA GTC GCT AAC TAT AAC GCG CCA ACG CAA TCA 2352
Lys Phe Asp Gly Val Lys Val Ala Asn Tyr Asn Ala Pro Thr Gln Ser
    770                 775                 780
GTA ATT GCA GGC CCA ACA GCA ACT ACC GCT GAT GCG GCT AAA GCG CTA 2400
Val Ile Ala Gly Pro Thr Ala Thr Thr Ala Asp Ala Ala Lys Ala Leu
785                 790                 795                 800
ACT GAG CTT GGT TAC AAA GCG ATT AAC CTG CCA GTA TCA GGT GCA TTC 2448
Thr Glu Leu Gly Tyr Lys Ala Ile Asn Leu Pro Val Ser Gly Ala Phe
                805                 810                 815

CAC ACT GAA CTT GTT GGT CAC GCT CAA GCG CCA TTT GCT AAA GCG ATT 2496
His Thr Glu Leu Val Gly His Ala Gln Ala Pro Phe Ala Lys Ala Ile
            820                 825                 830
GAC GCA GCC AAA TTT ACT AAA ACA AGC CGA GCA CTT TAC TCA AAT GCA 2544
Asp Ala Ala Lys Phe Thr Lys Thr Ser Arg Ala Leu Tyr Ser Asn Ala
        835                 840                 845
ACT GGC GGA CTT TAT GAA AGC ACT GCT GCA AAG ATT AAA GCC TCG TTT 2592
Thr Gly Gly Leu Tyr Glu Ser Thr Ala Ala Lys Ile Lys Ala Ser Phe
    850                 855                 860
AAG AAA CAT ATG CTT CAA TCA GTG CGC TTT ACT AGC CAG CTA GAA GCC 2640
Lys Lys His Met Leu Gln Ser Val Arg Phe Thr Ser Gln Leu Glu Ala
865                 870                 875                 880
ATG TAC AAC GAC GGC GCC CGT GTA TTT GTT GAA TTT GGT CCA AAG AAC 2688
Met Tyr Asn Asp Gly Ala Arg Val Phe Val Glu Phe Gly Pro Lys Asn
                885                 890                 895
ATC TTA CAA AAA TTA GTT CAA GGC ACG CTT GTC AAC ACT GAA AAT GAA 2736
Ile Leu Gln Lys Leu Val Gln Gly Thr Leu Val Asn Thr Glu Asn Glu
            900                 905                 910
GTT TGC ACT ATC TCT ATC AAC CCT AAT CCT AAA GTT GAT AGT GAT CTG 2784
Val Cys Thr Ile Ser Ile Asn Pro Asn Pro Lys Val Asp Ser Asp Leu
        915                 920                 925
CAG CTT AAG CAA GCA GCA ATG CAG CTA GCG GTT ACT GGT GTG GTA CTC 2832
Gln Leu Lys Gln Ala Ala Met Gln Leu Ala Val Thr Gly Val Val Leu
    930                 935                 940
AGT GAA ATT GAC CCA TAC CAA GCC GAT ATT GCC GCA CCA GCG AAA AAG 2880
Ser Glu Ile Asp Pro Tyr Gln Ala Asp Ile Ala Ala Pro Ala Lys Lys
945                 950                 955                 960
TCG CCA ATG AGC ATT TCG CTT AAT GCT GCT AAC CAT ATC AGC AAA GCA 2928
Ser Pro Met Ser Ile Ser Leu Asn Ala Ala Asn His Ile Ser Lys Ala
                965                 970                 975
ACT CGC GCT AAG ATG GCC AAG TCT TTA GAG ACA GGT ATC GTC ACC TCG 2976
Thr Arg Ala Lys Met Ala Lys Ser Leu Glu Thr Gly Ile Val Thr Ser
            980                 985                 990
CAA ATA GAA CAT GTT ATT GAA GAA AAA ATC GTT GAA GTT GAG AAA CTG 3024
Gln Ile Glu His Val Ile Glu Glu Lys Ile Val Glu Val Glu Lys Leu
        995                 1000                1005

GTT GAA GTC GAA AAG ATC GTC GAA AAA GTG GTT GAA GTA GAG AAA GTT 3072
Val Glu Val Glu Lys Ile Val Glu Lys Val Val Glu Val Glu Lys Val
    1010                1015                1020
GTT GAG GTT GAA GCT CCT GTT AAT TCA GTG CAA GCC AAT GCA ATT CAA 3120
Val Glu Val Glu Ala Pro Val Asn Ser Val Gln Ala Asn Ala Ile Gln
1025                1030                1035                1040
ACC CGT TCA GTT GTC GCT CCA GTA ATA GAG AAC CAA GTC GTG TCT AAA 3168
Thr Arg Ser Val Val Ala Pro Val Ile Glu Asn Gln Val Val Ser Lys
                1045                1050                1055
AAC AGT AAG CCA GCA GTC CAG AGC ATT AGT GGT GAT GCA CTC AGC AAC 3216
Asn Ser Lys Pro Ala Val Gln Ser Ile Ser Gly Asp Ala Leu Ser Asn
            1060                1065                1070
TTT TTT GCT GCA CAG CAG CAA ACC GCA CAG TTG CAT CAG CAG TTC TTA 3264
Phe Phe Ala Ala Gln Gln Gln Thr Ala Gln Leu His Gln Gln Phe Leu
        1075                1080                1085
GCT ATT CCG CAG CAA TAT GGT GAG ACG TTC ACT ACG CTG ATG ACC GAG 3312
Ala Ile Pro Gln Gln Tyr Gly Glu Thr Phe Thr Thr Leu Met Thr Glu
    1090                1095                1100
CAA GCT AAA CTG GCA AGT TCT GGT GTT GCA ATT CCA GAG AGT CTG CAA 3360
Gln Ala Lys Leu Ala Ser Ser Gly Val Ala Ile Pro Glu Ser Leu Gln
1105                1110                1115                1120
CGC TCA ATG GAG CAA TTC CAC CAA CTA CAA GCG CAA ACA CTA CAA AGC 3408
Arg Ser Met Glu Gln Phe His Gln Leu Gln Ala Gln Thr Leu Gln Ser
                1125                1130                1135
CAC ACC CAG TTC CTT GAG ATG CAA GCG GGT AGC AAC ATT GCA GCG TTA 3456
His Thr Gln Phe Leu Glu Met Gln Ala Gly Ser Asn Ile Ala Ala Leu
            1140                1145                1150
AAC CTA CTC AAT AGC AGC CAA GCA ACT TAC GCT CCA GCC ATT CAC AAT 3504
Asn Leu Leu Asn Ser Ser Gln Ala Thr Tyr Ala Pro Ala Ile His Asn
        1155                1160                1165
GAA GCG ATT CAA AGC CAA GTG GTT CAA AGC CAA ACT GCA GTC CAG CCA 3552
Glu Ala Ile Gln Ser Gln Val Val Gln Ser Gln Thr Ala Val Gln Pro
    1170                1175                1180
GTA ATT TCA ACA CAA GTT AAC CAT GTG TCA GAG CAG CCA ACT CAA GCT 3600
Val Ile Ser Thr Gln Val Asn His Val Ser Glu Gln Pro Thr Gln Ala
1185                1190                1195                1200

CCA GCT CCA AAA GCG CAG CCA GCA CCT GTG ACA ACT GCA GTT CAA ACT 3648
Pro Ala Pro Lys Ala Gln Pro Ala Pro Val Thr Thr Ala Val Gln Thr
                1205                1210                1215
GCT CCG GCA CAA GTT GTT CGT CAA GCC GCA CCA GTT CAA GCC GCT ATT 3696
Ala Pro Ala Gln Val Val Arg Gln Ala Ala Pro Val Gln Ala Ala Ile
            1220                1225                1230
GAA CCG ATT AAT ACA AGT GTT GCG ACT ACA ACG CCT TCA GCC TTC AGC 3744
Glu Pro Ile Asn Thr Ser Val Ala Thr Thr Thr Pro Ser Ala Phe Ser
        1235                1240                1245
GCC GAA ACA GCC CTG AGC GCA ACA AAA GTC CAA GCC ACT ATG CTT GAA 3792
Ala Glu Thr Ala Leu Ser Ala Thr Lys Val Gln Ala Thr Met Leu Glu
    1250                1255                1260
GTG GTT GCT GAG AAA ACC GGT TAC CCA ACT GAA ATG CTA GAG CTT GAA 3840
Val Val Ala Glu Lys Thr Gly Tyr Pro Thr Glu Met Leu Glu Leu Glu
1265                1270                1275                1280
ATG GAT ATG GAA GCC GAT TTA GGC ATC GAT TCT ATC AAG CGT GTA GAA 3888
Met Asp Met Glu Ala Asp Leu Gly Ile Asp Ser Ile Lys Arg Val Glu
                1285                1290                1295
ATT CTT GGC ACA GTA CAA GAT GAG CTA CCG GGT CTA CCT GAG CTT AGC 3936
Ile Leu Gly Thr Val Gln Asp Glu Leu Pro Gly Leu Pro Glu Leu Ser
            1300                1305                1310
CCT GAA GAT CTA GCT GAG TGT CGA ACG CTA GGC GAA ATC GTT GAC TAT 3984
Pro Glu Asp Leu Ala Glu Cys Arg Thr Leu Gly Glu Ile Val Asp Tyr
        1315                1320                1325
ATG GGC AGT AAA CTG CCG GCT GAA GGC TCT ATG AAT TCT CAG CTG TCT 4032
Met Gly Ser Lys Leu Pro Ala Glu Gly Ser Met Asn Ser Gln Leu Ser
    1330                1335                1340
ACA GGT TCC GCA GCT GCG ACT CCT GCA GCG AAT GGT CTT TCT GCG GAG 4080
Thr Gly Ser Ala Ala Ala Thr Pro Ala Ala Asn Gly Leu Ser Ala Glu
1345                1350                1355                1360
AAA GTT CAA GCG ACT ATG ATG TCT GTG GTT GCC GAA AAG ACT GGC TAC 4128
Lys Val Gln Ala Thr Met Met Ser Val Val Ala Glu Lys Thr Gly Tyr
                1365                1370                1375
CCA ACT GAA ATG CTA GAG CTT GAA ATG GAT ATG GAA GCC GAT TTA GGC 4176
Pro Thr Glu Met Leu Glu Leu Glu Met Asp Met Glu Ala Asp Leu Gly
            1380                1385                1390

ATA GAT TCT ATC AAG CGC GTT GAA ATT CTT GGC ACA GTA CAA GAT GAG 4224
Ile Asp Ser Ile Lys Arg Val Glu Ile Leu Gly Thr Val Gln Asp Glu
        1395                1400                1405
CTA CCG GGT CTA CCT GAG CTT AGC CCT GAA GAT CTA GCT GAG TGT CGT 4272
Leu Pro Gly Leu Pro Glu Leu Ser Pro Glu Asp Leu Ala Glu Cys Arg
    1410                1415                1420
ACT CTA GGC GAA ATC GTT GAC TAT ATG AAC TCT AAA CTC GCT GAC GGC 4320
Thr Leu Gly Glu Ile Val Asp Tyr Met Asn Ser Lys Leu Ala Asp Gly
1425                1430                1435                1440
TCT AAG CTG CCG GCT GAA GGC TCT ATG AAT TCT CAG CTG TCT ACA AGT 4368
Ser Lys Leu Pro Ala Glu Gly Ser Met Asn Ser Gln Leu Ser Thr Ser
                1445                1450                1455
GCC GCA GCT GCG ACT CCT GCA GCG AAT GGT CTC TCT GCG GAG AAA GTT 4416
Ala Ala Ala Ala Thr Pro Ala Ala Asn Gly Leu Ser Ala Glu Lys Val
            1460                1465                1470
CAA GCG ACT ATG ATG TCT GTG GTT GCC GAA AAG ACT GGC TAC CCA ACT 4464
Gln Ala Thr Met Met Ser Val Val Ala Glu Lys Thr Gly Tyr Pro Thr
        1475                1480                1485
GAA ATG CTA GAA CTT GAA ATG GAT ATG GAA GCT GAC CTT GGC ATC GAT 4512
Glu Met Leu Glu Leu Glu Met Asp Met Glu Ala Asp Leu Gly Ile Asp
    1490                1495                1500
TCA ATC AAG CGC GTT GAA ATT CTT GGC ACA GTA CAA GAT GAG CTA CCG 4560
Ser Ile Lys Arg Val Glu Ile Leu Gly Thr Val Gln Asp Glu Leu Pro
1505                1510                1515                1520
GGT TTA CCT GAG CTA AAT CCA GAA GAT TTG GCA GAG TGT CGT ACT CTT 4608
Gly Leu Pro Glu Leu Asn Pro Glu Asp Leu Ala Glu Cys Arg Thr Leu
                1525                1530                1535
GGC GAA ATC GTG ACT TAT ATG AAC TCT AAA CTC GCT GAC GGC TCT AAG 4656
Gly Glu Ile Val Thr Tyr Met Asn Ser Lys Leu Ala Asp Gly Ser Lys
            1540                1545                1550
CTG CCA GCT GAA GGC TCT ATG CAC TAT CAG CTG TCT ACA AGT ACC GCT 4704
Leu Pro Ala Glu Gly Ser Met His Tyr Gln Leu Ser Thr Ser Thr Ala
        1555                1560                1565
GCT GCG ACT CCT GTA GCG AAT GGT CTC TCT GCA GAA AAA GTT CAA GCG 4752
Ala Ala Thr Pro Val Ala Asn Gly Leu Ser Ala Glu Lys Val Gln Ala
    1570                1575                1580

ACC ATG ATG TCT GTA GTT GCA GAT AAA ACT GGC TAC CCA ACT GAA ATG 4800
Thr Met Met Ser Val Val Ala Asp Lys Thr Gly Tyr Pro Thr Glu Met
1585                1590                1595                1600
CTT GAA CTT GAA ATG GAT ATG GAA GCC GAT TTA GGT ATC GAT TCT ATC 4848
Leu Glu Leu Glu Met Asp Met Glu Ala Asp Leu Gly Ile Asp Ser Ile
                1605                1610                1615
AAG CGC GTT GAA ATT CTT GGC ACA GTA CAA GAT GAG CTA CCG GGT TTA 4896
Lys Arg Val Glu Ile Leu Gly Thr Val Gln Asp Glu Leu Pro Gly Leu
            1620                1625                1630
CCT GAG CTA AAT CCA GAA GAT CTA GCA GAG TGT CGC ACC CTA GGC GAA 4944
Pro Glu Leu Asn Pro Glu Asp Leu Ala Glu Cys Arg Thr Leu Gly Glu
        1635                1640                1645
ATC GTT GAC TAT ATG GGC AGT AAA CTG CCG GCT GAA GGC TCT GCT AAT 4992
Ile Val Asp Tyr Met Gly Ser Lys Leu Pro Ala Glu Gly Ser Ala Asn
    1650                1655                1660
ACA AGT GCC GCT GCG TCT CTT AAT GTT AGT GCC GTT GCG GCG CCT CAA 5040
Thr Ser Ala Ala Ala Ser Leu Asn Val Ser Ala Val Ala Ala Pro Gln
1665                1670                1675                1680
GCT GCT GCG ACT CCT GTA TCG AAC GGT CTC TCT GCA GAG AAA GTG CAA 5088
Ala Ala Ala Thr Pro Val Ser Asn Gly Leu Ser Ala Glu Lys Val Gln
                1685                1690                1695
AGC ACT ATG ATG TCA GTA GTT GCA GAA AAG ACC GGC TAC CCA ACT GAA 5136
Ser Thr Met Met Ser Val Val Ala Glu Lys Thr Gly Tyr Pro Thr Glu
            1700                1705                1710
ATG CTA GAA CTT GGC ATG GAT ATG GAA GCC GAT TTA GGT ATC GAC TCA 5184
Met Leu Glu Leu Gly Met Asp Met Glu Ala Asp Leu Gly Ile Asp Ser
        1715                1720                1725
ATT AAA CGC GTT GAG ATT CTT GGC ACA GTA CAA GAT GAG CTA CCG GGT 5232
Ile Lys Arg Val Glu Ile Leu Gly Thr Val Gln Asp Glu Leu Pro Gly
    1730                1735                1740
CTA CCA GAG CTT AAT CCT GAA GAT TTA GCT GAG TGC CGT ACG CTG GGC 5280
Leu Pro Glu Leu Asn Pro Glu Asp Leu Ala Glu Cys Arg Thr Leu Gly
1745                1750                1755                1760
GAA ATC GTT GAC TAT ATG AAC TCT AAG CTG GCT GAC GGC TCT AAG CTT 5328
Glu Ile Val Asp Tyr Met Asn Ser Lys Leu Ala Asp Gly Ser Lys Leu
                1765                1770                1775

CCA GCT GAA GGC TCT GCT AAT ACA AGT GCC ACT GCT GCG ACT CCT GCA 5376
Pro Ala Glu Gly Ser Ala Asn Thr Ser Ala Thr Ala Ala Thr Pro Ala
            1780                1785                1790
GTG AAT GGT CTT TCT GCT GAC AAG GTA CAG GCG ACT ATG ATG TCT GTA 5424
Val Asn Gly Leu Ser Ala Asp Lys Val Gln Ala Thr Met Met Ser Val
        1795                1800                1805
GTT GCT GAA AAG ACC GGC TAC CCA ACT GAA ATG CTA GAA CTT GGC ATG 5472
Val Ala Glu Lys Thr Gly Tyr Pro Thr Glu Met Leu Glu Leu Gly Met
    1810                1815                1820
GAT ATG GAA GCA GAC CTT GGT ATT GAT TCT ATT AAG CGC GTT GAA ATT 5520
Asp Met Glu Ala Asp Leu Gly Ile Asp Ser Ile Lys Arg Val Glu Ile
1825                1830                1835                1840
CTT GGC ACA GTA CAA GAT GAG CTC CCA GGT TTA CCT GAG CTT AAT CCT 5568
Leu Gly Thr Val Gln Asp Glu Leu Pro Gly Leu Pro Glu Leu Asn Pro
                1845                1850                1855
GAA GAT CTC GCT GAG TGC CGC ACG CTT GGC GAA ATC GTT AGC TAT ATG 5616
Glu Asp Leu Ala Glu Cys Arg Thr Leu Gly Glu Ile Val Ser Tyr Met
            1860                1865                1870
AAC TCT CAA CTG GCT GAT GGC TCT AAA CTT TCT ACA AGT GCG GCT GAA 5664
Asn Ser Gln Leu Ala Asp Gly Ser Lys Leu Ser Thr Ser Ala Ala Glu
        1875                1880                1885
GGC TCT GCT GAT ACA AGT GCT GCA AAT GCT GCA AAG CCG GCA GCA ATT 5712
Gly Ser Ala Asp Thr Ser Ala Ala Asn Ala Ala Lys Pro Ala Ala Ile
    1890                1895                1900
TCG GCA GAA CCA AGT GTT GAG CTT CCT CCT CAT AGC GAG GTA GCG CTA 5760
Ser Ala Glu Pro Ser Val Glu Leu Pro Pro His Ser Glu Val Ala Leu
1905                1910                1915                1920
AAA AAG CTT AAT GCG GCG AAC AAG CTA GAA AAT TGT TTC GCC GCA GAC 5808
Lys Lys Leu Asn Ala Ala Asn Lys Leu Glu Asn Cys Phe Ala Ala Asp
                1925                1930                1935
GCA AGT GTT GTG ATT AAC GAT GAT GGT CAC AAC GCA GGC GTT TTA GCT 5856
Ala Ser Val Val Ile Asn Asp Asp Gly His Asn Ala Gly Val Leu Ala
            1940                1945                1950
GAG AAA CTT ATT AAA CAA GGC CTA AAA GTA GCC GTT GTG CGT TTA CCG 5904
Glu Lys Leu Ile Lys Gln Gly Leu Lys Val Ala Val Val Arg Leu Pro
        1955                1960                1965

AAA GGT CAG CCT CAA TCG CCA CTT TCA AGC GAT GTT GCT AGC TTT GAG 5952
Lys Gly Gln Pro Gln Ser Pro Leu Ser Ser Asp Val Ala Ser Phe Glu
    1970                1975                1980
CTT GCC TCA AGC CAA GAA TCT GAG CTT GAA GCC AGT ATC ACT GCA GTT 6000
Leu Ala Ser Ser Gln Glu Ser Glu Leu Glu Ala Ser Ile Thr Ala Val
1985                1990                1995                2000
ATC GCG CAG ATT GAA ACT CAG GTT GGC GCT ATT GGT GGC TTT ATT CAC 6048
Ile Ala Gln Ile Glu Thr Gln Val Gly Ala Ile Gly Gly Phe Ile His
                2005                2010                2015
TTG CAA CCA GAA GCG AAT ACA GAA GAG CAA ACG GCA GTA AAC CTA GAT 6096
Leu Gln Pro Glu Ala Asn Thr Glu Glu Gln Thr Ala Val Asn Leu Asp
            2020                2025                2030
GCG CAA AGT TTT ACT CAC GTT AGC AAT GCG TTC TTG TGG GCC AAA TTA 6144
Ala Gln Ser Phe Thr His Val Ser Asn Ala Phe Leu Trp Ala Lys Leu
        2035                2040                2045
TTG CAA CCA AAG CTC GTT GCT GGA GCA GAT GCG CGT CGC TGT TTT GTA 6192
Leu Gln Pro Lys Leu Val Ala Gly Ala Asp Ala Arg Arg Cys Phe Val
    2050                2055                2060
ACA GTA AGC CGT ATC GAC GGT GGC TTT GGT TAC CTA AAT ACT GAC GCC 6240
Thr Val Ser Arg Ile Asp Gly Gly Phe Gly Tyr Leu Asn Thr Asp Ala
2065                2070                2075                2080
CTA AAA GAT GCT GAG CTA AAC CAA GCA GCA TTA GCT GGT TTA ACT AAA 6288
Leu Lys Asp Ala Glu Leu Asn Gln Ala Ala Leu Ala Gly Leu Thr Lys
                2085                2090                2095
ACC TTA AGC CAT GAA TGG CCA CAA GTG TTC TGT CGC GCG CTA GAT ATT 6336
Thr Leu Ser His Glu Trp Pro Gln Val Phe Cys Arg Ala Leu Asp Ile
            2100                2105                2110
GCA ACA GAT GTT GAT GCA ACC CAT CTT GCT GAT GCA ATC ACC AGT GAA 6384
Ala Thr Asp Val Asp Ala Thr His Leu Ala Asp Ala Ile Thr Ser Glu
        2115                2120                2125
CTA TTT GAT AGC CAA GCT CAG CTA CCT GAA GTG GGC TTA AGC TTA ATT 6432
Leu Phe Asp Ser Gln Ala Gln Leu Pro Glu Val Gly Leu Ser Leu Ile
    2130                2135                2140
GAT GGC AAA GTT AAC CGC GTA ACT CTA GTT GCT GCT GAA GCT GCA GAT 6480
Asp Gly Lys Val Asn Arg Val Thr Leu Val Ala Ala Glu Ala Ala Asp
2145                2150                2155                2160

AAA ACA GCA AAA GCA GAG CTT AAC AGC ACA GAT AAA ATC TTA GTG ACT 6528
Lys Thr Ala Lys Ala Glu Leu Asn Ser Thr Asp Lys Ile Leu Val Thr
                2165                2170                2175
GGT GGG GCA AAA GGG GTG ACA TTT GAA TGT GCA CTG GCA TTA GCA TCT 6576
Gly Gly Ala Lys Gly Val Thr Phe Glu Cys Ala Leu Ala Leu Ala Ser
            2180                2185                2190
CGC AGC CAG TCT CAC TTT ATC TTA GCT GGG CGC AGT GAA TTA CAA GCT 6624
Arg Ser Gln Ser His Phe Ile Leu Ala Gly Arg Ser Glu Leu Gln Ala
        2195                2200                2205
TTA CCA AGC TGG GCT GAG GGT AAG CAA ACT AGC GAG CTA AAA TCA GCT 6672
Leu Pro Ser Trp Ala Glu Gly Lys Gln Thr Ser Glu Leu Lys Ser Ala
    2210                2215                2220
GCA ATC GCA CAT ATT ATT TCT ACT GGT CAA AAG CCA ACG CCT AAG CAA 6720
Ala Ile Ala His Ile Ile Ser Thr Gly Gln Lys Pro Thr Pro Lys Gln
2225                2230                2235                2240
GTT GAA GCC GCT GTG TGG CCA GTG CAA AGC AGC ATT GAA ATT AAT GCC 6768
Val Glu Ala Ala Val Trp Pro Val Gln Ser Ser Ile Glu Ile Asn Ala
                2245                2250                2255
GCC CTA GCC GCC TTT AAC AAA GTT GGC GCC TCA GCT GAA TAC GTC AGC 6816
Ala Leu Ala Ala Phe Asn Lys Val Gly Ala Ser Ala Glu Tyr Val Ser
            2260                2265                2270
ATG GAT GTT ACC GAT AGC GCC GCA ATC ACA GCA GCA CTT AAT GGT CGC 6864
Met Asp Val Thr Asp Ser Ala Ala Ile Thr Ala Ala Leu Asn Gly Arg
        2275                2280                2285
TCA AAT GAG ATC ACC GGT CTT ATT CAT GGC GCA GGT GTA CTA GCC GAC 6912
Ser Asn Glu Ile Thr Gly Leu Ile His Gly Ala Gly Val Leu Ala Asp
    2290                2295                2300
AAG CAT ATT CAA GAC AAG ACT CTT GCT GAA CTT GCT AAA GTT TAT GGC 6960
Lys His Ile Gln Asp Lys Thr Leu Ala Glu Leu Ala Lys Val Tyr Gly
2305                2310                2315                2320
ACT AAA GTC AAC GGC CTA AAA GCG CTG CTC GCG GCA CTT GAG CCA AGC 7008
Thr Lys Val Asn Gly Leu Lys Ala Leu Leu Ala Ala Leu Glu Pro Ser
                2325                2330                2335
AAA ATT AAA TTA CTT GCT ATG TTC TCA TCT GCA GCA GGT TTT TAC GGT 7056
Lys Ile Lys Leu Leu Ala Met Phe Ser Ser Ala Ala Gly Phe Tyr Gly
            2340                2345                2350

AAT ATC GGC CAA AGC GAT TAC GCG ATG TCG AAC GAT ATT CTT AAC AAG 7104
Asn Ile Gly Gln Ser Asp Tyr Ala Met Ser Asn Asp Ile Leu Asn Lys
        2355                2360                2365
GCA GCG CTG CAG TTC ACC GCT CGC AAC CCA CAA GCT AAA GTC ATG AGC 7152
Ala Ala Leu Gln Phe Thr Ala Arg Asn Pro Gln Ala Lys Val Met Ser
    2370                2375                2380
TTT AAC TGG GGT CCT TGG GAT GGC GGC ATG GTT AAC CCA GCG CTT AAA 7200
Phe Asn Trp Gly Pro Trp Asp Gly Gly Met Val Asn Pro Ala Leu Lys
2385                2390                2395                2400
AAG ATG TTT ACC GAG CGT GGT GTG TAC GTT ATT CCA CTA AAA GCA GGT 7248
Lys Met Phe Thr Glu Arg Gly Val Tyr Val Ile Pro Leu Lys Ala Gly
                2405                2410                2415
GCA GAG CTA TTT GCC ACT CAG CTA TTG GCT GAA ACT GGC GTG CAG TTG 7296
Ala Glu Leu Phe Ala Thr Gln Leu Leu Ala Glu Thr Gly Val Gln Leu
            2420                2425                2430
CTC ATT GGT ACG TCA ATG CAA GGT GGC AGC GAC ACT AAA GCA ACT GAG 7344
Leu Ile Gly Thr Ser Met Gln Gly Gly Ser Asp Thr Lys Ala Thr Glu
        2435                2440                2445
ACT GCT TCT GTA AAA AAG CTT AAT GCG GGT GAG GTG CTA AGT GCA TCG 7392
Thr Ala Ser Val Lys Lys Leu Asn Ala Gly Glu Val Leu Ser Ala Ser
    2450                2455                2460
CAT CCG CGT GCT GGT GCA CAA AAA ACA CCA CTA CAA GCT GTC ACT GCA 7440
His Pro Arg Ala Gly Ala Gln Lys Thr Pro Leu Gln Ala Val Thr Ala
2465                2470                2475                2480
ACG CGT CTG TTA ACC CCA AGT GCC ATG GTC TTC ATT GAA GAT CAC CGC 7488
Thr Arg Leu Leu Thr Pro Ser Ala Met Val Phe Ile Glu Asp His Arg
                2485                2490                2495
ATT GGC GGT AAC AGT GTG TTG CCA ACG GTA TGC GCC ATC GAC TGG ATG 7536
Ile Gly Gly Asn Ser Val Leu Pro Thr Val Cys Ala Ile Asp Trp Met
            2500                2505                2510
CGT GAA GCG GCA AGC GAC ATG CTT GGC GCT CAA GTT AAG GTA CTT GAT 7584
Arg Glu Ala Ala Ser Asp Met Leu Gly Ala Gln Val Lys Val Leu Asp
        2515                2520                2525
TAC AAG CTA TTA AAA GGC ATT GTA TTT GAG ACT GAT GAG CCG CAA GAG 7632
Tyr Lys Leu Leu Lys Gly Ile Val Phe Glu Thr Asp Glu Pro Gln Glu
    2530                2535                2540

TTA ACA CTT GAG CTA ACG CCA GAC GAT TCA GAC GAA GCT ACG CTA CAA 7680
Leu Thr Leu Glu Leu Thr Pro Asp Asp Ser Asp Glu Ala Thr Leu Gln
2545                2550                2555                2560
GCA TTA ATC AGC TGT AAT GGG CGT CCG CAA TAC AAG GCG ACG CTT ATC 7728
Ala Leu Ile Ser Cys Asn Gly Arg Pro Gln Tyr Lys Ala Thr Leu Ile
                2565                2570                2575
AGT GAT AAT GCC GAT ATT AAG CAA CTT AAC AAG CAG TTT GAT TTA AGC 7776
Ser Asp Asn Ala Asp Ile Lys Gln Leu Asn Lys Gln Phe Asp Leu Ser
            2580                2585                2590
GCT AAG GCG ATT ACC ACA GCA AAA GAG CTT TAT AGC AAC GGC ACC TTG 7824
Ala Lys Ala Ile Thr Thr Ala Lys Glu Leu Tyr Ser Asn Gly Thr Leu
        2595                2600                2605
TTC CAC GGT CCG CGT CTA CAA GGG ATC CAA TCT GTA GTG CAG TTC GAT 7872
Phe His Gly Pro Arg Leu Gln Gly Ile Gln Ser Val Val Gln Phe Asp
    2610                2615                2620
GAT CAA GGC TTA ATT GCT AAA GTC GCT CTG CCT AAG GTT GAA CTT AGC 7920
Asp Gln Gly Leu Ile Ala Lys Val Ala Leu Pro Lys Val Glu Leu Ser
2625                2630                2635                2640
GAT TGT GGT GAG TTC TTG CCG CAA ACC CAC ATG GGT GGC AGT CAA CCT 7968
Asp Cys Gly Glu Phe Leu Pro Gln Thr His Met Gly Gly Ser Gln Pro
                2645                2650                2655
TTT GCT GAG GAC TTG CTA TTA CAA GCT ATG CTG GTT TGG GCT CGC CTT 8016
Phe Ala Glu Asp Leu Leu Leu Gln Ala Met Leu Val Trp Ala Arg Leu
            2660                2665                2670
AAA ACT GGC TCG GCA AGT TTG CCA TCA AGC ATT GGT GAG TTT ACC TCA 8064
Lys Thr Gly Ser Ala Ser Leu Pro Ser Ser Ile Gly Glu Phe Thr Ser
        2675                2680                2685
TAC CAA CCA ATG GCC TTT GGT GAA ACT GGT ACC ATA GAG CTT GAA GTG 8112
Tyr Gln Pro Met Ala Phe Gly Glu Thr Gly Thr Ile Glu Leu Glu Val
    2690                2695                2700
ATT AAG CAC AAC AAA CGC TCA CTT GAA GCG AAT GTT GCG CTA TAT CGT 8160
Ile Lys His Asn Lys Arg Ser Leu Glu Ala Asn Val Ala Leu Tyr Arg
2705                2710                2715                2720
GAC AAC GGC GAG TTA AGT GCC ATG TTT AAG TCA GCT AAA ATC ACC ATT 8208
Asp Asn Gly Glu Leu Ser Ala Met Phe Lys Ser Ala Lys Ile Thr Ile
                2725                2730                2735

AGC AAA AGC TTA AAT TCA GCA TTT TTA CCT GCT GTC TTA GCA AAC GAC 8256
Ser Lys Ser Leu Asn Ser Ala Phe Leu Pro Ala Val Leu Ala Asn Asp
            2740                2745                2750
AGT GAG GCG AAT 8268
Ser Glu Ala Asn
        2755

SEQ ID NO: 11
Sequence Length: 2756
Sequence Type: Amino acid
Molecular Type: Peptide
Sequence
Met Ser Gln Thr Ser Lys Pro Thr Asn Ser Ala Thr Glu Gln Ala Gln
1               5                   10                  15
Asp Ser Gln Ala Asp Ser Arg Leu Asn Lys Arg Leu Lys Asp Met Pro
            20                  25                  30
Ile Ala Ile Val Gly Met Ala Ser Ile Phe Ala Asn Ser Arg Tyr Leu
        35                  40                  45
Asn Lys Phe Trp Asp Leu Ile Ser Glu Lys Ile Asp Ala Ile Thr Glu
    50                  55                  60
Leu Pro Ser Thr His Trp Gln Pro Glu Glu Tyr Tyr Asp Ala Asp Lys
65                  70                  75                  80
Thr Ala Ala Asp Lys Ser Tyr Cys Lys Arg Gly Gly Phe Leu Pro Asp
                85                  90                  95
Val Asp Phe Asn Pro Met Glu Phe Gly Leu Pro Pro Asn Ile Leu Glu
            100                 105                 110
Leu Thr Asp Ser Ser Gln Leu Leu Ser Leu Ile Val Ala Lys Glu Val
        115                 120                 125
Leu Ala Asp Ala Asn Leu Pro Glu Asn Tyr Asp Arg Asp Lys Ile Gly
    130                 135                 140
Ile Thr Leu Gly Val Gly Gly Gly Gln Lys Ile Ser His Ser Leu Thr
145                 150                 155                 160
Ala Arg Leu Gln Tyr Pro Val Leu Lys Lys Val Phe Ala Asn Ser Gly
                165                 170                 175
Ile Ser Asp Thr Asp Ser Glu Met Leu Ile Lys Lys Phe Gln Asp Gln
            180                 185                 190

Tyr Val His Trp Glu Glu Asn Ser Phe Pro Gly Ser Leu Gly Asn Val
        195                 200                 205
Ile Ala Gly Arg Ile Ala Asn Arg Phe Asp Phe Gly Gly Met Asn Cys
    210                 215                 220
Val Val Asp Ala Ala Cys Ala Gly Ser Leu Ala Ala Met Arg Met Ala
225                 230                 235                 240
Leu Thr Glu Leu Thr Glu Gly Arg Ser Glu Met Met Ile Thr Gly Gly
                245                 250                 255
Val Cys Thr Asp Asn Ser Pro Ser Met Tyr Met Ser Phe Ser Lys Thr
            260                 265                 270
Pro Ala Phe Thr Thr Asn Glu Thr Ile Gln Pro Phe Asp Ile Asp Ser
        275                 280                 285
Lys Gly Met Met Ile Gly Glu Gly Ile Gly Met Val Ala Leu Lys Arg
    290                 295                 300
Leu Glu Asp Ala Glu Arg Asp Gly Asp Arg Ile Tyr Ser Val Ile Lys
305                 310                 315                 320
Gly Val Gly Ala Ser Ser Asp Gly Lys Phe Lys Ser Ile Tyr Ala Pro
                325                 330                 335
Arg Pro Ser Gly Gln Ala Lys Ala Leu Asn Arg Ala Tyr Asp Asp Ala
            340                 345                 350
Gly Phe Ala Pro His Thr Leu Gly Leu Ile Glu Ala His Gly Thr Gly
        355                 360                 365
Thr Ala Ala Gly Asp Ala Ala Glu Phe Ala Gly Leu Cys Ser Val Phe
    370                 375                 380
Ala Glu Gly Asn Asp Thr Lys Gln His Ile Ala Leu Gly Ser Val Lys
385                 390                 395                 400
Ser Gln Ile Gly His Thr Lys Ser Thr Ala Gly Thr Ala Gly Leu Ile
                405                 410                 415
Lys Ala Ala Leu Ala Leu His His Lys Val Leu Pro Pro Thr Ile Asn
            420                 425                 430
Val Ser Gln Pro Ser Pro Lys Leu Asp Ile Glu Asn Ser Pro Phe Tyr
        435                 440                 445
Leu Asn Thr Glu Thr Arg Pro Trp Leu Pro Arg Val Asp Gly Thr Pro
    450                 455                 460
Arg Arg Ala Gly Ile Ser Ser Phe Gly Phe Gly Gly Thr Asn Phe His
465                 470                 475                 480

Phe Val Leu Glu Glu Tyr Asn Gln Glu His Ser Arg Thr Asp Ser Glu
                485                 490                 495
Lys Ala Lys Tyr Arg Gln Arg Gln Val Ala Gln Ser Phe Leu Val Ser
            500                 505                 510
Ala Ser Asp Lys Ala Ser Leu Ile Asn Glu Leu Asn Val Leu Ala Ala
        515                 520                 525
Ser Ala Ser Gln Ala Glu Phe Ile Leu Lys Asp Ala Ala Ala Asn Tyr
    530                 535                 540
Gly Val Arg Glu Leu Asp Lys Asn Ala Pro Arg Ile Gly Leu Val Ala
545                 550                 555                 560
Asn Thr Ala Glu Glu Leu Ala Gly Leu Ile Lys Gln Ala Leu Ala Lys
                565                 570                 575
Leu Ala Ala Ser Asp Asp Asn Ala Trp Gln Leu Pro Gly Gly Thr Ser
            580                 585                 590
Tyr Arg Ala Ala Ala Val Glu Gly Lys Val Ala Ala Leu Phe Ala Gly
        595                 600                 605
Gln Gly Ser Gln Tyr Leu Asn Met Gly Arg Asp Leu Thr Cys Tyr Tyr
    610                 615                 620
Pro Glu Met Arg Gln Gln Phe Val Thr Ala Asp Lys Val Phe Ala Ala
625                 630                 635                 640
Asn Asp Lys Thr Pro Leu Ser Gln Thr Leu Tyr Pro Lys Pro Val Phe
                645                 650                 655
Asn Lys Asp Glu Leu Lys Ala Gln Glu Ala Ile Leu Thr Asn Thr Ala
            660                 665                 670
Asn Ala Gln Ser Ala Ile Gly Ala Ile Ser Met Gly Gln Tyr Asp Leu
        675                 680                 685
Phe Thr Ala Ala Gly Phe Asn Ala Asp Met Val Ala Gly His Ser Phe
    690                 695                 700
Gly Glu Leu Ser Ala Leu Cys Ala Ala Gly Val Ile Ser Ala Asp Asp
705                 710                 715                 720
Tyr Tyr Lys Leu Ala Phe Ala Arg Gly Glu Ala Met Ala Thr Lys Ala
                725                 730                 735
Pro Ala Lys Asp Gly Val Glu Ala Asp Ala Gly Ala Met Phe Ala Ile
            740                 745                 750
Ile Thr Lys Ser Ala Ala Asp Leu Glu Thr Val Glu Ala Thr Ile Ala
        755                 760                 765

Lys Phe Asp Gly Val Lys Val Ala Asn Tyr Asn Ala Pro Thr Gln Ser
    770                 775                 780
Val Ile Ala Gly Pro Thr Ala Thr Thr Ala Asp Ala Ala Lys Ala Leu
785                 790                 795                 800
Thr Glu Leu Gly Tyr Lys Ala Ile Asn Leu Pro Val Ser Gly Ala Phe
                805                 810                 815
His Thr Glu Leu Val Gly His Ala Gln Ala Pro Phe Ala Lys Ala Ile
            820                 825                 830
Asp Ala Ala Lys Phe Thr Lys Thr Ser Arg Ala Leu Tyr Ser Asn Ala
        835                 840                 845
Thr Gly Gly Leu Tyr Glu Ser Thr Ala Ala Lys Ile Lys Ala Ser Phe
    850                 855                 860
Lys Lys His Met Leu Gln Ser Val Arg Phe Thr Ser Gln Leu Glu Ala
865                 870                 875                 880
Met Tyr Asn Asp Gly Ala Arg Val Phe Val Glu Phe Gly Pro Lys Asn
                885                 890                 895
Ile Leu Gln Lys Leu Val Gln Gly Thr Leu Val Asn Thr Glu Asn Glu
            900                 905                 910
Val Cys Thr Ile Ser Ile Asn Pro Asn Pro Lys Val Asp Ser Asp Leu
        915                 920                 925
Gln Leu Lys Gln Ala Ala Met Gln Leu Ala Val Thr Gly Val Val Leu
    930                 935                 940
Ser Glu Ile Asp Pro Tyr Gln Ala Asp Ile Ala Ala Pro Ala Lys Lys
945                 950                 955                 960
Ser Pro Met Ser Ile Ser Leu Asn Ala Ala Asn His Ile Ser Lys Ala
                965                 970                 975
Thr Arg Ala Lys Met Ala Lys Ser Leu Glu Thr Gly Ile Val Thr Ser
            980                 985                 990
Gln Ile Glu His Val Ile Glu Glu Lys Ile Val Glu Val Glu Lys Leu
        995                 1000                1005
Val Glu Val Glu Lys Ile Val Glu Lys Val Val Glu Val Glu Lys Val
    1010                1015                1020
Val Glu Val Glu Ala Pro Val Asn Ser Val Gln Ala Asn Ala Ile Gln
1025                1030                1035                1040
Thr Arg Ser Val Val Ala Pro Val Ile Glu Asn Gln Val Val Ser Lys
                1045                1050                1055

Asn Ser Lys Pro Ala Val Gln Ser Ile Ser Gly Asp Ala Leu Ser Asn
            1060                1065                1070
Phe Phe Ala Ala Gln Gln Gln Thr Ala Gln Leu His Gln Gln Phe Leu
        1075                1080                1085
Ala Ile Pro Gln Gln Tyr Gly Glu Thr Phe Thr Thr Leu Met Thr Glu
    1090                1095                1100
Gln Ala Lys Leu Ala Ser Ser Gly Val Ala Ile Pro Glu Ser Leu Gln
1105                1110                1115                1120
Arg Ser Met Glu Gln Phe His Gln Leu Gln Ala Gln Thr Leu Gln Ser
                1125                1130                1135
His Thr Gln Phe Leu Glu Met Gln Ala Gly Ser Asn Ile Ala Ala Leu
            1140                1145                1150
Asn Leu Leu Asn Ser Ser Gln Ala Thr Tyr Ala Pro Ala Ile His Asn
        1155                1160                1165
Glu Ala Ile Gln Ser Gln Val Val Gln Ser Gln Thr Ala Val Gln Pro
    1170                1175                1180
Val Ile Ser Thr Gln Val Asn His Val Ser Glu Gln Pro Thr Gln Ala
1185                1190                1195                1200
Pro Ala Pro Lys Ala Gln Pro Ala Pro Val Thr Thr Ala Val Gln Thr
                1205                1210                1215
Ala Pro Ala Gln Val Val Arg Gln Ala Ala Pro Val Gln Ala Ala Ile
            1220                1225                1230
Glu Pro Ile Asn Thr Ser Val Ala Thr Thr Thr Pro Ser Ala Phe Ser
        1235                1240                1245
Ala Glu Thr Ala Leu Ser Ala Thr Lys Val Gln Ala Thr Met Leu Glu
    1250                1255                1260
Val Val Ala Glu Lys Thr Gly Tyr Pro Thr Glu Met Leu Glu Leu Glu
1265                1270                1275                1280
Met Asp Met Glu Ala Asp Leu Gly Ile Asp Ser Ile Lys Arg Val Glu
                1285                1290                1295
Ile Leu Gly Thr Val Gln Asp Glu Leu Pro Gly Leu Pro Glu Leu Ser
            1300                1305                1310
Pro Glu Asp Leu Ala Glu Cys Arg Thr Leu Gly Glu Ile Val Asp Tyr
        1315                1320                1325
Met Gly Ser Lys Leu Pro Ala Glu Gly Ser Met Asn Ser Gln Leu Ser
    1330                1335                1340

Thr Gly Ser Ala Ala Ala Thr Pro Ala Ala Asn Gly Leu Ser Ala Glu
1345                1350                1355                1360
Lys Val Gln Ala Thr Met Met Ser Val Val Ala Glu Lys Thr Gly Tyr
                1365                1370                1375
Pro Thr Glu Met Leu Glu Leu Glu Met Asp Met Glu Ala Asp Leu Gly
            1380                1385                1390
Ile Asp Ser Ile Lys Arg Val Glu Ile Leu Gly Thr Val Gln Asp Glu
        1395                1400                1405
Leu Pro Gly Leu Pro Glu Leu Ser Pro Glu Asp Leu Ala Glu Cys Arg
    1410                1415                1420
Thr Leu Gly Glu Ile Val Asp Tyr Met Asn Ser Lys Leu Ala Asp Gly
1425                1430                1435                1440
Ser Lys Leu Pro Ala Glu Gly Ser Met Asn Ser Gln Leu Ser Thr Ser
                1445                1450                1455
Ala Ala Ala Ala Thr Pro Ala Ala Asn Gly Leu Ser Ala Glu Lys Val
            1460                1465                1470
Gln Ala Thr Met Met Ser Val Val Ala Glu Lys Thr Gly Tyr Pro Thr
        1475                1480                1485
Glu Met Leu Glu Leu Glu Met Asp Met Glu Ala Asp Leu Gly Ile Asp
    1490                1495                1500
Ser Ile Lys Arg Val Glu Ile Leu Gly Thr Val Gln Asp Glu Leu Pro
1505                1510                1515                1520
Gly Leu Pro Glu Leu Asn Pro Glu Asp Leu Ala Glu Cys Arg Thr Leu
                1525                1530                1535
Gly Glu Ile Val Thr Tyr Met Asn Ser Lys Leu Ala Asp Gly Ser Lys
            1540                1545                1550
Leu Pro Ala Glu Gly Ser Met His Tyr Gln Leu Ser Thr Ser Thr Ala
        1555                1560                1565
Ala Ala Thr Pro Val Ala Asn Gly Leu Ser Ala Glu Lys Val Gln Ala
    1570                1575                1580
Thr Met Met Ser Val Val Ala Asp Lys Thr Gly Tyr Pro Thr Glu Met
1585                1590                1595                1600
Leu Glu Leu Glu Met Asp Met Glu Ala Asp Leu Gly Ile Asp Ser Ile
                1605                1610                1615
Lys Arg Val Glu Ile Leu Gly Thr Val Gln Asp Glu Leu Pro Gly Leu
            1620                1625                1630

Pro Glu Leu Asn Pro Glu Asp Leu Ala Glu Cys Arg Thr Leu Gly Glu
        1635                1640                1645
Ile Val Asp Tyr Met Gly Ser Lys Leu Pro Ala Glu Gly Ser Ala Asn
    1650                1655                1660
Thr Ser Ala Ala Ala Ser Leu Asn Val Ser Ala Val Ala Ala Pro Gln
1665                1670                1675                1680
Ala Ala Ala Thr Pro Val Ser Asn Gly Leu Ser Ala Glu Lys Val Gln
                1685                1690                1695
Ser Thr Met Met Ser Val Val Ala Glu Lys Thr Gly Tyr Pro Thr Glu
            1700                1705                1710
Met Leu Glu Leu Gly Met Asp Met Glu Ala Asp Leu Gly Ile Asp Ser
        1715                1720                1725
Ile Lys Arg Val Glu Ile Leu Gly Thr Val Gln Asp Glu Leu Pro Gly
    1730                1735                1740
Leu Pro Glu Leu Asn Pro Glu Asp Leu Ala Glu Cys Arg Thr Leu Gly
1745                1750                1755                1760
Glu Ile Val Asp Tyr Met Asn Ser Lys Leu Ala Asp Gly Ser Lys Leu
                1765                1770                1775
Pro Ala Glu Gly Ser Ala Asn Thr Ser Ala Thr Ala Ala Thr Pro Ala
            1780                1785                1790
Val Asn Gly Leu Ser Ala Asp Lys Val Gln Ala Thr Met Met Ser Val
        1795                1800                1805
Val Ala Glu Lys Thr Gly Tyr Pro Thr Glu Met Leu Glu Leu Gly Met
    1810                1815                1820
Asp Met Glu Ala Asp Leu Gly Ile Asp Ser Ile Lys Arg Val Glu Ile
1825                1830                1835                1840
Leu Gly Thr Val Gln Asp Glu Leu Pro Gly Leu Pro Glu Leu Asn Pro
                1845                1850                1855
Glu Asp Leu Ala Glu Cys Arg Thr Leu Gly Glu Ile Val Ser Tyr Met
            1860                1865                1870
Asn Ser Gln Leu Ala Asp Gly Ser Lys Leu Ser Thr Ser Ala Ala Glu
        1875                1880                1885
Gly Ser Ala Asp Thr Ser Ala Ala Asn Ala Ala Lys Pro Ala Ala Ile
    1890                1895                1900
Ser Ala Glu Pro Ser Val Glu Leu Pro Pro His Ser Glu Val Ala Leu
1905                1910                1915                1920

Lys Lys Leu Asn Ala Ala Asn Lys Leu Glu Asn Cys Phe Ala Ala Asp
                1925                1930                1935
Ala Ser Val Val Ile Asn Asp Asp Gly His Asn Ala Gly Val Leu Ala
            1940                1945                1950
Glu Lys Leu Ile Lys Gln Gly Leu Lys Val Ala Val Val Arg Leu Pro
        1955                1960                1965
Lys Gly Gln Pro Gln Ser Pro Leu Ser Ser Asp Val Ala Ser Phe Glu
    1970                1975                1980
Leu Ala Ser Ser Gln Glu Ser Glu Leu Glu Ala Ser Ile Thr Ala Val
1985                1990                1995                2000
Ile Ala Gln Ile Glu Thr Gln Val Gly Ala Ile Gly Gly Phe Ile His
                2005                2010                2015
Leu Gln Pro Glu Ala Asn Thr Glu Glu Gln Thr Ala Val Asn Leu Asp
            2020                2025                2030
Ala Gln Ser Phe Thr His Val Ser Asn Ala Phe Leu Trp Ala Lys Leu
        2035                2040                2045
Leu Gln Pro Lys Leu Val Ala Gly Ala Asp Ala Arg Arg Cys Phe Val
    2050                2055                2060
Thr Val Ser Arg Ile Asp Gly Gly Phe Gly Tyr Leu Asn Thr Asp Ala
2065                2070                2075                2080
Leu Lys Asp Ala Glu Leu Asn Gln Ala Ala Leu Ala Gly Leu Thr Lys
                2085                2090                2095
Thr Leu Ser His Glu Trp Pro Gln Val Phe Cys Arg Ala Leu Asp Ile
            2100                2105                2110
Ala Thr Asp Val Asp Ala Thr His Leu Ala Asp Ala Ile Thr Ser Glu
        2115                2120                2125
Leu Phe Asp Ser Gln Ala Gln Leu Pro Glu Val Gly Leu Ser Leu Ile
    2130                2135                2140
Asp Gly Lys Val Asn Arg Val Thr Leu Val Ala Ala Glu Ala Ala Asp
2145                2150                2155                2160
Lys Thr Ala Lys Ala Glu Leu Asn Ser Thr Asp Lys Ile Leu Val Thr
                2165                2170                2175
Gly Gly Ala Lys Gly Val Thr Phe Glu Cys Ala Leu Ala Leu Ala Ser
            2180                2185                2190
Arg Ser Gln Ser His Phe Ile Leu Ala Gly Arg Ser Glu Leu Gln Ala
        2195                2200                2205

Leu Pro Ser Trp Ala Glu Gly Lys Gln Thr Ser Glu Leu Lys Ser Ala
    2210                2215                2220
Ala Ile Ala His Ile Ile Ser Thr Gly Gln Lys Pro Thr Pro Lys Gln
2225                2230                2235                2240
Val Glu Ala Ala Val Trp Pro Val Gln Ser Ser Ile Glu Ile Asn Ala
                2245                2250                2255
Ala Leu Ala Ala Phe Asn Lys Val Gly Ala Ser Ala Glu Tyr Val Ser
            2260                2265                2270
Met Asp Val Thr Asp Ser Ala Ala Ile Thr Ala Ala Leu Asn Gly Arg
        2275                2280                2285
Ser Asn Glu Ile Thr Gly Leu Ile His Gly Ala Gly Val Leu Ala Asp
    2290                2295                2300
Lys His Ile Gln Asp Lys Thr Leu Ala Glu Leu Ala Lys Val Tyr Gly
2305                2310                2315                2320
Thr Lys Val Asn Gly Leu Lys Ala Leu Leu Ala Ala Leu Glu Pro Ser
                2325                2330                2335
Lys Ile Lys Leu Leu Ala Met Phe Ser Ser Ala Ala Gly Phe Tyr Gly
            2340                2345                2350
Asn Ile Gly Gln Ser Asp Tyr Ala Met Ser Asn Asp Ile Leu Asn Lys
        2355                2360                2365
Ala Ala Leu Gln Phe Thr Ala Arg Asn Pro Gln Ala Lys Val Met Ser
    2370                2375                2380
Phe Asn Trp Gly Pro Trp Asp Gly Gly Met Val Asn Pro Ala Leu Lys
2385                2390                2395                2400
Lys Met Phe Thr Glu Arg Gly Val Tyr Val Ile Pro Leu Lys Ala Gly
                2405                2410                2415
Ala Glu Leu Phe Ala Thr Gln Leu Leu Ala Glu Thr Gly Val Gln Leu
            2420                2425                2430
Leu Ile Gly Thr Ser Met Gln Gly Gly Ser Asp Thr Lys Ala Thr Glu
        2435                2440                2445
Thr Ala Ser Val Lys Lys Leu Asn Ala Gly Glu Val Leu Ser Ala Ser
    2450                2455                2460
His Pro Arg Ala Gly Ala Gln Lys Thr Pro Leu Gln Ala Val Thr Ala
2465                2470                2475                2480
Thr Arg Leu Leu Thr Pro Ser Ala Met Val Phe Ile Glu Asp His Arg
                2485                2490                2495

Ile Gly Gly Asn Ser Val Leu Pro Thr Val Cys Ala Ile Asp Trp Met
            2500                2505                2510
Arg Glu Ala Ala Ser Asp Met Leu Gly Ala Gln Val Lys Val Leu Asp
        2515                2520                2525
Tyr Lys Leu Leu Lys Gly Ile Val Phe Glu Thr Asp Glu Pro Gln Glu
    2530                2535                2540
Leu Thr Leu Glu Leu Thr Pro Asp Asp Ser Asp Glu Ala Thr Leu Gln
2545                2550                2555                2560
Ala Leu Ile Ser Cys Asn Gly Arg Pro Gln Tyr Lys Ala Thr Leu Ile
                2565                2570                2575
Ser Asp Asn Ala Asp Ile Lys Gln Leu Asn Lys Gln Phe Asp Leu Ser
            2580                2585                2590
Ala Lys Ala Ile Thr Thr Ala Lys Glu Leu Tyr Ser Asn Gly Thr Leu
        2595                2600                2605
Phe His Gly Pro Arg Leu Gln Gly Ile Gln Ser Val Val Gln Phe Asp
    2610                2615                2620
Asp Gln Gly Leu Ile Ala Lys Val Ala Leu Pro Lys Val Glu Leu Ser
2625                2630                2635                2640
Asp Cys Gly Glu Phe Leu Pro Gln Thr His Met Gly Gly Ser Gln Pro
                2645                2650                2655
Phe Ala Glu Asp Leu Leu Leu Gln Ala Met Leu Val Trp Ala Arg Leu
            2660                2665                2670
Lys Thr Gly Ser Ala Ser Leu Pro Ser Ser Ile Gly Glu Phe Thr Ser
        2675                2680                2685
Tyr Gln Pro Met Ala Phe Gly Glu Thr Gly Thr Ile Glu Leu Glu Val
    2690                2695                2700
Ile Lys His Asn Lys Arg Ser Leu Glu Ala Asn Val Ala Leu Tyr Arg
2705                2710                2715                2720
Asp Asn Gly Glu Leu Ser Ala Met Phe Lys Ser Ala Lys Ile Thr Ile
                2725                2730                2735
Ser Lys Ser Leu Asn Ser Ala Phe Leu Pro Ala Val Leu Ala Asn Asp
            2740                2745                2750
Ser Glu Ala Asn
        2755

SEQ ID NO: 12
Sequence Length: 2340

Sequence Type: Nucleic acid
Strandedness: Double strand
Topology: Linear
Molecular Type: Genomic DNA
Source: Shewanella putrefaciens SCRC-2874 (FERM BP-1625)
Sequence
GTG GAA CAA ACG CCT AAA GCT AGT GCG ATG CCG CTG CGC ATC GCA CTT 48
Val Glu Gln Thr Pro Lys Ala Ser Ala Met Pro Leu Arg Ile Ala Leu
1               5                   10                  15
ATC TTA CTG CCA ACA CCG CAG TTT GAA GTT AAC TCT GTC GAC CAG TCA 96
Ile Leu Leu Pro Thr Pro Gln Phe Glu Val Asn Ser Val Asp Gln Ser
            20                  25                  30
GTA TTA GCC AGC TAT CAA ACA CTG CAG CCT GAG CTA AAT GCC CTG CTT 144
Val Leu Ala Ser Tyr Gln Thr Leu Gln Pro Glu Leu Asn Ala Leu Leu
        35                  40                  45
AAT AGT GCG CCG ACA CCT GAA ATG CTC AGC ATC ACT ATC TCA GAT GAT 192
Asn Ser Ala Pro Thr Pro Glu Met Leu Ser Ile Thr Ile Ser Asp Asp
    50                  55                  60
AGC GAT GCA AAC AGC TTT GAG TCG CAG CTA AAT GCT GCG ACC AAC GCA 240
Ser Asp Ala Asn Ser Phe Glu Ser Gln Leu Asn Ala Ala Thr Asn Ala
65                  70                  75                  80
ATT AAC AAT GGC TAT ATC GTC AAG CTT GCT ACG GCA ACT CAC GCT TTG 288
Ile Asn Asn Gly Tyr Ile Val Lys Leu Ala Thr Ala Thr His Ala Leu
                85                  90                  95
TTA ATG CTG CCT GCA TTA AAA GCG GCG CAA ATG CGG ATC CAT CCT CAT 336
Leu Met Leu Pro Ala Leu Lys Ala Ala Gln Met Arg Ile His Pro His
            100                 105                 110
GCG CAG CTT GCC GCT ATG CAG CAA GCT AAA TCG ACG CCA ATG AGT CAA 384
Ala Gln Leu Ala Ala Met Gln Gln Ala Lys Ser Thr Pro Met Ser Gln
        115                 120                 125
GTA TCT GGT GAG CTA AAG CTT GGC GCT AAT GCG CTA AGC CTA GCT CAG 432
Val Ser Gly Glu Leu Lys Leu Gly Ala Asn Ala Leu Ser Leu Ala Gln
    130                 135                 140
ACT AAT GCG CTG TCT CAT GCT TTA AGC CAA GCC AAG CGT AAC TTA ACT 480
Thr Asn Ala Leu Ser His Ala Leu Ser Gln Ala Lys Arg Asn Leu Thr
145                 150                 155                 160

GAT GTC AGC GTG AAT GAG TGT TTT GAG AAC CTC AAA AGT GAA CAG CAG 528
Asp Val Ser Val Asn Glu Cys Phe Glu Asn Leu Lys Ser Glu Gln Gln
                165                 170                 175
TTC ACA GAG GTT TAT TCG CTT ATT CAG CAA CTT GCT AGC CGC ACC CAT 576
Phe Thr Glu Val Tyr Ser Leu Ile Gln Gln Leu Ala Ser Arg Thr His
            180                 185                 190
GTG AGA AAA GAG GTT AAT CAA GGT GTG GAA CTT GGC CCT AAA CAA GCC 624
Val Arg Lys Glu Val Asn Gln Gly Val Glu Leu Gly Pro Lys Gln Ala
        195                 200                 205
AAA AGC CAC TAT TGG TTT AGC GAA TTT CAC CAA AAC CGT GTT GCT GCC 672
Lys Ser His Tyr Trp Phe Ser Glu Phe His Gln Asn Arg Val Ala Ala
    210                 215                 220
ATC AAC TTT ATT AAT GGC CAA CAA GCA ACC AGC TAT GTG CTT ACT CAA 720
Ile Asn Phe Ile Asn Gly Gln Gln Ala Thr Ser Tyr Val Leu Thr Gln
225                 230                 235                 240
GGT TCA GGA TTG TTA GCT GCG AAA TCA ATG CTA AAC CAG CAA AGA TTA 768
Gly Ser Gly Leu Leu Ala Ala Lys Ser Met Leu Asn Gln Gln Arg Leu
                245                 250                 255
ATG TTT ATC TTG CCG GGT AAC AGT CAG CAA CAA ATA ACC GCA TCA ATA 816
Met Phe Ile Leu Pro Gly Asn Ser Gln Gln Gln Ile Thr Ala Ser Ile
            260                 265                 270
ACT CAG TTA ATG CAG CAA TTA GAG CGT TTG CAG GTA ACT GAG GTT AAT 864
Thr Gln Leu Met Gln Gln Leu Glu Arg Leu Gln Val Thr Glu Val Asn
        275                 280                 285
GAG CTT TCT CTA GAA TGC CAA CTA GAG CTG CTC AGC ATA ATG TAT GAC 912
Glu Leu Ser Leu Glu Cys Gln Leu Glu Leu Leu Ser Ile Met Tyr Asp
    290                 295                 300
AAC TTA GTC AAC GCA GAC AAA CTC ACT ACT CGC GAT AGT AAG CCC GCT 960
Asn Leu Val Asn Ala Asp Lys Leu Thr Thr Arg Asp Ser Lys Pro Ala
305                 310                 315                 320
TAT CAG GCT GTG ATT CAA GCA AGC TCT GTT AGC GCT GCA AAG CAA GAG 1008
Tyr Gln Ala Val Ile Gln Ala Ser Ser Val Ser Ala Ala Lys Gln Glu
                325                 330                 335
TTA AGC GCG CTT AAC GAT GCA CTC ACA GCG CTG TTT GCT GAG CAA ACA 1056
Leu Ser Ala Leu Asn Asp Ala Leu Thr Ala Leu Phe Ala Glu Gln Thr
            340                 345                 350

AAC GCC ACA TCA ACG AAT AAA GGC TTA ATC CAA TAC AAA ACA CCG GCG 1104
Asn Ala Thr Ser Thr Asn Lys Gly Leu Ile Gln Tyr Lys Thr Pro Ala
        355                 360                 365
GGC AGT TAC TTA ACC CTA ACA CCG CTT GGC AGC AAC AAT GAC AAC GCC 1152
Gly Ser Tyr Leu Thr Leu Thr Pro Leu Gly Ser Asn Asn Asp Asn Ala
    370                 375                 380
CAA GCG GGT CTT GCT TTT GTC TAT CCG GGT GTG GGA ACG GTT TAC GCC 1200
Gln Ala Gly Leu Ala Phe Val Tyr Pro Gly Val Gly Thr Val Tyr Ala
385                 390                 395                 400
GAT ATG CTT AAT GAG CTG CAT CAG TAC TTC CCT GCG CTT TAC GCC AAA 1248
Asp Met Leu Asn Glu Leu His Gln Tyr Phe Pro Ala Leu Tyr Ala Lys
                405                 410                 415
CTT GAG CGT GAA GGC GAT TTA AAG GCG ATG CTA CAA GCA GAA GAT ATC 1296
Leu Glu Arg Glu Gly Asp Leu Lys Ala Met Leu Gln Ala Glu Asp Ile
            420                 425                 430
TAT CAT CTT GAC CCT AAA CAT GCT GCC CAA ATG AGC TTA GGT GAC TTA 1344
Tyr His Leu Asp Pro Lys His Ala Ala Gln Met Ser Leu Gly Asp Leu
        435                 440                 445
GCC ATT GCT GGC GTG GGG AGC AGC TAC CTG TTA ACT CAG CTG CTC ACC 1392
Ala Ile Ala Gly Val Gly Ser Ser Tyr Leu Leu Thr Gln Leu Leu Thr
    450                 455                 460
GAT GAG TTT AAT ATT AAG CCT AAT TTT GCA TTA GGT TAC TCA ATG GGT 1440
Asp Glu Phe Asn Ile Lys Pro Asn Phe Ala Leu Gly Tyr Ser Met Gly
465                 470                 475                 480
GAA GCA TCA ATG TGG GCA AGC TTA GGC GTA TGG CAA AAC CCG CAT GCG 1488
Glu Ala Ser Met Trp Ala Ser Leu Gly Val Trp Gln Asn Pro His Ala
                485                 490                 495
CTG ATC AGC AAA ACC CAA ACC GAC CCG CTA TTT ACT TCT GCT ATT TCC 1536
Leu Ile Ser Lys Thr Gln Thr Asp Pro Leu Phe Thr Ser Ala Ile Ser
            500                 505                 510
GGC AAA TTG ACC GCG GTT AGA CAA GCT TGG CAG CTT GAT GAT ACC GCA 1584
Gly Lys Leu Thr Ala Val Arg Gln Ala Trp Gln Leu Asp Asp Thr Ala
        515                 520                 525
GCG GAA ATC CAG TGG AAT AGC TTT GTG GTT AGA AGT GAA GCA GCG CCG 1632
Ala Glu Ile Gln Trp Asn Ser Phe Val Val Arg Ser Glu Ala Ala Pro
    530                 535                 540

ATT GAA GCC TTG CTA AAA GAT TAC CCA CAC GCT TAC CTC GCG ATT ATT 1680
Ile Glu Ala Leu Leu Lys Asp Tyr Pro His Ala Tyr Leu Ala Ile Ile
545                 550                 555                 560
CAA GGG GAT ACC TGC GTA ATC GCT GGC TGT GAA ATC CAA TGT AAA GCG 1728
Gln Gly Asp Thr Cys Val Ile Ala Gly Cys Glu Ile Gln Cys Lys Ala
                565                 570                 575
CTA CTT GCA GCA CTG GGT AAA CGC GGT ATT GCA GCT AAT CGT GTA ACG 1776
Leu Leu Ala Ala Leu Gly Lys Arg Gly Ile Ala Ala Asn Arg Val Thr
            580                 585                 590
GCG ATG CAT ACG CAG CCT GCG ATG CAA GAG CAT CAA AAT GTG ATG GAT 1824
Ala Met His Thr Gln Pro Ala Met Gln Glu His Gln Asn Val Met Asp
        595                 600                 605
TTT TAT CTG CAA CCG TTA AAA GCA GAG CTT CCT AGT GAA ATA AGC TTT 1872
Phe Tyr Leu Gln Pro Leu Lys Ala Glu Leu Pro Ser Glu Ile Ser Phe
    610                 615                 620
ATC AGC GCC GCT GAT TTA ACT GCC AAG CAA ACG GTG AGT GAG CAA GCA 1920
Ile Ser Ala Ala Asp Leu Thr Ala Lys Gln Thr Val Ser Glu Gln Ala
625                 630                 635                 640
CTT AGC AGC CAA GTC GTT GCT CAG TCT ATT GCC GAC ACC TTC TGC CAA 1968
Leu Ser Ser Gln Val Val Ala Gln Ser Ile Ala Asp Thr Phe Cys Gln
                645                 650                 655
ACC TTG GAC TTT ACC GCG CTA GTA CAT CAC GCC CAA CAT CAA GGC GCT 2016
Thr Leu Asp Phe Thr Ala Leu Val His His Ala Gln His Gln Gly Ala
            660                 665                 670
AAG CTG TTT GTT GAA ATT GGC GCG GAT AGA CAA AAC TGC ACC TTG ATA 2064
Lys Leu Phe Val Glu Ile Gly Ala Asp Arg Gln Asn Cys Thr Leu Ile
        675                 680                 685
GAC AAG ATT GTT AAA CAA GAT GGT GCC AGC AGT GTA CAA CAT CAA CCT 2112
Asp Lys Ile Val Lys Gln Asp Gly Ala Ser Ser Val Gln His Gln Pro
    690                 695                 700
TGT TGC ACA GTG CCT ATG AAC GCA AAA GGT AGC CAA GAT ATT ACC AGC 2160
Cys Cys Thr Val Pro Met Asn Ala Lys Gly Ser Gln Asp Ile Thr Ser
705                 710                 715                 720
GTG ATT AAA GCG CTT GGC CAA TTA ATT AGC CAT CAG GTG CCA TTA TCG 2208
Val Ile Lys Ala Leu Gly Gln Leu Ile Ser His Gln Val Pro Leu Ser
                725                 730                 735

GTG CAA CCA TTT ATT GAT GGA CTC AAG CGC GAG CTA ACA CTT TGC CAA 2256
Val Gln Pro Phe Ile Asp Gly Leu Lys Arg Glu Leu Thr Leu Cys Gln
            740                 745                 750
TTG ACC AGC CAA CAG CTG GCA GCA CAT GCA AAT GTT GAC AGC AAG TTT 2304
Leu Thr Ser Gln Gln Leu Ala Ala His Ala Asn Val Asp Ser Lys Phe
        755                 760                 765
GAG TCT AAC CAA GAC CAT TTA CTT CAA GGG GAA GTC 2340
Glu Ser Asn Gln Asp His Leu Leu Gln Gly Glu Val
    770                 775                 780

SEQ ID NO: 13
Sequence Length: 780
Sequence Type: Amino acid
Molecular Type: Peptide
Sequence
Val Glu Gln Thr Pro Lys Ala Ser Ala Met Pro Leu Arg Ile Ala Leu
1               5                   10                  15
Ile Leu Leu Pro Thr Pro Gln Phe Glu Val Asn Ser Val Asp Gln Ser
            20                  25                  30
Val Leu Ala Ser Tyr Gln Thr Leu Gln Pro Glu Leu Asn Ala Leu Leu
        35                  40                  45
Asn Ser Ala Pro Thr Pro Glu Met Leu Ser Ile Thr Ile Ser Asp Asp
    50                  55                  60
Ser Asp Ala Asn Ser Phe Glu Ser Gln Leu Asn Ala Ala Thr Asn Ala
65                  70                  75                  80
Ile Asn Asn Gly Tyr Ile Val Lys Leu Ala Thr Ala Thr His Ala Leu
                85                  90                  95
Leu Met Leu Pro Ala Leu Lys Ala Ala Gln Met Arg Ile His Pro His
            100                 105                 110
Ala Gln Leu Ala Ala Met Gln Gln Ala Lys Ser Thr Pro Met Ser Gln
        115                 120                 125
Val Ser Gly Glu Leu Lys Leu Gly Ala Asn Ala Leu Ser Leu Ala Gln
    130                 135                 140
Thr Asn Ala Leu Ser His Ala Leu Ser Gln Ala Lys Arg Asn Leu Thr
145                 150                 155                 160
Asp Val Ser Val Asn Glu Cys Phe Glu Asn Leu Lys Ser Glu Gln Gln
                165                 170                 175

Phe Thr Glu Val Tyr Ser Leu Ile Gln Gln Leu Ala Ser Arg Thr His
            180                 185                 190
Val Arg Lys Glu Val Asn Gln Gly Val Glu Leu Gly Pro Lys Gln Ala
        195                 200                 205
Lys Ser His Tyr Trp Phe Ser Glu Phe His Gln Asn Arg Val Ala Ala
    210                 215                 220
Ile Asn Phe Ile Asn Gly Gln Gln Ala Thr Ser Tyr Val Leu Thr Gln
225                 230                 235                 240
Gly Ser Gly Leu Leu Ala Ala Lys Ser Met Leu Asn Gln Gln Arg Leu
                245                 250                 255
Met Phe Ile Leu Pro Gly Asn Ser Gln Gln Gln Ile Thr Ala Ser Ile
            260                 265                 270
Thr Gln Leu Met Gln Gln Leu Glu Arg Leu Gln Val Thr Glu Val Asn
        275                 280                 285
Glu Leu Ser Leu Glu Cys Gln Leu Glu Leu Leu Ser Ile Met Tyr Asp
    290                 295                 300
Asn Leu Val Asn Ala Asp Lys Leu Thr Thr Arg Asp Ser Lys Pro Ala
305                 310                 315                 320
Tyr Gln Ala Val Ile Gln Ala Ser Ser Val Ser Ala Ala Lys Gln Glu
                325                 330                 335
Leu Ser Ala Leu Asn Asp Ala Leu Thr Ala Leu Phe Ala Glu Gln Thr
            340                 345                 350
Asn Ala Thr Ser Thr Asn Lys Gly Leu Ile Gln Tyr Lys Thr Pro Ala
        355                 360                 365
Gly Ser Tyr Leu Thr Leu Thr Pro Leu Gly Ser Asn Asn Asp Asn Ala
    370                 375                 380
Gln Ala Gly Leu Ala Phe Val Tyr Pro Gly Val Gly Thr Val Tyr Ala
385                 390                 395                 400
Asp Met Leu Asn Glu Leu His Gln Tyr Phe Pro Ala Leu Tyr Ala Lys
                405                 410                 415
Leu Glu Arg Glu Gly Asp Leu Lys Ala Met Leu Gln Ala Glu Asp Ile
            420                 425                 430
Tyr His Leu Asp Pro Lys His Ala Ala Gln Met Ser Leu Gly Asp Leu
        435                 440                 445
Ala Ile Ala Gly Val Gly Ser Ser Tyr Leu Leu Thr Gln Leu Leu Thr
    450                 455                 460

Asp Glu Phe Asn Ile Lys Pro Asn Phe Ala Leu Gly Tyr Ser Met Gly
465                 470                 475                 480
Glu Ala Ser Met Trp Ala Ser Leu Gly Val Trp Gln Asn Pro His Ala
                485                 490                 495
Leu Ile Ser Lys Thr Gln Thr Asp Pro Leu Phe Thr Ser Ala Ile Ser
            500                 505                 510
Gly Lys Leu Thr Ala Val Arg Gln Ala Trp Gln Leu Asp Asp Thr Ala
        515                 520                 525
Ala Glu Ile Gln Trp Asn Ser Phe Val Val Arg Ser Glu Ala Ala Pro
    530                 535                 540
Ile Glu Ala Leu Leu Lys Asp Tyr Pro His Ala Tyr Leu Ala Ile Ile
545                 550                 555                 560
Gln Gly Asp Thr Cys Val Ile Ala Gly Cys Glu Ile Gln Cys Lys Ala
                565                 570                 575
Leu Leu Ala Ala Leu Gly Lys Arg Gly Ile Ala Ala Asn Arg Val Thr
            580                 585                 590
Ala Met His Thr Gln Pro Ala Met Gln Glu His Gln Asn Val Met Asp
        595                 600                 605
Phe Tyr Leu Gln Pro Leu Lys Ala Glu Leu Pro Ser Glu Ile Ser Phe
    610                 615                 620
Ile Ser Ala Ala Asp Leu Thr Ala Lys Gln Thr Val Ser Glu Gln Ala
625                 630                 635                 640
Leu Ser Ser Gln Val Val Ala Gln Ser Ile Ala Asp Thr Phe Cys Gln
                645                 650                 655
Thr Leu Asp Phe Thr Ala Leu Val His His Ala Gln His Gln Gly Ala
            660                 665                 670
Lys Leu Phe Val Glu Ile Gly Ala Asp Arg Gln Asn Cys Thr Leu Ile
        675                 680                 685
Asp Lys Ile Val Lys Gln Asp Gly Ala Ser Ser Val Gln His Gln Pro
    690                 695                 700
Cys Cys Thr Val Pro Met Asn Ala Lys Gly Ser Gln Asp Ile Thr Ser
705                 710                 715                 720
Val Ile Lys Ala Leu Gly Gln Leu Ile Ser His Gln Val Pro Leu Ser
                725                 730                 735
Val Gln Pro Phe Ile Asp Gly Leu Lys Arg Glu Leu Thr Leu Cys Gln
            740                 745                 750

Leu Thr Ser Gln Gln Leu Ala Ala His Ala Asn Val Asp Ser Lys Phe
        755                 760                 765
Glu Ser Asn Gln Asp His Leu Leu Gln Gly Glu Val
    770                 775                 780

SEQ ID NO: 14
Sequence Length: 6012
Sequence Type: Nucleic acid
Strandedness: Double strand
Topology: Linear
Molecular Type: Genomic DNA
Source: Shewanella putrefaciens SCRC-2874 (FERM BP-1625)
Sequence
ATG TCA TTA CCA GAC AAT GCT TCT AAC CAC CTT TCT GCC AAC CAG AAA 48
Met Ser Leu Pro Asp Asn Ala Ser Asn His Leu Ser Ala Asn Gln Lys
1               5                   10                  15
GGC GCA TCT CAG GCA AGT AAA ACC AGT AAG CAA AGC AAA ATC GCC ATT 96
Gly Ala Ser Gln Ala Ser Lys Thr Ser Lys Gln Ser Lys Ile Ala Ile
            20                  25                  30
GTC GGT TTA GCC ACT CTG TAT CCA GAC GCT AAA ACC CCG CAA GAA TTT 144
Val Gly Leu Ala Thr Leu Tyr Pro Asp Ala Lys Thr Pro Gln Glu Phe
        35                  40                  45
TGG CAG AAT TTG CTG GAT AAA CGC GAC TCT CGC AGC ACC TTA ACT AAC 192
Trp Gln Asn Leu Leu Asp Lys Arg Asp Ser Arg Ser Thr Leu Thr Asn
    50                  55                  60
GAA AAA CTC GGC GCT AAC AGC CAA GAT TAT CAA GGT GTG CAA GGC CAA 240
Glu Lys Leu Gly Ala Asn Ser Gln Asp Tyr Gln Gly Val Gln Gly Gln
65                  70                  75                  80
TCT GAC CGT TTT TAT TGT AAT AAA GGC GGC TAC ATT GAG AAC TTC AGC 288
Ser Asp Arg Phe Tyr Cys Asn Lys Gly Gly Tyr Ile Glu Asn Phe Ser
                85                  90                  95
TTT AAT GCT GCA GGC TAC AAA TTG CCG GAG CAA AGC TTA AAT GGC TTG 336
Phe Asn Ala Ala Gly Tyr Lys Leu Pro Glu Gln Ser Leu Asn Gly Leu
            100                 105                 110
GAC GAC AGC TTC CTT TGG GCG CTC GAT ACT AGC CGT AAC GCA CTA ATT 384
Asp Asp Ser Phe Leu Trp Ala Leu Asp Thr Ser Arg Asn Ala Leu Ile
        115                 120                 125

GAT GCT GGT ATT GAT ATC AAC GGC GCT GAT TTA AGC CGC GCA GGT GTA 432
Asp Ala Gly Ile Asp Ile Asn Gly Ala Asp Leu Ser Arg Ala Gly Val
    130                 135                 140
GTC ATG GGC GCG CTG TCG TTC CCA ACT ACC CGC TCA AAC GAT CTG TTT 480
Val Met Gly Ala Leu Ser Phe Pro Thr Thr Arg Ser Asn Asp Leu Phe
145                 150                 155                 160
TTG CCA ATT TAT CAC AGC GCC GTT GAA AAA GCC CTG CAA GAT AAA CTA 528
Leu Pro Ile Tyr His Ser Ala Val Glu Lys Ala Leu Gln Asp Lys Leu
                165                 170                 175
GGC GTA AAG GCA TTT AAG CTA AGC CCA ACT AAT GCT CAT ACC GCT CGC 576
Gly Val Lys Ala Phe Lys Leu Ser Pro Thr Asn Ala His Thr Ala Arg
            180                 185                 190
GCG GCA AAT GAG AGC AGC CTA AAT GCA GCC AAT GGT GCC ATT GCC CAT 624
Ala Ala Asn Glu Ser Ser Leu Asn Ala Ala Asn Gly Ala Ile Ala His
        195                 200                 205
AAC AGC TCA AAA GTG GTG GCC GAT GCA CTT GGC CTT GGC GGC GCA CAA 672
Asn Ser Ser Lys Val Val Ala Asp Ala Leu Gly Leu Gly Gly Ala Gln
    210                 215                 220
CTA AGC CTA GAT GCT GCC TGT GCT AGT TCG GTT TAC TCA TTA AAG CTT 720
Leu Ser Leu Asp Ala Ala Cys Ala Ser Ser Val Tyr Ser Leu Lys Leu
225                 230                 235                 240
GCC TGC GAT TAC CTA AGC ACT GGC AAA GCC GAT ATC ATG CTA GCA GGC 768
Ala Cys Asp Tyr Leu Ser Thr Gly Lys Ala Asp Ile Met Leu Ala Gly
                245                 250                 255
GCA GTA TCT GGC GCG GAT CCT TTC TTT ATT AAT ATG GGA TTC TCA ATC 816
Ala Val Ser Gly Ala Asp Pro Phe Phe Ile Asn Met Gly Phe Ser Ile
            260                 265                 270
TTC CAC GCC TAC CCA GAC CAT GGT ATC TCA GTA CCG TTT GAT GCC AGC 864
Phe His Ala Tyr Pro Asp His Gly Ile Ser Val Pro Phe Asp Ala Ser
        275                 280                 285
AGT AAA GGT TTG TTT GCT GGC GAA GGC GCT GGC GTA TTA GTG CTT AAA 912
Ser Lys Gly Leu Phe Ala Gly Glu Gly Ala Gly Val Leu Val Leu Lys
    290                 295                 300
CGT CTT GAA GAT GCC GAG CGC GAC AAT GAC AAA ATC TAT GCG GTT GTT 960
Arg Leu Glu Asp Ala Glu Arg Asp Asn Asp Lys Ile Tyr Ala Val Val
305                 310                 315                 320

AGC GGC GTA GGT CTA TCA AAC GAC GGT AAA GGC CAG TTT GTA TTA AGC 1008
Ser Gly Val Gly Leu Ser Asn Asp Gly Lys Gly Gln Phe Val Leu Ser
                325                 330                 335
CCT AAT CCA AAA GGT CAG GTG AAG GCC TTT GAA CGT GCT TAT GCT GCC 1056
Pro Asn Pro Lys Gly Gln Val Lys Ala Phe Glu Arg Ala Tyr Ala Ala
            340                 345                 350
AGT GAC ATT GAG CCA AAA GAC ATT GAA GTG ATT GAG TGC CAC GCA ACA 1104
Ser Asp Ile Glu Pro Lys Asp Ile Glu Val Ile Glu Cys His Ala Thr
        355                 360                 365
GGC ACA CCG CTT GGC GAT AAA ATT GAG CTC ACT TCA ATG GAA ACC TTC 1152
Gly Thr Pro Leu Gly Asp Lys Ile Glu Leu Thr Ser Met Glu Thr Phe
    370                 375                 380
TTT GAA GAC AAG CTG CAA GGC ACC GAT GCA CCG TTA ATT GGC TCA GCT 1200
Phe Glu Asp Lys Leu Gln Gly Thr Asp Ala Pro Leu Ile Gly Ser Ala
385                 390                 395                 400
AAG TCT AAC TTA GGC CAC CTA TTA ACT GCA GCG CAT GCG GGG ATC ATG 1248
Lys Ser Asn Leu Gly His Leu Leu Thr Ala Ala His Ala Gly Ile Met
                405                 410                 415
AAG ATG ATC TTC GCC ATG AAA GAA GGT TAC CTG CCG CCA AGT ATC AAT 1296
Lys Met Ile Phe Ala Met Lys Glu Gly Tyr Leu Pro Pro Ser Ile Asn
            420                 425                 430
ATT AGT GAT GCT ATC GCT TCG CCG AAA AAA CTC TTC GGT AAA CCA ACC 1344
Ile Ser Asp Ala Ile Ala Ser Pro Lys Lys Leu Phe Gly Lys Pro Thr
        435                 440                 445
CTG CCT AGC ATG GTT CAA GGC TGG CCA GAT AAG CCA TCG AAT AAT CAT 1392
Leu Pro Ser Met Val Gln Gly Trp Pro Asp Lys Pro Ser Asn Asn His
    450                 455                 460
TTT GGT GTA AGA ACC CGT CAC GCA GGC GTA TCG GTA TTT GGC TTT GGT 1440
Phe Gly Val Arg Thr Arg His Ala Gly Val Ser Val Phe Gly Phe Gly
465                 470                 475                 480
GGC TGT AAC GCC CAT CTG TTG CTT GAG TCA TAC AAC GGC AAA GGA ACA 1488
Gly Cys Asn Ala His Leu Leu Leu Glu Ser Tyr Asn Gly Lys Gly Thr
                485                 490                 495
GTA AAG GCA GAA GCC ACT CAA GTA CCG CGT CAA GCT GAG CCG CTA AAA 1536
Val Lys Ala Glu Ala Thr Gln Val Pro Arg Gln Ala Glu Pro Leu Lys
            500                 505                 510

GTG GTT GGC CTT GCC TCG CAC TTT GGG CCT CTT AGC AGC ATT AAT GCA 1584
Val Val Gly Leu Ala Ser His Phe Gly Pro Leu Ser Ser Ile Asn Ala
        515                 520                 525
CTC AAC AAT GCT GTG ACC CAA GAT GGG AAT GGC TTT ATC GAA CTG CCG 1632
Leu Asn Asn Ala Val Thr Gln Asp Gly Asn Gly Phe Ile Glu Leu Pro
    530                 535                 540
AAA AAG CGC TGG AAA GGC CTT GAA AAG CAC AGT GAA CTG TTA GCT GAA 1680
Lys Lys Arg Trp Lys Gly Leu Glu Lys His Ser Glu Leu Leu Ala Glu
545                 550                 555                 560
TTT GGC TTA GCA TCT GCG CCA AAA GGT GCT TAT GTT GAT AAC TTC GAG 1728
Phe Gly Leu Ala Ser Ala Pro Lys Gly Ala Tyr Val Asp Asn Phe Glu
                565                 570                 575
CTG GAC TTT TTA CGC TTT AAA CTG CCG CCA AAC GAA GAT GAC CGT TTG 1776
Leu Asp Phe Leu Arg Phe Lys Leu Pro Pro Asn Glu Asp Asp Arg Leu
            580                 585                 590
ATC TCA CAG CAG CTA ATG CTA ATG CGA GTA ACA GAC GAA GCC ATT CGT 1824
Ile Ser Gln Gln Leu Met Leu Met Arg Val Thr As[?] Glu Ala Ile Arg
595 600 605
GAT GCC AAG CTT GAG CCG GGG CAA AAA GTA GCT G[?][?] TTA G[?][?] G[?]A ATG 1872
Asp Ala Lys Leu Glu Pro Gly Gln Lys Val Ala Va[?] [?]eu Val Ala Me[?]
610 615 62[?]
GAA ACT GAG CTT GAA CTG CAT CAG TTC CGC GGC CGG GTT AAC TTG CAT 1920
Glu Thr Glu Leu Glu Leu His Gln Phe Arg Gly Arg Val Asn Leu His
625 630 635 640
ACT CAA TTA GCG CAA AGT CTT GCC GCC ATG GGC GTG AGT TTA TCA ACG 1968
Thr Gln Leu Ala Gln Ser Leu Ala Ala Met Gly Val Ser Leu Ser Thr
645 650 655
GAT GAA TAC CAA GCG CTT GAA GCC ATC GCC ATG GAC AGC GTG CTT GAT 2016
Asp Glu Tyr Gln Ala Leu Glu Ala Ile Ala Met Asp Ser Val Leu Asp
660 665 670
GCT GCC AAG CTC AAT CAG TAC ACC AGC TTT ATT GGT AAT ATT ATG GCG 2064
Ala Ala Lys Leu Asn Gln Tyr Thr Ser Phe Ile Gly Asn Ile Met Ala
675 680 685
TCA CGC GTG GCG TCA CTA TGG GAC TTT AAT GGC CCA GCC TTC ACT ATT 2112
Ser Arg Val Ala Ser Leu Trp Asp Phe Asn Gly Pro Ala Phe Thr Ile
690 695 700

TCA GCA GCA GAG CAA TCT GTG AGC CGC TGT ATC GAT GTG GCG CAA AAC 2160
Ser Ala Ala Glu Gln Ser Val Ser Arg Cys Ile Asp Val Ala Gln Asn
705 710 715 720
CTC ATC ATG GAG GAT AAC CTA GAT GCG GTG GTG ATT GCA GCG GTC GAT 2208
Leu Ile Met Glu Asp Asn Leu Asp Ala Val Val Ile Ala Ala Val Asp
725 730 735
CTC TCT GGT AGC TTT GAG CAA GTC ATT CTT AAA AAT GCC ATT GCA CCT 2256
Leu Ser Gly Ser Phe Glu Gln Val Ile Leu Lys Asn Ala Ile Ala Pro
740 745 750
GTA GCC ATT GAG CCA AAC CTC GAA GCA AGC CTT AAT CCA ACA TCA GCA 2304
Val Ala Ile Glu Pro Asn Leu Glu Ala Ser Leu Asn Pro Thr Ser Ala
755 760 765
AGC TGG AAT GTC GGT GAA GGT GCT GGC GCG GTC GTG CTT GTT AAA AAT 2352
Ser Trp Asn Val Gly Glu Gly Ala Gly Ala Val Val Leu Val Lys Asn
770 775 780
GAA GCT ACA TCG GGC TGC TCA TAC GGC CAA ATT GAT GCA CTT GGC TTT 2400
Glu Ala Thr Ser Gly Cys Ser Tyr Gly Gln Ile Asp Ala Leu Gly Phe
785 790 795 800
GCT AAA ACT GCC GAA ACA GCG TTG GCT ACC GAC AAG CTA CTG AGC CAA 2448
Ala Lys Thr Ala Glu Thr Ala Leu Ala Thr Asp Lys Leu Leu Ser Gln
805 810 815
ACT GCC ACA GAC TTT AAT AAG GTT AAA GTG ATT GAA ACT ATG GCA GCG 2496
Thr Ala Thr Asp Phe Asn Lys Val Lys Val Ile Glu Thr Met Ala Ala
820 825 830
CCT GCT AGC CAA ATT CAA TTA GCG CCA ATA GTT AGC TCT CAA GTG ACT 2544
Pro Ala Ser Gln Ile Gln Leu Ala Pro Ile Val Ser Ser Gln Val Thr
835 840 845
CAC ACT GCT GCA GAG CAG CGT GTT GGT CAC TGC TTT GCT GCA GCG GGT 2592
His Thr Ala Ala Glu Gln Arg Val Gly His Cys Phe Ala Ala Ala Gly
850 855 860
ATG GCA AGC CTA TTA CAC GGC TTA CTT AAC TTA AAT ACT GTA GCC CAA 2640
Met Ala Ser Leu Leu His Gly Leu Leu Asn Leu Asn Thr Val Ala Gln
865 870 875 880
ACC AAT AAA GCC AAT TGC GCG CTT ATC AAC AAT ATC AGT GAA AAC CAA 2688
Thr Asn Lys Ala Asn Cys Ala Leu Ile Asn Asn Ile Ser Glu Asn Gln
885 890 895

TTA TCA CAG CTG TTG ATT AGC CAA ACA GCG AGC GAA CAA CAA GCA TTA 2736
Leu Ser Gln Leu Leu Ile Ser Gln Thr Ala Ser Glu Gln Gln Ala Leu
900 905 910
ACC GCG CGT TTA AGC AAT GAG CTT AAA TCC GAT GCT AAA CAC CAA CTG 2784
Thr Ala Arg Leu Ser Asn Glu Leu Lys Ser Asp Ala Lys His Gln Leu
915 920 925
GTT AAG CAA GTC ACC TTA GGT GGC CGT GAT ATC TAC CAG CAT ATT GTT 2832
Val Lys Gln Val Thr Leu Gly Gly Arg Asp Ile Tyr Gln His Ile Val
930 935 940
GAT ACA CCG CTT GCA AGC CTT GAA AGC ATT ACT CAG AAA TTG GCG CAA 2880
Asp Thr Pro Leu Ala Ser Leu Glu Ser Ile Thr Gln Lys Leu Ala Gln
945 950 955 960
GCG ACA GCA TCG ACA GTG GTC AAC CAA GTT AAA CCT ATT AAG GCC GCT 2928
Ala Thr Ala Ser Thr Val Val Asn Gln Val Lys Pro Ile Lys Ala Ala
965 970 975
GGC TCA GTC GAA ATG GCT AAC TCA TTC GAA ACG GAA AGC TCA GCA GAG 2976
Gly Ser Val Glu Met Ala Asn Ser Phe Glu Thr Glu Ser Ser Ala Glu
980 985 990
CCA CAA ATA ACA ATT GCA GCA CAA CAG ACT GCA AAC ATT GGC GTC ACC 3024
Pro Gln Ile Thr Ile Ala Ala Gln Gln Thr Ala Asn Ile Gly Val Thr
995 1000 1005
GCT CAG GCA ACC AAA CGT GAA TTA GGT ACC CCA CCA ATG ACA ACA AAT 3072
Ala Gln Ala Thr Lys Arg Glu Leu Gly Thr Pro Pro Met Thr Thr Asn
1010 1015 1020
ACC ATT GCT AAT ACA GCA AAT AAT TTA GAC AAG ACT CTT GAG ACT GTT 3120
Thr Ile Ala Asn Thr Ala Asn Asn Leu Asp Lys Thr Leu Glu Thr Val
1025 1030 1035 1040
GCT GGC AAT ACT GTT GCT AGC AAG GTT GGC TCT GGC GAC ATA GTC AAT 3168
Ala Gly Asn Thr Val Ala Ser Lys Val Gly Ser Gly Asp Ile Val Asn
1045 1050 1055
TTT CAA CAG AAC CAA CAA TTG GCT CAA CAA GCT CAC CTC GCC TTT CTT 3216
Phe Gln Gln Asn Gln Gln Leu Ala Gln Gln Ala His Leu Ala Phe Leu
1060 1065 1070
GAA AGC CGC AGT GCG GGT ATG AAG GTG GCT GAT GCT TTA TTG AAG CAA 3264
Glu Ser Arg Ser Ala Gly Met Lys Val Ala Asp Ala Leu Leu Lys Gln
1075 1080 1085

CAG CTA GCT CAA GTA ACA GGC CAA ACT ATC GAT AAT CAG GCC CTC GAT 3312
Gln Leu Ala Gln Val Thr Gly Gln Thr Ile Asp Asn Gln Ala Leu Asp
1090 1095 1100
ACT CAA GCC GTC GAT ACT CAA ACA AGC GAG AAT GTA GCG ATT GCC GCA 3360
Thr Gln Ala Val Asp Thr Gln Thr Ser Glu Asn Val Ala Ile Ala Ala
1105 1110 1115 1120
GAA TCA CCA GTT CAA GTT ACA ACA CCT GTT CAA GTT ACA ACA CCT GTT 3408
Glu Ser Pro Val Gln Val Thr Thr Pro Val Gln Val Thr Thr Pro Val
1125 1130 1135
CAA ATC AGT GTT GTG GAG TTA AAA CCA GAT CAC GCT AAT GTG CCA CCA 3456
Gln Ile Ser Val Val Glu Leu Lys Pro Asp His Ala Asn Val Pro Pro
1140 1145 1150
TAC ACG CCG CCA GTG CCT GCA TTA AAG CCG TGT ATC TGG AAC TAT GCC 3504
Tyr Thr Pro Pro Val Pro Ala Leu Lys Pro Cys Ile Trp Asn Tyr Ala
1155 1160 1165
GAT TTA GTT GAG TAC GCA GAA GGC GAT ATC GCC AAG GTA TTT GGC AGT 3552
Asp Leu Val Glu Tyr Ala Glu Gly Asp Ile Ala Lys Val Phe Gly Ser
1170 1175 1180
GAT TAT GCC ATT ATC GAC AGC TAC TCG CGC CGC GTA CGT CTA CCG ACC 3600
Asp Tyr Ala Ile Ile Asp Ser Tyr Ser Arg Arg Val Arg Leu Pro Thr
1185 1190 1195 1200
ACT GAC TAC CTG TTG GTA TCG CGC GTG ACC AAA CTT GAT GCG ACC ATC 3648
Thr Asp Tyr Leu Leu Val Ser Arg Val Thr Lys Leu Asp Ala Thr Ile
1205 1210 1215
AAT CAA TTT AAG CCA TGC TCA ATG ACC ACT GAG TAC GAC ATC CCT GTT 3696
Asn Gln Phe Lys Pro Cys Ser Met Thr Thr Glu Tyr Asp Ile Pro Val
1220 1225 1230
GAT GCG CCG TAC TTA GTA GAC GGA CAA ATC CCT TGG GCG GTA GCA GTA 3744
Asp Ala Pro Tyr Leu Val Asp Gly Gln Ile Pro Trp Ala Val Ala Val
1235 1240 1245
GAA TCA GGC CAA TGT GAC TTG ATG CTT ATT AGC TAT CTC GGT ATC GAC 3792
Glu Ser Gly Gln Cys Asp Leu Met Leu Ile Ser Tyr Leu Gly Ile Asp
1250 1255 1260
TTT GAG AAC AAA GGC GAG CGG GTT TAT CGA CTA CTC GAT TGT ACC CTC 3840
Phe Glu Asn Lys Gly Glu Arg Val Tyr Arg Leu Leu Asp Cys Thr Leu
1265 1270 1275 1280

ACC TTC CTA GGC GAC TTG CCA CGT GGC GGA GAT ACC CTA CGT TAC GAC 3888
Thr Phe Leu Gly Asp Leu Pro Arg Gly Gly Asp Thr Leu Arg Tyr Asp
1285 1290 1295
ATT AAG ATC AAT AAC TAT GCT CGC AAC GGC GAC ACC CTG CTG TTC TTC 3936
Ile Lys Ile Asn Asn Tyr Ala Arg Asn Gly Asp Thr Leu Leu Phe Phe
1300 1305 1310
TTC TCG TAT GAG TGT TTT GTT GGC GAC AAG ATG ATC CTC AAG ATG GAT 3984
Phe Ser Tyr Glu Cys Phe Val Gly Asp Lys Met Ile Leu Lys Met Asp
1315 1320 1325
GGC GGC TGC GCT GGC TTC TTC ACT GAT GAA GAG CTT GCC GAC GGT AAA 4032
Gly Gly Cys Ala Gly Phe Phe Thr Asp Glu Glu Leu Ala Asp Gly Lys
1330 1335 1340
GGC GTG ATT CGC ACA GAA GAA GAG ATT AAA GCT CGC AGC CTA GTG CAA 4080
Gly Val Ile Arg Thr Glu Glu Glu Ile Lys Ala Arg Ser Leu Val Gln
1345 1350 1355 1360
AAG CAA CGC TTT AAT CCG TTA CTA GAT TGT CCT AAA ACC CAA TTT AGT 4128
Lys Gln Arg Phe Asn Pro Leu Leu Asp Cys Pro Lys Thr Gln Phe Ser
1365 1370 1375
TAT GGT GAT ATT CAT AAG CTA TTA ACT GCT GAT ATT GAG GGT TGT TTT 4176
Tyr Gly Asp Ile His Lys Leu Leu Thr Ala Asp Ile Glu Gly Cys Phe
1380 1385 1390
GGC CCA AGC CAC AGT GGC GTC CAC CAG CCG TCA CTT TGT TTC GCA TCT 4224
Gly Pro Ser His Ser Gly Val His Gln Pro Ser Leu Cys Phe Ala Ser
1395 1400 1405
GAA AAA TTC TTG ATG ATT GAA CAA GTC AGC AAG GTT GAT CGC ACT GGC 4272
Glu Lys Phe Leu Met Ile Glu Gln Val Ser Lys Val Asp Arg Thr Gly
1410 1415 1420
GGT ACT TGG GGA CTT GGC TTA ATT GAG GGT CAT AAG CAG CTT GAA GCA 4320
Gly Thr Trp Gly Leu Gly Leu Ile Glu Gly His Lys Gln Leu Glu Ala
1425 1430 1435 1440
GAC CAC TGG TAC TTC CCA TGT CAT TTC AAG GGC GAC CAA GTG ATG GCT 4368
Asp His Trp Tyr Phe Pro Cys His Phe Lys Gly Asp Gln Val Met Ala
1445 1450 1455
GGC TCG CTA ATG GCT GAA GGT TGT GGC CAG TTA TTG CAG TTC TAT ATG 4416
Gly Ser Leu Met Ala Glu Gly Cys Gly Gln Leu Leu Gln Phe Tyr Met
1460 1465 1470

CTG CAC CTT GGT ATG CAT ACC CAA ACT AAA AAT GGT CGT TTC CAA CCT 4464
Leu His Leu Gly Met His Thr Gln Thr Lys Asn Gly Arg Phe Gln Pro
1475 1480 1485
CTT GAA AAC GCC TCA CAG CAA GTA CGC TGT CGC GGT CAA GTG CTG CCA 4512
Leu Glu Asn Ala Ser Gln Gln Val Arg Cys Arg Gly Gln Val Leu Pro
1490 1495 1500
CAA TCA GGC GTG CTA ACT TAC CGT ATG GAA GTG ACT GAA ATC GGT TTC 4560
Gln Ser Gly Val Leu Thr Tyr Arg Met Glu Val Thr Glu Ile Gly Phe
1505 1510 1515 1520
AGT CCA CGC CCA TAT GCT AAA GCT AAC ATC GAT ATC TTG CTT AAT GGC 4608
Ser Pro Arg Pro Tyr Ala Lys Ala Asn Ile Asp Ile Leu Leu Asn Gly
1525 1530 1535
AAA GCG GTA GTG GAT TTC CAA AAC CTA GGG GTG ATG ATA AAA GAG GAA 4656
Lys Ala Val Val Asp Phe Gln Asn Leu Gly Val Met Ile Lys Glu Glu
1540 1545 1550
GAT GAG TGT ACT CGT TAT CCA CTT TTG ACT GAA TCA ACA ACG GCT AGC 4704
Asp Glu Cys Thr Arg Tyr Pro Leu Leu Thr Glu Ser Thr Thr Ala Ser
1555 1560 1565
ACT GCA CAA GTA AAC GCT CAA ACA AGT GCG AAA AAG GTA TAC AAG CCA 4752
Thr Ala Gln Val Asn Ala Gln Thr Ser Ala Lys Lys Val Tyr Lys Pro
1570 1575 1580
GCA TCA GTC AAT GCG CCA TTA ATG GCA CAA ATT CCT GAT CTG ACT AAA 4800
Ala Ser Val Asn Ala Pro Leu Met Ala Gln Ile Pro Asp Leu Thr Lys
1585 1590 1595 1600
GAG CCA AAC AAG GGC GTT ATT CCG ATT TCC CAT GTT GAA GCA CCA ATT 4848
Glu Pro Asn Lys Gly Val Ile Pro Ile Ser His Val Glu Ala Pro Ile
1605 1610 1615
ACG CCA GAC TAC CCG AAC CGT GTA CCT GAT ACA GTG CCA TTC ACG CCG 4896
Thr Pro Asp Tyr Pro Asn Arg Val Pro Asp Thr Val Pro Phe Thr Pro
1620 1625 1630
TAT CAC ATG TTT GAG TTT GCT ACA GGC AAT ATC GAA AAC TGT TTC GGG 4944
Tyr His Met Phe Glu Phe Ala Thr Gly Asn Ile Glu Asn Cys Phe Gly
1635 1640 1645
CCA GAG TTC TCA ATC TAT CGC GGC ATG ATC CCA CCA CGT ACA CCA TGC 4992
Pro Glu Phe Ser Ile Tyr Arg Gly Met Ile Pro Pro Arg Thr Pro Cys
1650 1655 1660

GGT GAC TTA CAA GTG ACC ACA CGT GTG ATT GAA GTT AAC GGT AAG CGT 5040
Gly Asp Leu Gln Val Thr Thr Arg Val Ile Glu Val Asn Gly Lys Arg
1665 1670 1675 1680
GGC GAC TTT AAA AAG CCA TCA TCG TGT ATC GCT GAA TAT GAA GTG CCT 5088
Gly Asp Phe Lys Lys Pro Ser Ser Cys Ile Ala Glu Tyr Glu Val Pro
1685 1690 1695
GCA GAT GCG TGG TAT TTC GAT AAA AAC AGC CAC GGC GCA GTG ATG CCA 5136
Ala Asp Ala Trp Tyr Phe Asp Lys Asn Ser His Gly Ala Val Met Pro
1700 1705 1710
TAT TCA ATT TTA ATG GAG ATC TCA CTG CAA CCT AAC GGC TTT ATC TCA 5184
Tyr Ser Ile Leu Met Glu Ile Ser Leu Gln Pro Asn Gly Phe Ile Ser
1715 1720 1725
GGT TAC ATG GGC ACA ACC CTA GGC TTC CCT GGC CTT GAG CTG TTC TTC 5232
Gly Tyr Met Gly Thr Thr Leu Gly Phe Pro Gly Leu Glu Leu Phe Phe
1730 1735 1740
CGT AAC TTA GAC GGT AGC GGT GAG TTA CTA CGT GAA GTA GAT TTA CGT 5280
Arg Asn Leu Asp Gly Ser Gly Glu Leu Leu Arg Glu Val Asp Leu Arg
1745 1750 1755 1760
GGT AAA ACC ATC CGT AAC GAC TCA CGT TTA TTA TCA ACA GTG ATG GCC 5328
Gly Lys Thr Ile Arg Asn Asp Ser Arg Leu Leu Ser Thr Val Met Ala
1765 1770 1775
GGC ACT AAC ATC ATC CAA AGC TTT AGC TTC GAG CTA AGC ACT GAC GGT 5376
Gly Thr Asn Ile Ile Gln Ser Phe Ser Phe Glu Leu Ser Thr Asp Gly
1780 1785 1790
GAG CCT TTC TAT CGC GGC ACT GCG GTA TTT GGC TAT TTT AAA GGT GAC 5424
Glu Pro Phe Tyr Arg Gly Thr Ala Val Phe Gly Tyr Phe Lys Gly Asp
1795 1800 1805
GCA CTT AAA GAT CAG CTA GGC CTA GAT AAC GGT AAA GTC ACT CAG CCA 5472
Ala Leu Lys Asp Gln Leu Gly Leu Asp Asn Gly Lys Val Thr Gln Pro
1810 1815 1820
TGG CAT GTA GCT AAC GGC GTT GCT GCA AGC ACT AAG GTG AAC CTG CTT 5520
Trp His Val Ala Asn Gly Val Ala Ala Ser Thr Lys Val Asn Leu Leu
1825 1830 1835 1840
GAT AAG AGC TGC CGT CAC TTT AAT GCG CCA GCT AAC CAG CCA CAC TAT 5568
Asp Lys Ser Cys Arg His Phe Asn Ala Pro Ala Asn Gln Pro His Tyr
1845 1850 1855

CGT CTA GCC GGT GGT CAG CTG AAC TTT ATC GAC AGT GTT GAA ATT GTT 5616
Arg Leu Ala Gly Gly Gln Leu Asn Phe Ile Asp Ser Val Glu Ile Val
1860 1865 1870
GAT AAT GGC GGC ACC GAA GGT TTA GGT TAC TTG TAT GCC GAG CGC ACC 5664
Asp Asn Gly Gly Thr Glu Gly Leu Gly Tyr Leu Tyr Ala Glu Arg Thr
1875 1880 1885
ATT GAC CCA AGT GAT TGG TTC TTC CAG TTC CAC TTC CAC CAA GAT CCG 5712
Ile Asp Pro Ser Asp Trp Phe Phe Gln Phe His Phe His Gln Asp Pro
1890 1895 1900
GTT ATG CCA GGC TCC TTA GGT GTT GAA GCA ATT ATT GAA ACC ATG CAA 5760
Val Met Pro Gly Ser Leu Gly Val Glu Ala Ile Ile Glu Thr Met Gln
1905 1910 1915 1920
GCT TAC GCT ATT AGT AAA GAC TTG GGC GCA GAT TTC AAA AAT CCT AAG 5808
Ala Tyr Ala Ile Ser Lys Asp Leu Gly Ala Asp Phe Lys Asn Pro Lys
1925 1930 1935
TTT GGT CAG ATT TTA TCG AAC ATC AAG TGG AAG TAT CGC GGT CAA ATC 5856
Phe Gly Gln Ile Leu Ser Asn Ile Lys Trp Lys Tyr Arg Gly Gln Ile
1940 1945 1950
AAT CCG CTG AAC AAG CAG ATG TCT ATG GAT GTC AGC ATT ACT TCA ATC 5904
Asn Pro Leu Asn Lys Gln Met Ser Met Asp Val Ser Ile Thr Ser Ile
1955 1960 1965
AAA GAT GAA GAC GGT AAG AAA GTC ATC ACA GGT AAT GCC AGC TTG AGT 5952
Lys Asp Glu Asp Gly Lys Lys Val Ile Thr Gly Asn Ala Ser Leu Ser
1970 1975 1980
AAA GAT GGT CTG CGC ATA TAC GAG GTC TTC GAT ATA GCT ATC AGC ATC 6000
Lys Asp Gly Leu Arg Ile Tyr Glu Val Phe Asp Ile Ala Ile Ser Ile
1985 1990 1995 2000
GAA GAA TCT GTA 6012
Glu Glu Ser Val

SEQ ID NO: 15
Sequence Length: 2004
Sequence Type: Amino acid
Molecular Type: Peptide
Sequence
Met Ser Leu Pro Asp Asn Ala Ser Asn His Leu Ser Ala Asn Gln Lys
1               5                   10                  15

Gly Ala Ser Gln Ala Ser Lys Thr Ser Lys Gln Ser Lys Ile Ala Ile
            20                  25                  30
Val Gly Leu Ala Thr Leu Tyr Pro Asp Ala Lys Thr Pro Gln Glu Phe
        35                  40                  45
Trp Gln Asn Leu Leu Asp Lys Arg Asp Ser Arg Ser Thr Leu Thr Asn
    50                  55                  60
Glu Lys Leu Gly Ala Asn Ser Gln Asp Tyr Gln Gly Val Gln Gly Gln
65                  70                  75                  80
Ser Asp Arg Phe Tyr Cys Asn Lys Gly Gly Tyr Ile Glu Asn Phe Ser
                85                  90                  95
Phe Asn Ala Ala Gly Tyr Lys Leu Pro Glu Gln Ser Leu Asn Gly Leu
            100                 105                 110
Asp Asp Ser Phe Leu Trp Ala Leu Asp Thr Ser Arg Asn Ala Leu Ile
        115                 120                 125
Asp Ala Gly Ile Asp Ile Asn Gly Ala Asp Leu Ser Arg Ala Gly Val
    130                 135                 140
Val Met Gly Ala Leu Ser Phe Pro Thr Thr Arg Ser Asn Asp Leu Phe
145                 150                 155                 160
Leu Pro Ile Tyr His Ser Ala Val Glu Lys Ala Leu Gln Asp Lys Leu
                165                 170                 175
Gly Val Lys Ala Phe Lys Leu Ser Pro Thr Asn Ala His Thr Ala Arg
            180                 185                 190
Ala Ala Asn Glu Ser Ser Leu Asn Ala Ala Asn Gly Ala Ile Ala His
        195                 200                 205
Asn Ser Ser Lys Val Val Ala Asp Ala Leu Gly Leu Gly Gly Ala Gln
    210                 215                 220
Leu Ser Leu Asp Ala Ala Cys Ala Ser Ser Val Tyr Ser Leu Lys Leu
225                 230                 235                 240
Ala Cys Asp Tyr Leu Ser Thr Gly Lys Ala Asp Ile Met Leu Ala Gly
                245                 250                 255
Ala Val Ser Gly Ala Asp Pro Phe Phe Ile Asn Met Gly Phe Ser Ile
            260                 265                 270
Phe His Ala Tyr Pro Asp His Gly Ile Ser Val Pro Phe Asp Ala Ser
        275                 280                 285
Ser Lys Gly Leu Phe Ala Gly Glu Gly Ala Gly Val Leu Val Leu Lys
    290                 295                 300

Arg Leu Glu Asp Ala Glu Arg Asp Asn Asp Lys Ile Tyr Ala Val Val
305                 310                 315                 320
Ser Gly Val Gly Leu Ser Asn Asp Gly Lys Gly Gln Phe Val Leu Ser
                325                 330                 335
Pro Asn Pro Lys Gly Gln Val Lys Ala Phe Glu Arg Ala Tyr Ala Ala
            340                 345                 350
Ser Asp Ile Glu Pro Lys Asp Ile Glu Val Ile Glu Cys His Ala Thr
        355                 360                 365
Gly Thr Pro Leu Gly Asp Lys Ile Glu Leu Thr Ser Met Glu Thr Phe
    370                 375                 380
Phe Glu Asp Lys Leu Gln Gly Thr Asp Ala Pro Leu Ile Gly Ser Ala
385                 390                 395                 400
Lys Ser Asn Leu Gly His Leu Leu Thr Ala Ala His Ala Gly Ile Met
                405                 410                 415
Lys Met Ile Phe Ala Met Lys Glu Gly Tyr Leu Pro Pro Ser Ile Asn
            420                 425                 430
Ile Ser Asp Ala Ile Ala Ser Pro Lys Lys Leu Phe Gly Lys Pro Thr
        435                 440                 445
Leu Pro Ser Met Val Gln Gly Trp Pro Asp Lys Pro Ser Asn Asn His
    450                 455                 460
Phe Gly Val Arg Thr Arg His Ala Gly Val Ser Val Phe Gly Phe Gly
465                 470                 475                 480
Gly Cys Asn Ala His Leu Leu Leu Glu Ser Tyr Asn Gly Lys Gly Thr
                485                 490                 495
Val Lys Ala Glu Ala Thr Gln Val Pro Arg Gln Ala Glu Pro Leu Lys
            500                 505                 510
Val Val Gly Leu Ala Ser His Phe Gly Pro Leu Ser Ser Ile Asn Ala
        515                 520                 525
Leu Asn Asn Ala Val Thr Gln Asp Gly Asn Gly Phe Ile Glu Leu Pro
    530                 535                 540
Lys Lys Arg Trp Lys Gly Leu Glu Lys His Ser Glu Leu Leu Ala Glu
545                 550                 555                 560
Phe Gly Leu Ala Ser Ala Pro Lys Gly Ala Tyr Val Asp Asn Phe Glu
                565                 570                 575
Leu Asp Phe Leu Arg Phe Lys Leu Pro Pro Asn Glu Asp Asp Arg Leu
            580                 585                 590

Ile Ser Gln Gln Leu Met Leu Met Arg Val Thr Asp Glu Ala Ile Arg
        595                 600                 605
Asp Ala Lys Leu Glu Pro Gly Gln Lys Val Ala Val Leu Val Ala Met
    610                 615                 620
Glu Thr Glu Leu Glu Leu His Gln Phe Arg Gly Arg Val Asn Leu His
625                 630                 635                 640
Thr Gln Leu Ala Gln Ser Leu Ala Ala Met Gly Val Ser Leu Ser Thr
                645                 650                 655
Asp Glu Tyr Gln Ala Leu Glu Ala Ile Ala Met Asp Ser Val Leu Asp
            660                 665                 670
Ala Ala Lys Leu Asn Gln Tyr Thr Ser Phe Ile Gly Asn Ile Met Ala
        675                 680                 685
Ser Arg Val Ala Ser Leu Trp Asp Phe Asn Gly Pro Ala Phe Thr Ile
    690                 695                 700
Ser Ala Ala Glu Gln Ser Val Ser Arg Cys Ile Asp Val Ala Gln Asn
705                 710                 715                 720
Leu Ile Met Glu Asp Asn Leu Asp Ala Val Val Ile Ala Ala Val Asp
                725                 730                 735
Leu Ser Gly Ser Phe Glu Gln Val Ile Leu Lys Asn Ala Ile Ala Pro
            740                 745                 750
Val Ala Ile Glu Pro Asn Leu Glu Ala Ser Leu Asn Pro Thr Ser Ala
        755                 760                 765
Ser Trp Asn Val Gly Glu Gly Ala Gly Ala Val Val Leu Val Lys Asn
    770                 775                 780
Glu Ala Thr Ser Gly Cys Ser Tyr Gly Gln Ile Asp Ala Leu Gly Phe
785                 790                 795                 800
Ala Lys Thr Ala Glu Thr Ala Leu Ala Thr Asp Lys Leu Leu Ser Gln
                805                 810                 815
Thr Ala Thr Asp Phe Asn Lys Val Lys Val Ile Glu Thr Met Ala Ala
            820                 825                 830
Pro Ala Ser Gln Ile Gln Leu Ala Pro Ile Val Ser Ser Gln Val Thr
        835                 840                 845
His Thr Ala Ala Glu Gln Arg Val Gly His Cys Phe Ala Ala Ala Gly
    850                 855                 860
Met Ala Ser Leu Leu His Gly Leu Leu Asn Leu Asn Thr Val Ala Gln
865                 870                 875                 880

Thr Asn Lys Ala Asn Cys Ala Leu Ile Asn Asn Ile Ser Glu Asn Gln
                885                 890                 895
Leu Ser Gln Leu Leu Ile Ser Gln Thr Ala Ser Glu Gln Gln Ala Leu
            900                 905                 910
Thr Ala Arg Leu Ser Asn Glu Leu Lys Ser Asp Ala Lys His Gln Leu
        915                 920                 925
Val Lys Gln Val Thr Leu Gly Gly Arg Asp Ile Tyr Gln His Ile Val
    930                 935                 940
Asp Thr Pro Leu Ala Ser Leu Glu Ser Ile Thr Gln Lys Leu Ala Gln
945                 950                 955                 960
Ala Thr Ala Ser Thr Val Val Asn Gln Val Lys Pro Ile Lys Ala Ala
                965                 970                 975
Gly Ser Val Glu Met Ala Asn Ser Phe Glu Thr Glu Ser Ser Ala Glu
            980                 985                 990
Pro Gln Ile Thr Ile Ala Ala Gln Gln Thr Ala Asn Ile Gly Val Thr
        995                 1000                1005
Ala Gln Ala Thr Lys Arg Glu Leu Gly Thr Pro Pro Met Thr Thr Asn
    1010                1015                1020
Thr Ile Ala Asn Thr Ala Asn Asn Leu Asp Lys Thr Leu Glu Thr Val
1025                1030                1035                1040
Ala Gly Asn Thr Val Ala Ser Lys Val Gly Ser Gly Asp Ile Val Asn
                1045                1050                1055
Phe Gln Gln Asn Gln Gln Leu Ala Gln Gln Ala His Leu Ala Phe Leu
            1060                1065                1070
Glu Ser Arg Ser Ala Gly Met Lys Val Ala Asp Ala Leu Leu Lys Gln
        1075                1080                1085
Gln Leu Ala Gln Val Thr Gly Gln Thr Ile Asp Asn Gln Ala Leu Asp
    1090                1095                1100
Thr Gln Ala Val Asp Thr Gln Thr Ser Glu Asn Val Ala Ile Ala Ala
1105                1110                1115                1120
Glu Ser Pro Val Gln Val Thr Thr Pro Val Gln Val Thr Thr Pro Val
                1125                1130                1135
Gln Ile Ser Val Val Glu Leu Lys Pro Asp His Ala Asn Val Pro Pro
            1140                1145                1150
Tyr Thr Pro Pro Val Pro Ala Leu Lys Pro Cys Ile Trp Asn Tyr Ala
        1155                1160                1165

Asp Leu Val Glu Tyr Ala Glu Gly Asp Ile Ala Lys Val Phe Gly Ser
    1170                1175                1180
Asp Tyr Ala Ile Ile Asp Ser Tyr Ser Arg Arg Val Arg Leu Pro Thr
1185                1190                1195                1200
Thr Asp Tyr Leu Leu Val Ser Arg Val Thr Lys Leu Asp Ala Thr Ile
                1205                1210                1215
Asn Gln Phe Lys Pro Cys Ser Met Thr Thr Glu Tyr Asp Ile Pro Val
            1220                1225                1230
Asp Ala Pro Tyr Leu Val Asp Gly Gln Ile Pro Trp Ala Val Ala Val
        1235                1240                1245
Glu Ser Gly Gln Cys Asp Leu Met Leu Ile Ser Tyr Leu Gly Ile Asp
    1250                1255                1260
Phe Glu Asn Lys Gly Glu Arg Val Tyr Arg Leu Leu Asp Cys Thr Leu
1265                1270                1275                1280
Thr Phe Leu Gly Asp Leu Pro Arg Gly Gly Asp Thr Leu Arg Tyr Asp
                1285                1290                1295
Ile Lys Ile Asn Asn Tyr Ala Arg Asn Gly Asp Thr Leu Leu Phe Phe
            1300                1305                1310
Phe Ser Tyr Glu Cys Phe Val Gly Asp Lys Met Ile Leu Lys Met Asp
        1315                1320                1325
Gly Gly Cys Ala Gly Phe Phe Thr Asp Glu Glu Leu Ala Asp Gly Lys
    1330                1335                1340
Gly Val Ile Arg Thr Glu Glu Glu Ile Lys Ala Arg Ser Leu Val Gln
1345                1350                1355                1360
Lys Gln Arg Phe Asn Pro Leu Leu Asp Cys Pro Lys Thr Gln Phe Ser
                1365                1370                1375
Tyr Gly Asp Ile His Lys Leu Leu Thr Ala Asp Ile Glu Gly Cys Phe
            1380                1385                1390
Gly Pro Ser His Ser Gly Val His Gln Pro Ser Leu Cys Phe Ala Ser
        1395                1400                1405
Glu Lys Phe Leu Met Ile Glu Gln Val Ser Lys Val Asp Arg Thr Gly
    1410                1415                1420
Gly Thr Trp Gly Leu Gly Leu Ile Glu Gly His Lys Gln Leu Glu Ala
1425                1430                1435                1440
Asp His Trp Tyr Phe Pro Cys His Phe Lys Gly Asp Gln Val Met Ala
                1445                1450                1455

Gly Ser Leu Met Ala Glu Gly Cys Gly Gln Leu Leu Gln Phe Tyr Met
            1460                1465                1470
Leu His Leu Gly Met His Thr Gln Thr Lys Asn Gly Arg Phe Gln Pro
        1475                1480                1485
Leu Glu Asn Ala Ser Gln Gln Val Arg Cys Arg Gly Gln Val Leu Pro
    1490                1495                1500
Gln Ser Gly Val Leu Thr Tyr Arg Met Glu Val Thr Glu Ile Gly Phe
1505                1510                1515                1520
Ser Pro Arg Pro Tyr Ala Lys Ala Asn Ile Asp Ile Leu Leu Asn Gly
                1525                1530                1535
Lys Ala Val Val Asp Phe Gln Asn Leu Gly Val Met Ile Lys Glu Glu
            1540                1545                1550
Asp Glu Cys Thr Arg Tyr Pro Leu Leu Thr Glu Ser Thr Thr Ala Ser
        1555                1560                1565
Thr Ala Gln Val Asn Ala Gln Thr Ser Ala Lys Lys Val Tyr Lys Pro
    1570                1575                1580
Ala Ser Val Asn Ala Pro Leu Met Ala Gln Ile Pro Asp Leu Thr Lys
1585                1590                1595                1600
Glu Pro Asn Lys Gly Val Ile Pro Ile Ser His Val Glu Ala Pro Ile
                1605                1610                1615
Thr Pro Asp Tyr Pro Asn Arg Val Pro Asp Thr Val Pro Phe Thr Pro
            1620                1625                1630
Tyr His Met Phe Glu Phe Ala Thr Gly Asn Ile Glu Asn Cys Phe Gly
        1635                1640                1645
Pro Glu Phe Ser Ile Tyr Arg Gly Met Ile Pro Pro Arg Thr Pro Cys
    1650                1655                1660
Gly Asp Leu Gln Val Thr Thr Arg Val Ile Glu Val Asn Gly Lys Arg
1665                1670                1675                1680
Gly Asp Phe Lys Lys Pro Ser Ser Cys Ile Ala Glu Tyr Glu Val Pro
                1685                1690                1695
Ala Asp Ala Trp Tyr Phe Asp Lys Asn Ser His Gly Ala Val Met Pro
            1700                1705                1710
Tyr Ser Ile Leu Met Glu Ile Ser Leu Gln Pro Asn Gly Phe Ile Ser
        1715                1720                1725
Gly Tyr Met Gly Thr Thr Leu Gly Phe Pro Gly Leu Glu Leu Phe Phe
    1730                1735                1740

Arg Asn Leu Asp Gly Ser Gly Glu Leu Leu Arg Glu Val Asp Leu Arg
1745                1750                1755                1760
Gly Lys Thr Ile Arg Asn Asp Ser Arg Leu Leu Ser Thr Val Met Ala
                1765                1770                1775
Gly Thr Asn Ile Ile Gln Ser Phe Ser Phe Glu Leu Ser Thr Asp Gly
            1780                1785                1790
Glu Pro Phe Tyr Arg Gly Thr Ala Val Phe Gly Tyr Phe Lys Gly Asp
        1795                1800                1805
Ala Leu Lys Asp Gln Leu Gly Leu Asp Asn Gly Lys Val Thr Gln Pro
    1810                1815                1820
Trp His Val Ala Asn Gly Val Ala Ala Ser Thr Lys Val Asn Leu Leu
1825                1830                1835                1840
Asp Lys Ser Cys Arg His Phe Asn Ala Pro Ala Asn Gln Pro His Tyr
                1845                1850                1855
Arg Leu Ala Gly Gly Gln Leu Asn Phe Ile Asp Ser Val Glu Ile Val
            1860                1865                1870
Asp Asn Gly Gly Thr Glu Gly Leu Gly Tyr Leu Tyr Ala Glu Arg Thr
        1875                1880                1885
Ile Asp Pro Ser Asp Trp Phe Phe Gln Phe His Phe His Gln Asp Pro
    1890                1895                1900
Val Met Pro Gly Ser Leu Gly Val Glu Ala Ile Ile Glu Thr Met Gln
1905                1910                1915                1920
Ala Tyr Ala Ile Ser Lys Asp Leu Gly Ala Asp Phe Lys Asn Pro Lys
                1925                1930                1935
Phe Gly Gln Ile Leu Ser Asn Ile Lys Trp Lys Tyr Arg Gly Gln Ile
            1940                1945                1950
Asn Pro Leu Asn Lys Gln Met Ser Met Asp Val Ser Ile Thr Ser Ile
        1955                1960                1965
Lys Asp Glu Asp Gly Lys Lys Val Ile Thr Gly Asn Ala Ser Leu Ser
    1970                1975                1980
Lys Asp Gly Leu Arg Ile Tyr Glu Val Phe Asp Ile Ala Ile Ser Ile
1985                1990                1995                2000
Glu Glu Ser Val

SEQ ID NO: 16
Sequence Length: 1629
Sequence Type: Nucleic acid

Strandedness: Double strand
Topology: Linear
Molecular Type: Genomic DNA
Source: Shewanella putrefaciens SCRC-2874 (FERM BP-1625)
Sequence
ATG AAT CCT ACA GCA ACT AAC GAA ATG CTT TCT CCG TGG CCA TGG GCT 48
Met Asn Pro Thr Ala Thr Asn Glu Met Leu Ser Pro Trp Pro Trp Ala
1               5                   10                  15
GTG ACA GAG TCA AAT ATC AGT TTT GAC GTG CAA GTG ATG GAA CAA CAA 96
Val Thr Glu Ser Asn Ile Ser Phe Asp Val Gln Val Met Glu Gln Gln
            20                  25                  30
CTT AAA GAT TTT AGC CGG GCA TGT TAC GTG GTC AAT CAT GCC GAC CAC 144
Leu Lys Asp Phe Ser Arg Ala Cys Tyr Val Val Asn His Ala Asp His
        35                  40                  45
GGC TTT GGT ATT GCG CAA ACT GCC GAT ATC GTG ACT GAA CAA GCG GCA 192
Gly Phe Gly Ile Ala Gln Thr Ala Asp Ile Val Thr Glu Gln Ala Ala
    50                  55                  60
AAC AGC ACA GAT TTA CCT GTT AGT GCT TTT ACT CCT GCA TTA GGT ACC 240
Asn Ser Thr Asp Leu Pro Val Ser Ala Phe Thr Pro Ala Leu Gly Thr
65                  70                  75                  80
GAA AGC CTA GGC GAC AAT AAT TTC CGC CGC GTT CAC GGC GTT AAA TAC 288
Glu Ser Leu Gly Asp Asn Asn Phe Arg Arg Val His Gly Val Lys Tyr
                85                  90                  95
GCT TAT TAC GCA GGC GCT ATG GCA AAC GGT ATT TCA TCT GAA GAG CTA 336
Ala Tyr Tyr Ala Gly Ala Met Ala Asn Gly Ile Ser Ser Glu Glu Leu
            100                 105                 110
GTG ATT GCC CTA GGT CAA GCT GGC ATT TTG TGT GGT TCG TTT GGA GCA 384
Val Ile Ala Leu Gly Gln Ala Gly Ile Leu Cys Gly Ser Phe Gly Ala
        115                 120                 125
GCC GGT CTT ATT CCA AGT CGC GTT GAA GCG GCA ATT AAC CGT ATT CAA 432
Ala Gly Leu Ile Pro Ser Arg Val Glu Ala Ala Ile Asn Arg Ile Gln
    130                 135                 140
GCA GCG CTG CCA AAT GGC CCT TAT ATG TTT AAC CTT ATC CAT AGT CCT 480
Ala Ala Leu Pro Asn Gly Pro Tyr Met Phe Asn Leu Ile His Ser Pro
145                 150                 155                 160

AGC GAG CCA GCA TTA GAG CGT GGC AGC GTA GAG CTA TTT TTA AAG CAT 528
Ser Glu Pro Ala Leu Glu Arg Gly Ser Val Glu Leu Phe Leu Lys His
                165                 170                 175
AAG GTA CGC ACC GTT GAA GCA TCA GCT TTC TTA GGT CTA ACA CCA CAA 576
Lys Val Arg Thr Val Glu Ala Ser Ala Phe Leu Gly Leu Thr Pro Gln
            180                 185                 190
ATC GTC TAT TAC CGT GCA GCA GGA TTG AGC CGA GAC GCA CAA GGT AAA 624
Ile Val Tyr Tyr Arg Ala Ala Gly Leu Ser Arg Asp Ala Gln Gly Lys
        195                 200                 205
GTT GTG GTT GGT AAC AAG GTT ATC GCT AAA GTA AGT CGC ACC GAA GTG 672
Val Val Val Gly Asn Lys Val Ile Ala Lys Val Ser Arg Thr Glu Val
    210                 215                 220
GCT GAA AAG TTT ATG ATG CCA GCG CCC GCA AAA ATG CTA CAA AAA CTA 720
Ala Glu Lys Phe Met Met Pro Ala Pro Ala Lys Met Leu Gln Lys Leu
225                 230                 235                 240
GTT GAT GAC GGT TCA ATT ACC GCT GAG CAA ATG GAG CTG GCG CAA CTT 768
Val Asp Asp Gly Ser Ile Thr Ala Glu Gln Met Glu Leu Ala Gln Leu
                245                 250                 255
GTA CCT ATG GCT GAC GAC ATC ACT GCA GAG GCC GAT TCA GGT GGC CAT 816
Val Pro Met Ala Asp Asp Ile Thr Ala Glu Ala Asp Ser Gly Gly His
            260                 265                 270
ACT GAT AAC CGT CCA TTA GTA ACA TTG CTG CCA ACC ATT TTA GCG CTG 864
Thr Asp Asn Arg Pro Leu Val Thr Leu Leu Pro Thr Ile Leu Ala Leu
        275                 280                 285
AAA GAA GAA ATT CAA GCT AAA TAC CAA TAC GAC ACT CCT ATT CGT GTC 912
Lys Glu Glu Ile Gln Ala Lys Tyr Gln Tyr Asp Thr Pro Ile Arg Val
    290                 295                 300
GGT TGT GGT GGC GGT GTG GGT ACG CCT GAT GCA GCG CTG GCA ACG TTT 960
Gly Cys Gly Gly Gly Val Gly Thr Pro Asp Ala Ala Leu Ala Thr Phe
305                 310                 315                 320
AAC ATG GGC GCG GCG TAT ATT GTT ACC GGC TCT ATC AAC CAA GCT TGT 1008
Asn Met Gly Ala Ala Tyr Ile Val Thr Gly Ser Ile Asn Gln Ala Cys
                325                 330                 335
GTT GAA GCG GGC GCA AGT GAT CAC ACT CGT AAA TTA CTT GCC ACC ACT 1056
Val Glu Ala Gly Ala Ser Asp His Thr Arg Lys Leu Leu Ala Thr Thr
            340                 345                 350

GAA ATG GCC GAT GTG ACT ATG GCA CCA GCT GCA GAT ATG TTC GAG ATG 1104
Glu Met Ala Asp Val Thr Met Ala Pro Ala Ala Asp Met Phe Glu Met
        355                 360                 365
GGC GTA AAA CTG CAG GTG GTT AAG CGC GGC ACG CTA TTC CCA ATG CGC 1152
Gly Val Lys Leu Gln Val Val Lys Arg Gly Thr Leu Phe Pro Met Arg
    370                 375                 380
GCT AAC AAG CTA TAT GAG ATC TAC ACC CGT TAC GAT TCA ATC GAA GCG 1200
Ala Asn Lys Leu Tyr Glu Ile Tyr Thr Arg Tyr Asp Ser Ile Glu Ala
385                 390                 395                 400
ATC CCA TTA GAC GAG CGT GAA AAG CTT GAG AAA CAA GTA TTC CGC TCA 1248
Ile Pro Leu Asp Glu Arg Glu Lys Leu Glu Lys Gln Val Phe Arg Ser
                405                 410                 415
AGC CTA GAT GAA ATA TGG GCA GGT ACA GTG GCG CAC TTT AAC GAG CGC 1296
Ser Leu Asp Glu Ile Trp Ala Gly Thr Val Ala His Phe Asn Glu Arg
            420                 425                 430
GAC CCT AAG CAA ATC GAA CGC GCA GAG GGT AAC CCT AAG CGT AAA ATG 1344
Asp Pro Lys Gln Ile Glu Arg Ala Glu Gly Asn Pro Lys Arg Lys Met
        435                 440                 445
GCA TTG ATT TTC CGT TGG TAC TTA GGT CTT TCT AGT CGC TGG TCA AAC 1392
Ala Leu Ile Phe Arg Trp Tyr Leu Gly Leu Ser Ser Arg Trp Ser Asn
    450                 455                 460
TCA GGC GAA GTG GGT CGT GAA ATG GAT TAT CAA ATT TGG GCT GGC CCT 1440
Ser Gly Glu Val Gly Arg Glu Met Asp Tyr Gln Ile Trp Ala Gly Pro
465                 470                 475                 480
GCT CTC GGT GCA TTT AAC CAA TGG GCA AAA GGC AGT TAC TTA GAT AAC 1488
Ala Leu Gly Ala Phe Asn Gln Trp Ala Lys Gly Ser Tyr Leu Asp Asn
                485                 490                 495
TAT CAA GAC CGA AAT GCC GTC GAT TTG GCA AAG CAC TTA ATG TAC GGC 1536
Tyr Gln Asp Arg Asn Ala Val Asp Leu Ala Lys His Leu Met Tyr Gly
            500                 505                 510
GCG GCT TAC TTA AAT CGT ATT AAC TCG CTA ACG GCT CAA GGC GTT AAA 1584
Ala Ala Tyr Leu Asn Arg Ile Asn Ser Leu Thr Ala Gln Gly Val Lys
        515                 520                 525
GTG CCA GCA CAG TTA CTT CGC TGG AAG CCA AAC CAA AGA ATG GCC 1629
Val Pro Ala Gln Leu Leu Arg Trp Lys Pro Asn Gln Arg Met Ala
    530                 535                 540

SEQ ID NO: 17
Sequence Length: 543
Sequence Type: Amino acid
Molecular Type: Peptide
Sequence
Met Asn Pro Thr Ala Thr Asn Glu Met Leu Ser Pro Trp Pro Trp Ala
1               5                   10                  15
Val Thr Glu Ser Asn Ile Ser Phe Asp Val Gln Val Met Glu Gln Gln
            20                  25                  30
Leu Lys Asp Phe Ser Arg Ala Cys Tyr Val Val Asn His Ala Asp His
        35                  40                  45
Gly Phe Gly Ile Ala Gln Thr Ala Asp Ile Val Thr Glu Gln Ala Ala
    50                  55                  60
Asn Ser Thr Asp Leu Pro Val Ser Ala Phe Thr Pro Ala Leu Gly Thr
65                  70                  75                  80
Glu Ser Leu Gly Asp Asn Asn Phe Arg Arg Val His Gly Val Lys Tyr
                85                  90                  95
Ala Tyr Tyr Ala Gly Ala Met Ala Asn Gly Ile Ser Ser Glu Glu Leu
            100                 105                 110
Val Ile Ala Leu Gly Gln Ala Gly Ile Leu Cys Gly Ser Phe Gly Ala
        115                 120                 125
Ala Gly Leu Ile Pro Ser Arg Val Glu Ala Ala Ile Asn Arg Ile Gln
    130                 135                 140
Ala Ala Leu Pro Asn Gly Pro Tyr Met Phe Asn Leu Ile His Ser Pro
145                 150                 155                 160
Ser Glu Pro Ala Leu Glu Arg Gly Ser Val Glu Leu Phe Leu Lys His
                165                 170                 175
Lys Val Arg Thr Val Glu Ala Ser Ala Phe Leu Gly Leu Thr Pro Gln
            180                 185                 190
Ile Val Tyr Tyr Arg Ala Ala Gly Leu Ser Arg Asp Ala Gln Gly Lys
        195                 200                 205
Val Val Val Gly Asn Lys Val Ile Ala Lys Val Ser Arg Thr Glu Val
    210                 215                 220
Ala Glu Lys Phe Met Met Pro Ala Pro Ala Lys Met Leu Gln Lys Leu
225                 230                 235                 240
Val Asp Asp Gly Ser Ile Thr Ala Glu Gln Met Glu Leu Ala Gln Leu
                245                 250                 255

Val Pro Met Ala Asp Asp Ile Thr Ala Glu Ala Asp Ser Gly Gly His
            260                 265                 270
Thr Asp Asn Arg Pro Leu Val Thr Leu Leu Pro Thr Ile Leu Ala Leu
        275                 280                 285
Lys Glu Glu Ile Gln Ala Lys Tyr Gln Tyr Asp Thr Pro Ile Arg Val
    290                 295                 300
Gly Cys Gly Gly Gly Val Gly Thr Pro Asp Ala Ala Leu Ala Thr Phe
305                 310                 315                 320
Asn Met Gly Ala Ala Tyr Ile Val Thr Gly Ser Ile Asn Gln Ala Cys
                325                 330                 335
Val Glu Ala Gly Ala Ser Asp His Thr Arg Lys Leu Leu Ala Thr Thr
            340                 345                 350
Glu Met Ala Asp Val Thr Met Ala Pro Ala Ala Asp Met Phe Glu Met
        355                 360                 365
Gly Val Lys Leu Gln Val Val Lys Arg Gly Thr Leu Phe Pro Met Arg
    370                 375                 380
Ala Asn Lys Leu Tyr Glu Ile Tyr Thr Arg Tyr Asp Ser Ile Glu Ala
385                 390                 395                 400
Ile Pro Leu Asp Glu Arg Glu Lys Leu Glu Lys Gln Val Phe Arg Ser
                405                 410                 415
Ser Leu Asp Glu Ile Trp Ala Gly Thr Val Ala His Phe Asn Glu Arg
            420                 425                 430
Asp Pro Lys Gln Ile Glu Arg Ala Glu Gly Asn Pro Lys Arg Lys Met
        435                 440                 445
Ala Leu Ile Phe Arg Trp Tyr Leu Gly Leu Ser Ser Arg Trp Ser Asn
    450                 455                 460
Ser Gly Glu Val Gly Arg Glu Met Asp Tyr Gln Ile Trp Ala Gly Pro
465                 470                 475                 480
Ala Leu Gly Ala Phe Asn Gln Trp Ala Lys Gly Ser Tyr Leu Asp Asn
                485                 490                 495
Tyr Gln Asp Arg Asn Ala Val Asp Leu Ala Lys His Leu Met Tyr Gly
            500                 505                 510
Ala Ala Tyr Leu Asn Arg Ile Asn Ser Leu Thr Ala Gln Gly Val Lys
        515                 520                 525
Val Pro Ala Gln Leu Leu Arg Trp Lys Pro Asn Gln Arg Met Ala
    530                 535                 540

SEQ ID NO: 18
Sequence Length: 1575
Sequence Type: Nucleic acid
Strandedness: Double strand
Topology: Linear
Molecular Type: Genomic DNA
Source: Shewanella putrefaciens SCRC-2874 (FERM BP-1625)
Sequence
GTG CAA CTA CCA TTA ATT GAG GCC TCA TTA GTT AAA TTA TCT GAG CAA 48
Val Gln Leu Pro Leu Ile Glu Ala Ser Leu Val Lys Leu Ser Glu Gln
1               5                   10                  15
GAG CTC ACC TCT TTA AAT TAC GCT TTT CAG CAA ATG AGA AAG CCA CTA 96
Glu Leu Thr Ser Leu Asn Tyr Ala Phe Gln Gln Met Arg Lys Pro Leu
            20                  25                  30
CAA ACC ATT AAT TAC GAC TAT GCG GTG TGG GAC AGA ACC TAC AGC TAT 144
Gln Thr Ile Asn Tyr Asp Tyr Ala Val Trp Asp Arg Thr Tyr Ser Tyr
        35                  40                  45
ATG AAA TCA AAC TCA GCG AGC GCT AAA AGG TAC TAT GAA AAA CAT GAG 192
Met Lys Ser Asn Ser Ala Ser Ala Lys Arg Tyr Tyr Glu Lys His Glu
    50                  55                  60
TAC CCA GAT GAT ACG TTC AAG AGT TTA AAA GTC GAC GGA GTA TTT ATA 240
Tyr Pro Asp Asp Thr Phe Lys Ser Leu Lys Val Asp Gly Val Phe Ile
65                  70                  75                  80
TTC AAC CGT ACA AAT CAG CCA GTT TTT AGT AAA GGT TTT AAT CAT AGA 288
Phe Asn Arg Thr Asn Gln Pro Val Phe Ser Lys Gly Phe Asn His Arg
                85                  90                  95
AAT GAT ATA CCG CTG GTC TTT GAA TTA ACT GAC TTT AAA CAA CAT CCA 336
Asn Asp Ile Pro Leu Val Phe Glu Leu Thr Asp Phe Lys Gln His Pro
            100                 105                 110
CAA AAC ATC GCA TTA TCT CCA CAA ACC AAA CAG GCA CAC CCA CCG GCA 384
Gln Asn Ile Ala Leu Ser Pro Gln Thr Lys Gln Ala His Pro Pro Ala
        115                 120                 125
AGT AAG CCG TTA GAC TCC CCT GAT GAT GTG CCT TCT ACC CAT GGG GTT 432
Ser Lys Pro Leu Asp Ser Pro Asp Asp Val Pro Ser Thr His Gly Val
    130                 135                 140

ATC GCC ACA CGA TAC GGT CCA GCA ATT TAT AGC TCT ACC AGC ATT TTA 480
Ile Ala Thr Arg Tyr Gly Pro Ala Ile Tyr Ser Ser Thr Ser Ile Leu
145                 150                 155                 160
AAA TCT GAT CGT AGC GGC TCC CAA CTT GGT TAT TTA GTC TTC ATT AGG 528
Lys Ser Asp Arg Ser Gly Ser Gln Leu Gly Tyr Leu Val Phe Ile Arg
                165                 170                 175
TTA ATT GAT GAA TGG TTC ATC GCT GAG CTA TCG CAA TAC ACT GCC GCA 576
Leu Ile Asp Glu Trp Phe Ile Ala Glu Leu Ser Gln Tyr Thr Ala Ala
            180                 185                 190
GGT GTT GAA ATC GCT ATG GCT GAT GCC GCA GAC GCA CAA TTA GCG AGA 624
Gly Val Glu Ile Ala Met Ala Asp Ala Ala Asp Ala Gln Leu Ala Arg
        195                 200                 205
TTA GGC GCA AAC ACT AAG CTT AAT AAA GTA ACC GCT ACA TCC GAA CGG 672
Leu Gly Ala Asn Thr Lys Leu Asn Lys Val Thr Ala Thr Ser Glu Arg
    210                 215                 220
TTA ATA ACT AAT GTC GAT GGT AAG CCT CTG TTG AAG TTA GTG CTT TAC 720
Leu Ile Thr Asn Val Asp Gly Lys Pro Leu Leu Lys Leu Val Leu Tyr
225                 230                 235                 240
CAT ACC AAT AAC CAA CCG CCG CCG ATG CTA GAT TAC AGT ATA ATA ATT 768
His Thr Asn Asn Gln Pro Pro Pro Met Leu Asp Tyr Ser Ile Ile Ile
                245                 250                 255
CTA TTA GTT GAG ATG TCA TTT TTA CTG ATC CTC GCT TAT TTC CTT TAC 816
Leu Leu Val Glu Met Ser Phe Leu Leu Ile Leu Ala Tyr Phe Leu Tyr
            260                 265                 270
TCC TAC TTC TTA GTC AGG CCA GTT AGA AAG CTG GCT TCA GAT ATT AAA 864
Ser Tyr Phe Leu Val Arg Pro Val Arg Lys Leu Ala Ser Asp Ile Lys
        275                 280                 285
AAA ATG GAT AAA AGT CGT GAA ATT AAA AAG CTA AGG TAT CAC TAC CCT 912
Lys Met Asp Lys Ser Arg Glu Ile Lys Lys Leu Arg Tyr His Tyr Pro
    290                 295                 300
ATT ACT GAG CTA GTC AAA GTT GCG ACT CAC TTC AAC GCC CTA ATG GGG 960
Ile Thr Glu Leu Val Lys Val Ala Thr His Phe Asn Ala Leu Met Gly
305                 310                 315                 320
ACG ATT CAG GAA CAA ACT AAA CAG CTT AAT GAA CAA GTT TTT ATT GAT 1008
Thr Ile Gln Glu Gln Thr Lys Gln Leu Asn Glu Gln Val Phe Ile Asp
                325                 330                 335

AAA TTA ACC AAT ATT CCC AAT CGT CGC GCT TTT GAG CAG CGA CTT GAA 1056
Lys Leu Thr Asn Ile Pro Asn Arg Arg Ala Phe Glu Gln Arg Leu Glu
            340                 345                 350
ACC TAT TGC CAA CTG CTA GCC CGG CAA CAA ATT GGC TTT ACT CTC ATC 1104
Thr Tyr Cys Gln Leu Leu Ala Arg Gln Gln Ile Gly Phe Thr Leu Ile
        355                 360                 365
ATT GCC GAT GTG GAT CAT TTT AAA GAG TAC AAC GAT ACT CTT GGG CAC 1152
Ile Ala Asp Val Asp His Phe Lys Glu Tyr Asn Asp Thr Leu Gly His
    370                 375                 380
CTT GCT GGG GAT GAA GCA TTA ATA AAA GTG GCA CAA ACA CTA TCG CAA 1200
Leu Ala Gly Asp Glu Ala Leu Ile Lys Val Ala Gln Thr Leu Ser Gln
385                 390                 395                 400
CAG TTT TAC CGT GCA GAA GAT ATT TGT GCC CGT TTT GGT GGT GAA GAA 1248
Gln Phe Tyr Arg Ala Glu Asp Ile Cys Ala Arg Phe Gly Gly Glu Glu
                405                 410                 415
TTT ATT ATG TTA TTT CGA GAC ATA CCT GAT GAG CCC TTG CAG AGA AAG 1296
Phe Ile Met Leu Phe Arg Asp Ile Pro Asp Glu Pro Leu Gln Arg Lys
            420                 425                 430
CTC GAT GCG ATG CTG CAC TCT TTT GCA GAG CTC AAC CTA CCT CAT CCA 1344
Leu Asp Ala Met Leu His Ser Phe Ala Glu Leu Asn Leu Pro His Pro
        435                 440                 445
AAC TCA TCA ACC GCT AAT TAC GTT ACT GTG AGC CTT GGG GTT TGC ACA 1392
Asn Ser Ser Thr Ala Asn Tyr Val Thr Val Ser Leu Gly Val Cys Thr
    450                 455                 460
GTT GTT GCT GTT GAT GAT TTT GAA TTT AAA AGT GAG TCG CAT ATT ATT 1440
Val Val Ala Val Asp Asp Phe Glu Phe Lys Ser Glu Ser His Ile Ile
465                 470                 475                 480
GGC AGT CAG GCT GCA TTA ATC GCA GAT AAG GCG CTT TAT CAT GCT AAA 1488
Gly Ser Gln Ala Ala Leu Ile Ala Asp Lys Ala Leu Tyr His Ala Lys
                485                 490                 495
GCC TGT GGT CGT AAC CAG TTG TCA AAA ACT ACT ATT ACT GTT GAT GAG 1536
Ala Cys Gly Arg Asn Gln Leu Ser Lys Thr Thr Ile Thr Val Asp Glu
            500                 505                 510
ATT GAG CAA TTA GAA GCA AAT AAA ATC GGT CAT CAA GCC 1575
Ile Glu Gln Leu Glu Ala Asn Lys Ile Gly His Gln Ala
        515                 520                 525

SEQ ID NO: 19
Sequence Length: 525
Sequence Type: Amino acid
Molecular Type: Peptide
Sequence
Val Gln Leu Pro Leu Ile Glu Ala Ser Leu Val Lys Leu Ser Glu Gln
1               5                   10                  15
Glu Leu Thr Ser Leu Asn Tyr Ala Phe Gln Gln Met Arg Lys Pro Leu
            20                  25                  30
Gln Thr Ile Asn Tyr Asp Tyr Ala Val Trp Asp Arg Thr Tyr Ser Tyr
        35                  40                  45
Met Lys Ser Asn Ser Ala Ser Ala Lys Arg Tyr Tyr Glu Lys His Glu
    50                  55                  60
Tyr Pro Asp Asp Thr Phe Lys Ser Leu Lys Val Asp Gly Val Phe Ile
65                  70                  75                  80
Phe Asn Arg Thr Asn Gln Pro Val Phe Ser Lys Gly Phe Asn His Arg
                85                  90                  95
Asn Asp Ile Pro Leu Val Phe Glu Leu Thr Asp Phe Lys Gln His Pro
            100                 105                 110
Gln Asn Ile Ala Leu Ser Pro Gln Thr Lys Gln Ala His Pro Pro Ala
        115                 120                 125
Ser Lys Pro Leu Asp Ser Pro Asp Asp Val Pro Ser Thr His Gly Val
    130                 135                 140
Ile Ala Thr Arg Tyr Gly Pro Ala Ile Tyr Ser Ser Thr Ser Ile Leu
145                 150                 155                 160
Lys Ser Asp Arg Ser Gly Ser Gln Leu Gly Tyr Leu Val Phe Ile Arg
                165                 170                 175
Leu Ile Asp Glu Trp Phe Ile Ala Glu Leu Ser Gln Tyr Thr Ala Ala
            180                 185                 190
Gly Val Glu Ile Ala Met Ala Asp Ala Ala Asp Ala Gln Leu Ala Arg
        195                 200                 205
Leu Gly Ala Asn Thr Lys Leu Asn Lys Val Thr Ala Thr Ser Glu Arg
    210                 215                 220
Leu Ile Thr Asn Val Asp Gly Lys Pro Leu Leu Lys Leu Val Leu Tyr
225                 230                 235                 240
His Thr Asn Asn Gln Pro Pro Pro Met Leu Asp Tyr Ser Ile Ile Ile
                245                 250                 255

Leu Leu Val Glu Met Ser Phe Leu Leu Ile Leu Ala Tyr Phe Leu Tyr
            260                 265                 270
Ser Tyr Phe Leu Val Arg Pro Val Arg Lys Leu Ala Ser Asp Ile Lys
        275                 280                 285
Lys Met Asp Lys Ser Arg Glu Ile Lys Lys Leu Arg Tyr His Tyr Pro
    290                 295                 300
Ile Thr Glu Leu Val Lys Val Ala Thr His Phe Asn Ala Leu Met Gly
305                 310                 315                 320
Thr Ile Gln Glu Gln Thr Lys Gln Leu Asn Glu Gln Val Phe Ile Asp
                325                 330                 335
Lys Leu Thr Asn Ile Pro Asn Arg Arg Ala Phe Glu Gln Arg Leu Glu
            340                 345                 350
Thr Tyr Cys Gln Leu Leu Ala Arg Gln Gln Ile Gly Phe Thr Leu Ile
        355                 360                 365
Ile Ala Asp Val Asp His Phe Lys Glu Tyr Asn Asp Thr Leu Gly His
    370                 375                 380
Leu Ala Gly Asp Glu Ala Leu Ile Lys Val Ala Gln Thr Leu Ser Gln
385                 390                 395                 400
Gln Phe Tyr Arg Ala Glu Asp Ile Cys Ala Arg Phe Gly Gly Glu Glu
                405                 410                 415
Phe Ile Met Leu Phe Arg Asp Ile Pro Asp Glu Pro Leu Gln Arg Lys
            420                 425                 430
Leu Asp Ala Met Leu His Ser Phe Ala Glu Leu Asn Leu Pro His Pro
        435                 440                 445
Asn Ser Ser Thr Ala Asn Tyr Val Thr Val Ser Leu Gly Val Cys Thr
    450                 455                 460
Val Val Ala Val Asp Asp Phe Glu Phe Lys Ser Glu Ser His Ile Ile
465                 470                 475                 480
Gly Ser Gln Ala Ala Leu Ile Ala Asp Lys Ala Leu Tyr His Ala Lys
                485                 490                 495
Ala Cys Gly Arg Asn Gln Leu Ser Lys Thr Thr Ile Thr Val Asp Glu
            500                 505                 510
Ile Glu Gln Leu Glu Ala Asn Lys Ile Gly His Gln Ala
        515                 520                 525

## Claims

1. A gene coding for a group of eicosapentaenoic acid synthesis enzymes encoded by a nucleotide sequence shown in SEQ ID NO.: 1.

2. A gene coding for a group of eicosapentaenoic acid synthesis enzymes encoded by a nucleotide sequence 5661 to 34631 of SEQ ID NO: 1.

3. A gene coding for a group of eicosapentaenoic acid synthesis enzymes encoded by a nucleotide sequence 7710 to 35564 of SEQ ID NO.: 1.

4. A gene coding for a group of eicosapentaenoic acid synthesis enzymes encoded by a nucleotide sequence 7710 to 34631 of SEQ ID NO.: 1.

5. A gene coding for a group of eicosapentaenoic acid synthesis enzymes at least comprising amino acid sequences shown by SEQ ID NOs.: 7 and 9.

6. A gene coding for a group of eicosapentaenoic acid synthesis enzymes, at least comprising nucleotide sequences shown by SEQ ID NOs.: 6 and 8.

7. A gene coding for a group of eicosapentaenoic acid synthesis enzymes comprising at least one amino acid sequence selected from the group of amino acid sequences shown by SEQ ID NOs.: 5, 11, 13, 15, 17 and 19.

8. A gene coding for a group of eicosapentaenoic acid synthesis enzymes, comprising at least one nucleotide sequence selected from the nucleotide sequences shown by SEQ ID NOs.: 4, 10, 12, 14, 16 and 18.

9. A gene coding for an enzyme participating in the biosynthesis of eicosapentaenoic acid, having a amino acid sequence selected from the group of amino acid sequences shown by SEQ ID NOs.: 13 and 15.

10. A gene coding for an enzyme participating in the biosynthesis of eicosapentaenoic acid, having a nucleotide sequence selected from the group of nucleotide sequences shown by SEQ ID NOs.: 12 and 14.

11. Plasmid comprising a gene according to any one of claims 1, 2, 3, 4, 5, 6, 7, 8, 9 and 10.

12. Bacterium transformed with a plasmid comprising a gene according to claim 1.

13. Bacterium transformed with a plasmid comprising a gene according to any one of claims 2, 3 and 4.

14. A process for production of eicosapentaenoic acid, comprising the step of culturing a bacterium according to claim 12.

15. A process for production of eicosapentaenoic acid, comprising the step of culturing a bacterium according to claim 13.

16. A gene coding for a group of eicosapentaenoic acid synthesis enzyme, obtainable by the following steps:
(1) obtaining cells by culturing a microorganism-belonging to the genus Shewanella capable of producing eicosapentaenoic acid;
(2) extracting chromosomal DNA from the cultured cells;
(3) obtaining DNA fragments by partial digestion of the chromosomal DNA with a restriction enzyme Sau3A1;
(4) obtaining a recombinant expression vector by inserting the DNA fragment into a cosmid pWE15 which has been completely digested by a restriction enzyme BamHI;
(5) obtaining a transformed microorganism by transforming E. coli with the recombinant expression vector;
(6) obtaining transformed microbial cells by culturing the transformed microorganisms;
(7) selecting a transformed microorganism capable of producing eicosapentaenoic acid by detecting eicosapentaenoic acid from the transformed cells; and
(8) recovering a DNA coding for a group of eicosapentaenoic acid synthesis enzymes by cleaving with a restriction enzyme Sau3A1 the recombinant expression vector extracted from the transformed microorganism capable of producing eicosapentaenoic acid.
